(19) **Europäisches Patentamt**
**European Patent Office**
**Office européen des brevets**

(11) **EP 4 169 948 A1**

(12) **EUROPEAN PATENT APPLICATION**
published in accordance with Art. 153(4) EPC

(43) Date of publication:
**26.04.2023 Bulletin 2023/17**

(21) Application number: **21829463.5**

(22) Date of filing: **21.06.2021**

(51) International Patent Classification (IPC):
***C07K 16/28*** (2006.01)   ***A61P 35/00*** (2006.01)

(52) Cooperative Patent Classification (CPC):
**A61P 35/00; C07K 16/28**

(86) International application number:
**PCT/CN2021/101233**

(87) International publication number:
**WO 2021/259199 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.06.2020   CN 202010594227**

(71) Applicant: **Innovent Biologics (Suzhou) Co., Ltd.
Suzhou, Jiangsu 215123 (CN)**

(72) Inventors:
• **JING, Hua**
  **Suzhou, Jiangsu 215123 (CN)**

• **SHEN, Haoran**
  **Suzhou, Jiangsu 215123 (CN)**
• **TANG, Rong**
  **Suzhou, Jiangsu 215123 (CN)**
• **LIU, Junjian**
  **Suzhou, Jiangsu 215123 (CN)**

(74) Representative: **Sagittarius IP
Marlow International
Parkway
Marlow, SL7 1YL (GB)**

Remarks:
The complete document including Reference
Table(s) and the Sequence Listing(s) can be
downloaded from the EPO website

(54) **ANTI-CD73 ANTIBODY AND USE THEREOF**

(57)    The present invention relates to novel antibodies or antigen-binding fragments thereof that specifically bind to CD73 and compositions comprising the antibodies or antigen-binding fragments thereof. The present invention also relates to a nucleic acid encoding the antibody or antigen-binding fragment thereof of the present invention, a vector comprising the polynucleotide, a host cell comprising the nucleic acid or the vector, an immunoconjugate and pharmaceutical composition comprising the antibody or antigen-binding fragment thereof. Furthermore, the present invention relates to the uses of these antibodies or antigen-binding fragments thereof in the immunotherapy, prevention and/or diagnosis of diseases.

EP 4 169 948 A1

**Description**

**FIELD OF THE INVENTION**

The present invention generally relates to the field of immunology and antibody engineering. In particular, the present invention relates to novel antibodies or antigen-binding fragments thereof that specifically bind to CD73 and compositions comprising the antibodies or antigen-binding fragments thereof. Furthermore, the present invention relates to nucleic acids encoding said antibodies or antigen-binding fragments thereof, vectors comprising the nucleic acids, host cells comprising the nucleic acids or vectors, immunoconjugates and pharmaceutical compositions comprising the antibodies or antigen-binding fragments thereof. Furthermore, the present invention relates to the uses of these antibodies or antigen-binding fragments thereof in the immunotherapy, prevention and/or diagnosis of diseases.

**BACKGROUND OF THE INVENTION**

CD73 (Cluster of Differentiation 73), also known as extracellular-5'-nucleotidase (Ecto-5'-NT), is a 70-kDa glycosylphosphatidylinositol (GPI)-anchored protein. CD73 plays an extremely important role in the process of converting ATP to adenosine. It can dephosphorylate ATP to adenosine in combination with CD39, in which CD39 mainly converts ATP to ADP and AMP, and CD73 dephosphorylates AMP to adenosine.

CD73 is highly expressed in different tumor cells such as breast cancer and lung cancer, and the high expression of CD73 is positively correlated with poor prognosis. High expression of CD73 is positively correlated with shorter disease-free survival in breast cancer patients treated with trastuzumab, a monoclonal antibody against HER2/ErbB2. In addition, CD73 is also expressed in endothelial cells, fibroblasts, lymphocytes and myeloid cells in tumor tissues. CD73 molecules on the surface of cell membrane can be cleaved by phospholipases and enter the intercellular substance and blood. The CD73 in the intercellular substance and blood also has 5'-nucleotidase activity. ATP and its metabolites AMP and adenosine have important roles in cellular metabolism, signal transduction and immune homeostasis, among which adenosine has immunosuppressive activity. Extracellular adenosine accumulates in cancerous tissues, underlying an important mechanism for tumor immune escape. Adenosine has been shown to regulate proliferation and migration in many cancers and has immunosuppressive effects by modulating anti-tumor T cells (Zhang et al. Cancer Res 2010; 70:6407-11). Therefore, an effective anti-CD73 molecule antibody is required to efficiently inhibit the enzymatic activity of the cell surface CD73 molecule and soluble CD73 molecule, thereby blocking the conversion of AMP to adenosine, and releasing the inhibition of T cell proliferation and activity caused by the accumulation of adenosine, thereby achieving the purposes of changing the tumor microenvironment, activating tumor immune response, and inhibiting tumor growth.

Some anti-CD73 molecule antibodies have been developed in the prior art, but they also have their own defects, such as the anti-CD73 molecule antibody disclosed in patent US20180194858A1, which shows poor inhibitory effect on soluble CD73 enzymatic activity at high antibody concentrations (hook effect); the anti-CD73 molecule antibodies of patents WO2018013611A1 and WO2016055609 A1 cannot completely block the enzymatic activity of CD73. Therefore, it is still necessary to develop some more excellent anti-CD73 molecule antibodies with different antibody sequences, and the present invention satisfies these requirements.

**SUMMARY OF THE INVENTION**

The present invention provides a novel antibody or antigen-binding fragment thereof that binds to the CD73 molecule.

In some embodiments, the anti-CD73 antibody or antigen-binding fragment thereof of the invention comprises a heavy chain variable region (VH), wherein the VH comprises

(i) three complementarity determining regions (CDRs) comprised in the VH of any antibody listed in Table B; or
(ii) sequences totally comprising at least one and no more than 5, 4, 3, 2 or 1 amino acid change (preferably amino acid substitution, preferably conservative substitution) in the three CDR regions relative to the sequences of (i), or
(iii) sequences having at least 90%, 91%, 92%, 93%, 94%, 95%, 96%, 97%, 98%, 99% or 100% identity to the VH sequence of any one of the antibodies listed in Table B, and comprising the corresponding CDRs of the sequence.

In some embodiments, the anti-CD73 antibody or antigen-binding fragment thereof of the invention comprises a light chain variable region (VL), wherein the VL comprises

(i) three complementarity determining regions (CDRs) comprised in the VL of any antibody listed in Table B; or
(ii) sequences totally comprising at least one and no more than 5, 4, 3, 2 or 1 amino acid change (preferably amino acid substitution, preferably conservative substitution) in the three CDR regions relative to the sequences of (i), or

(iii) sequences having at least 90%, 91%, 92%, 93%, 94%, 95%, 96%, 97%, 98%, 99% or 100% identity to the VL sequence of any one of the antibodies listed in Table B, and comprising the corresponding CDRs of the sequence.

In some embodiments, the anti-CD73 antibody or antigen-binding fragment thereof of the invention comprises a heavy chain variable region VH and/or a light chain variable region VL, wherein

(a) the VH comprises

(i) three complementarity determining regions (CDRs) comprised in the VH of any antibody listed in Table B; or
(ii) sequences totally comprising at least one and no more than 5, 4, 3, 2 or 1 amino acid change (preferably amino acid substitution, preferably conservative substitution) in the three CDR regions relative to the sequences of (i), or
(iii) sequences having at least 90%, 91%, 92%, 93%, 94%, 95%, 96%, 97%, 98%, 99% or 100% identity to the VH sequence of any one of the antibodies listed in Table B, and comprising the corresponding CDRs of the sequence;
(iv) a combination of HCDR1, HCDR2 and HCDR3 as shown in Table A or Table D;

and / or

(a) the VL comprises

(i) three complementarity determining regions (CDRs) comprised in the VL of any antibody listed in Table B; or
(ii) sequences totally comprising at least one and no more than 5, 4, 3, 2 or 1 amino acid change (preferably amino acid substitution, preferably conservative substitution) in the three CDR regions relative to the sequences of (i), or
(iii) sequences having at least 90%, 91%, 92%, 93%, 94%, 95%, 96%, 97%, 98%, 99% or 100% identity to the VL sequence of any one of the antibodies listed in Table B, and comprising the corresponding CDRs of the sequence; or
(iv) a combination of LCDR1, LCDR2 and LCDR3 as shown in Table A or Table D.

In some embodiments, the present invention provides an anti-CD73 antibody or antigen-binding fragment thereof that binds to CD73 molecule, preferably human CD73 protein, comprising a heavy chain variable region VH and/or a light chain variable region VL, wherein,

1) the VH comprises the three complementarity determining region (CDR) HCDR1, HCDR2 and HCDR3 comprised in the VH as shown in SEQ ID NO:55, and the VL comprises LCDR1, LCDR2 and LCDR3 comprised in the VL as shown in SEQ ID NO:91;

2) the VH comprises the three complementarity determining region (CDR) HCDR1, HCDR2 and HCDR3 comprised in the VH as shown in SEQ ID NO:56, and the VL comprises LCDR1, LCDR2 and LCDR3 comprised in the VL as shown in SEQ ID NO:92;

3) the VH comprises the three complementarity determining region (CDR) HCDR1, HCDR2 and HCDR3 comprised in the VH as shown in SEQ ID NO:57, and the VL comprises LCDR1, LCDR2 and LCDR3 comprised in the VL as shown in SEQ ID NO:92;

4) the VH comprises the three complementarity determining region (CDR) HCDR1, HCDR2 and HCDR3 comprised in the VH as shown in SEQ ID NO:58, and the VL comprises LCDR1, LCDR2 and LCDR3 comprised in the VL as shown in SEQ ID NO:92;

5) the VH comprises the three complementarity determining region (CDR) HCDR1, HCDR2 and HCDR3 comprised in the VH as shown in SEQ ID NO:59, and the VL comprises LCDR1, LCDR2 and LCDR3 comprised in the VL as shown in SEQ ID NO:93;

6) the VH comprises the three complementarity determining region (CDR) HCDR1, HCDR2 and HCDR3 comprised in the VH as shown in SEQ ID NO:60, and the VL comprises LCDR1, LCDR2 and LCDR3 comprised in the VL as shown in SEQ ID NO:93;

7) the VH comprises the three complementarity determining region (CDR) HCDR1, HCDR2 and HCDR3 comprised in the VH as shown in SEQ ID NO:61, and the VL comprises LCDR1, LCDR2 and LCDR3 comprised in the VL as shown in SEQ ID NO:93;

8) the VH comprises the three complementarity determining region (CDR) HCDR1, HCDR2 and HCDR3 comprised in the VH as shown in SEQ ID NO:62, and the VL comprises LCDR1, LCDR2 and LCDR3 comprised in the VL as shown in SEQ ID NO:93;

9) the VH comprises the three complementarity determining region (CDR) HCDR1, HCDR2 and HCDR3 comprised in the VH as shown in SEQ ID NO:63, and the VL comprises LCDR1, LCDR2 and LCDR3 comprised in the VL as shown in SEQ ID NO:93;

10) the VH comprises the three complementarity determining region (CDR) HCDR1, HCDR2 and HCDR3 comprised in the VH as shown in SEQ ID NO:64, and the VL comprises LCDR1, LCDR2 and LCDR3 comprised in the VL as shown in SEQ ID NO:93;

11) the VH comprises the three complementarity determining region (CDR) HCDR1, HCDR2 and HCDR3 comprised in the VH as shown in SEQ ID NO:65, and the VL comprises LCDR1, LCDR2 and LCDR3 comprised in the VL as shown in SEQ ID NO:94;

12) the VH comprises the three complementarity determining region (CDR) HCDR1, HCDR2 and HCDR3 comprised in the VH as shown in SEQ ID NO:66, and the VL comprises LCDR1, LCDR2 and LCDR3 comprised in the VL as shown in SEQ ID NO:94;

13) the VH comprises the three complementarity determining region (CDR) HCDR1, HCDR2 and HCDR3 comprised in the VH as shown in SEQ ID NO:67, and the VL comprises LCDR1, LCDR2 and LCDR3 comprised in the VL as shown in SEQ ID NO:94;

14) the VH comprises the three complementarity determining region (CDR) HCDR1, HCDR2 and HCDR3 comprised in the VH as shown in SEQ ID NO:68, and the VL comprises LCDR1, LCDR2 and LCDR3 comprised in the VL as shown in SEQ ID NO:95;

15) the VH comprises the three complementarity determining region (CDR) HCDR1, HCDR2 and HCDR3 comprised in the VH as shown in SEQ ID NO:69, and the VL comprises LCDR1, LCDR2 and LCDR3 comprised in the VL as shown in SEQ ID NO:96;

16) the VH comprises the three complementarity determining region (CDR) HCDR1, HCDR2 and HCDR3 comprised in the VH as shown in SEQ ID NO:70, and the VL comprises LCDR1, LCDR2 and LCDR3 comprised in the VL as shown in SEQ ID NO:97;

17) the VH comprises the three complementarity determining region (CDR) HCDR1, HCDR2 and HCDR3 comprised in the VH as shown in SEQ ID NO:71, and the VL comprises LCDR1, LCDR2 and LCDR3 comprised in the VL as shown in SEQ ID NO:97; or

18) the VH comprises the three complementarity determining region (CDR) HCDR1, HCDR2 and HCDR3 comprised in the VH as shown in SEQ ID NO:72, and the VL comprises LCDR1, LCDR2 and LCDR3 comprised in the VL as shown in SEQ ID NO:97.

In some embodiments, the invention provides an anti-CD73 antibody or antigen-binding fragment thereof comprising a heavy chain variable region VH and/or a light chain variable region VL, wherein

(i) the VH comprises complementarity determining region (CDR) HCDR1, HCDR2 and HCDR3, wherein the HCDR1 comprises an amino acid sequence as shown in any one of SEQ ID Nos: 1-15, 132-133 or 136-149; the HCDR2 comprises an amino acid sequence as shown in any one of SEQ ID Nos: 16-30, 134 or 135; the HCDR3 comprises an amino acid sequence as shown in any one of SEQ ID Nos: 31-37 or 150-156; and / or

(ii) wherein the VL comprises complementarity determining region (CDR) LCDR1, LCDR2 and LCDR3, wherein the LCDR1 comprises an amino acid sequence as shown in any one of SEQ ID Nos: 38-43; the LCDR2 comprises an amino acid sequence as shown in any one of SEQ ID Nos:, 44-48; the LCDR3 comprises an amino acid sequence as shown in any one of SEQ ID Nos: 49-54.

In some embodiments, the invention provides an anti-CD73 antibody or antigen-binding fragment thereof comprising a heavy chain variable region VH and/or a light chain variable region VL, wherein

1) the VH comprises complementarity determining region (CDR) HCDR1, HCDR2 and HCDR3, wherein the HCDR1 comprises or consists of the amino acid sequence as shown in SEQ ID NO: 1 or 136; the HCDR2 comprises or consists of the amino acid sequence as shown in SEQ ID NO: 16; the HCDR3 comprises or consists of the amino acid sequence as shown in SEQ ID NO: 31 or 150; the VL comprises complementarity determining region (CDR) LCDR1, LCDR2 and LCDR3, wherein the LCDR1 comprises or consists of the amino acid sequence as shown in SEQ ID NO: 38; the LCDR2 comprises or consists of the amino acid sequence as shown in SEQ ID NO: 44; the LCDR3 comprises or consists of the amino acid sequence as shown in SEQ ID NO: 49;

2) the VH comprises complementarity determining region (CDR) HCDR1, HCDR2 and HCDR3, wherein the HCDR1 comprises or consists of the amino acid sequence as shown in SEQ ID NO: 1 or 136; the HCDR2 comprises or consists of the amino acid sequence as shown in SEQ ID NO: 16; the HCDR3 comprises or consists of the amino acid sequence as shown in SEQ ID NO: 32 or 151; the VL comprises complementarity determining region (CDR) LCDR1, LCDR2 and LCDR3, wherein the LCDR1 comprises or consists of the amino acid sequence as shown in SEQ ID NO: 39; the LCDR2 comprises or consists of the amino acid sequence as shown in SEQ ID NO: 45; the LCDR3 comprises or consists of the amino acid sequence as shown in SEQ ID NO: 50;

3) the VH comprises complementarity determining region (CDR) HCDR1, HCDR2 and HCDR3, wherein the HCDR1 comprises or consists of the amino acid sequence as shown in SEQ ID No: 2 or 137; the HCDR2 comprises or consists of the amino acid sequence as shown in SEQ ID NO: 17; the HCDR3 comprises or consists of the amino acid sequence as shown in SEQ ID NO: 32 or 151; the VL comprises complementarity determining region (CDR) LCDR1, LCDR2 and LCDR3, wherein the LCDR1 comprises or consists of the amino acid sequence as shown in SEQ ID NO: 39; the LCDR2 comprises or consists of the amino acid sequence as shown in SEQ ID NO: 45; the LCDR3 comprises or consists of the amino acid sequence as shown in SEQ ID NO: 50;

4) the VH comprises complementarity determining region (CDR) HCDR1, HCDR2 and HCDR3, wherein the HCDR1 comprises or consists of the amino acid sequence as shown in SEQ ID No: 3 or 138; the HCDR2 comprises or consists of the amino acid sequence as shown in SEQ ID NO: 18; the HCDR3 comprises or consists of the amino acid sequence as shown in SEQ ID NO: 32 or 151; the VL comprises complementarity determining region (CDR) LCDR1, LCDR2 and LCDR3, wherein the LCDR1 comprises or consists of the amino acid sequence as shown in SEQ ID NO: 39; the LCDR2 comprises or consists of the amino acid sequence as shown in SEQ ID NO: 45; the LCDR3 comprises or consists of the amino acid sequence as shown in SEQ ID NO: 50;

5) the VH comprises complementarity determining region (CDR) HCDR1, HCDR2 and HCDR3, wherein the HCDR1 comprises or consists of the amino acid sequence as shown in SEQ ID No: 4 or 139; the HCDR2 comprises or consists of the amino acid sequence as shown in SEQ ID NO: 19; the HCDR3 comprises or consists of the amino acid sequence as shown in SEQ ID NO: 33 or 152; the VL comprises complementarity determining region (CDR) LCDR1, LCDR2 and LCDR3, wherein the LCDR1 comprises or consists of the amino acid sequence as shown in SEQ ID NO: 40; the LCDR2 comprises or consists of the amino acid sequence as shown in SEQ ID NO: 46; the LCDR3 comprises or consists of the amino acid sequence as shown in SEQ ID NO: 51;

6) the VH comprises complementarity determining region (CDR) HCDR1, HCDR2 and HCDR3, wherein the HCDR1 comprises or consists of the amino acid sequence as shown in SEQ ID No: 4 or 139; the HCDR2 comprises or consists of the amino acid sequence as shown in SEQ ID NO: 20; the HCDR3 comprises or consists of the amino acid sequence as shown in SEQ ID NO: 33 or 152; the VL comprises complementarity determining region (CDR) LCDR1, LCDR2 and LCDR3, wherein the LCDR1 comprises or consists of the amino acid sequence as shown in SEQ ID NO: 40; the LCDR2 comprises or consists of the amino acid sequence as shown in SEQ ID NO: 46; the LCDR3 comprises or consists of the amino acid sequence as shown in SEQ ID NO: 51;

7) the VH comprises complementarity determining region (CDR) HCDR1, HCDR2 and HCDR3, wherein the HCDR1 comprises or consists of the amino acid sequence as shown in SEQ ID NO: 5 or 139; the HCDR2 comprises or consists of the amino acid sequence as shown in SEQ ID NO: 21; the HCDR3 comprises or consists of the amino acid sequence as shown in SEQ ID NO: 33 or 152; the VL comprises complementarity determining region (CDR) LCDR1, LCDR2 and LCDR3, wherein the LCDR1 comprises or consists of the amino acid sequence as shown in SEQ ID NO: 40; the LCDR2 comprises or consists of the amino acid sequence as shown in SEQ ID NO: 46; the LCDR3 comprises or consists of the amino acid sequence as shown in SEQ ID NO: 51;

8) the VH comprises complementarity determining region (CDR) HCDR1, HCDR2 and HCDR3, wherein the HCDR1 comprises or consists of the amino acid sequence as shown in SEQ ID NO: 6 or 140; the HCDR2 comprises or consists of the amino acid sequence as shown in SEQ ID NO: 22; the HCDR3 comprises or consists of the amino acid sequence as shown in SEQ ID NO: 33 or 152; the VL comprises complementarity determining region (CDR) LCDR1, LCDR2 and LCDR3, wherein the LCDR1 comprises or consists of the amino acid sequence as shown in SEQ ID NO: 40; the LCDR2 comprises or consists of the amino acid sequence as shown in SEQ ID NO: 46; the LCDR3 comprises or consists of the amino acid sequence as shown in SEQ ID NO: 51;

9) the VH comprises complementarity determining region (CDR) HCDR1, HCDR2 and HCDR3, wherein the HCDR1 comprises or consists of the amino acid sequence as shown in SEQ ID No: 7 or 141; the HCDR2 comprises or consists of the amino acid sequence as shown in SEQ ID NO: 23; the HCDR3 comprises or consists of the amino acid sequence as shown in SEQ ID NO: 33 or 152; the VL comprises complementarity determining region (CDR) LCDR1, LCDR2 and LCDR3, wherein the LCDR1 comprises or consists of the amino acid sequence as shown in SEQ ID No: 40; the LCDR2 comprises or consists of the amino acid sequence as shown in SEQ ID No: 46; the LCDR3 comprises or consists of the amino acid sequence as shown in SEQ ID No: 51;

10) the VH comprises complementarity determining region (CDR) HCDR1, HCDR2 and HCDR3, wherein the HCDR1 comprises or consists of the amino acid sequence as shown in SEQ ID NO: 8 or 142; the HCDR2 comprises or consists of the amino acid sequence as shown in SEQ ID NO: 23; the HCDR3 comprises or consists of the amino acid sequence as shown in SEQ ID NO: 33 or 152; the VL comprises complementarity determining region (CDR) LCDR1, LCDR2 and LCDR3, wherein the LCDR1 comprises or consists of the amino acid sequence as shown in SEQ ID NO: 40; the LCDR2 comprises or consists of the amino acid sequence as shown in SEQ ID NO: 46; the LCDR3 comprises or consists of the amino acid sequence as shown in SEQ ID NO: 51;

11) the VH comprises complementarity determining region (CDR) HCDR1, HCDR2 and HCDR3, wherein the HCDR1 comprises or consists of the amino acid sequence as shown in SEQ ID No: 9 or 143; the HCDR2 comprises or consists of the amino acid sequence as shown in SEQ ID NO: 24; the HCDR3 comprises or consists of the amino acid sequence as shown in SEQ ID NO: 34 or 153; the VL comprises complementarity determining region (CDR) LCDR1, LCDR2 and LCDR3, wherein the LCDR1 comprises or consists of the amino acid sequence as shown in SEQ ID NO: 41; the LCDR2 comprises or consists of the amino acid sequence as shown in SEQ ID NO: 47; the LCDR3 comprises or consists of the amino acid sequence as shown in SEQ ID NO: 52;

12) the VH comprises complementarity determining region (CDR) HCDR1, HCDR2 and HCDR3, wherein the HCDR1 comprises or consists of the amino acid sequence as shown in SEQ ID No: 10 or 144; the HCDR2 comprises or consists of the amino acid sequence as shown in SEQ ID NO: 25; the HCDR3 comprises or consists of the amino acid sequence as shown in SEQ ID NO: 34 or 153; the VL comprises complementarity determining region (CDR) LCDR1, LCDR2 and LCDR3, wherein the LCDR1 comprises or consists of the amino acid sequence as shown in SEQ ID NO: 41; the LCDR2 comprises or consists of the amino acid sequence as shown in SEQ ID NO: 47; the LCDR3 comprises or consists of the amino acid sequence as shown in SEQ ID NO: 52;

13) the VH comprises complementarity determining region (CDR) HCDR1, HCDR2 and HCDR3, wherein the HCDR1 comprises or consists of the amino acid sequence as shown in SEQ ID NO: 11 or 145; the HCDR2 comprises or consists of the amino acid sequence as shown in SEQ ID NO: 26; the HCDR3 comprises or consists of the amino acid sequence as shown in SEQ ID NO: 35 or 154; the VL comprises complementarity determining region (CDR) LCDR1, LCDR2 and LCDR3, wherein the LCDR1 comprises or consists of the amino acid sequence as shown in SEQ ID NO: 41; the LCDR2 comprises or consists of the amino acid sequence as shown in SEQ ID NO: 47; the LCDR3 comprises or consists of the amino acid sequence as shown in SEQ ID NO: 52;

14) the VH comprises complementarity determining region (CDR) HCDR1, HCDR2 and HCDR3, wherein the HCDR1 comprises or consists of the amino acid sequence as shown in SEQ ID No: 9 or 143; the HCDR2 comprises or consists of the amino acid sequence as shown in SEQ ID No: 24; the HCDR3 comprises or consists of the amino acid sequence as shown in SEQ ID NO: 36 or 155; the VL comprises complementarity determining region (CDR) LCDR1, LCDR2 and LCDR3, wherein the LCDR1 comprises or consists of the amino acid sequence as shown in SEQ ID NO: 39; the LCDR2 comprises or consists of the amino acid sequence as shown in SEQ ID NO: 45; the LCDR3 comprises or consists of the amino acid sequence as shown in SEQ ID NO: 53;

15) the VH comprises complementarity determining region (CDR) HCDR1, HCDR2 and HCDR3, wherein the HCDR1 comprises or consists of the amino acid sequence as shown in SEQ ID NO: 12 or 146; the HCDR2 comprises or consists of the amino acid sequence as shown in SEQ ID NO: 27; the HCDR3 comprises or consists of the amino acid sequence as shown in SEQ ID NO: 36 or 155; the VL comprises complementarity determining region (CDR) LCDR1, LCDR2 and LCDR3, wherein the LCDR1 comprises or consists of the amino acid sequence as shown in SEQ ID NO: 42; the LCDR2 comprises or consists of the amino acid sequence as shown in SEQ ID NO: 45; the LCDR3 comprises or consists of the amino acid sequence as shown in SEQ ID NO: 53;

16) the VH comprises complementarity determining region (CDR) HCDR1, HCDR2 and HCDR3, wherein the HCDR1 comprises or consists of the amino acid sequence as shown in SEQ ID NO: 13 or 147; the HCDR2 comprises or consists of the amino acid sequence as shown in SEQ ID NO: 28; the HCDR3 comprises or consists of the amino acid sequence as shown in SEQ ID NO: 37 or 156; the VL comprises complementarity determining region (CDR) LCDR1, LCDR2 and LCDR3, wherein the LCDR1 comprises or consists of the amino acid sequence as shown in SEQ ID NO: 43; the LCDR2 comprises or consists of the amino acid sequence as shown in SEQ ID NO: 48; the LCDR3 comprises or consists of the amino acid sequence as shown in SEQ ID NO: 54;

17) the VH comprises complementarity determining region (CDR) HCDR1, HCDR2 and HCDR3, wherein the HCDR1 comprises or consists of the amino acid sequence as shown in SEQ ID No: 14 or 148; the HCDR2 comprises or consists of the amino acid sequence as shown in SEQ ID NO: 29; the HCDR3 comprises or consists of the amino acid sequence as shown in SEQ ID NO: 37 or 156; the VL comprises complementarity determining region (CDR) LCDR1, LCDR2 and LCDR3, wherein the LCDR1 comprises or consists of the amino acid sequence as shown in SEQ ID NO: 43; the LCDR2 comprises or consists of the amino acid sequence as shown in SEQ ID No: 48; the LCDR3 comprises or consists of the amino acid sequence as shown in SEQ ID No: 54;

18) the VH comprises complementarity determining region (CDR) HCDR1, HCDR2 and HCDR3, wherein the HCDR1 comprises or consists of the amino acid sequence as shown in SEQ ID NO: 15 or 149; the HCDR2 comprises or consists of the amino acid sequence as shown in SEQ ID NO: 30; the HCDR3 comprises or consists of the amino acid sequence as shown in SEQ ID NO: 37 or 156; the VL comprises complementarity determining region (CDR) LCDR1, LCDR2 and LCDR3, wherein the LCDR1 comprises or consists of the amino acid sequence as shown in SEQ ID NO: 43; the LCDR2 comprises or consists of the amino acid sequence as shown in SEQ ID No: 48; the LCDR3 comprises or consists of the amino acid sequence as shown in SEQ ID No: 54; or

In some embodiments, the invention provides an anti-CD73 antibody or antigen-binding fragment thereof comprising a heavy chain variable region VH and/or a light chain variable region VL, wherein

(a) the heavy chain variable region VH

(i) comprises or consists of an amino acid sequence having at least 90%, 91%, 92%, 93%, 94%, 95%, 96%, 97%, 98% or 99% identity to the amino acid sequence as shown in any one of SEQ ID NOs: 55-72, and comprises the corresponding CDR sequences of the sequence as shown in any one of SEQ ID NOs: 55-72; or
(ii) comprises or consists of the amino acid sequence as shown in any one of SEQ ID NOs: 55-72; or
(iii) comprises an amino acid sequence having one or more (preferably no more than 10, more preferably no more than 5, 4, 3, 2, 1) amino acid changes (preferably amino acid substitutions, more preferably amino acid conservative substitutions) compared to the amino acid sequence as shown in any one of SEQ ID NOs: 55-72, preferably, the amino acid change does not occur in the CDR regions;
and/or

(b) the light chain variable region VL

(i) comprises or consists of an amino acid sequence having at least 90%, 91%, 92%, 93%, 94%, 95%, 96%, 97%, 98% or 99% identity to the amino acid sequence as shown in any one of SEQ ID NOs: 91-97, and comprises the corresponding CDR sequences of the sequence as shown in any one of SEQ ID NOs: 91-97;
(ii) comprises or consists of the amino acid sequence as shown in any one of SEQ ID NOs: 91-97; or
(iii) comprises an amino acid sequence having one or more (preferably no more than 10, more preferably no more than 5, 4, 3, 2, 1) amino acid changes (preferably amino acid substitutions, more preferably amino acid conservative substitutions) compared to the amino acid sequence as shown in any one of SEQ ID NOs: 91-97, preferably, the amino acid change does not occur in the CDR regions.

In some embodiments, the present invention provides an anti-CD73 antibody or antigen-binding fragment thereof comprising

1) a heavy chain variable region VH comprising an amino acid sequence having at least 90%, 91%, 92%, 93%, 94%, 95%, 96%, 97%, 98% or 99% identity to the amino acid sequence as shown in SEQ ID NO:55, and a light chain variable region VL comprising an amino acid sequence having at least 90%, 91%, 92%, 93%, 94%, 95%, 96%, 97%, 98% or 99% identity to the amino acid sequence as shown in SEQ ID NO:91;

2) a heavy chain variable region VH comprising an amino acid sequence having at least 90%, 91%, 92%, 93%, 94%, 95%, 96%, 97%, 98% or 99% identity to the amino acid sequence as shown in SEQ ID NO:56, and a light chain variable region comprising an amino acid sequence having at least 90%, 91%, 92%, 93%, 94%, 95%, 96%, 97%, 98% or 99% identity to the amino acid sequence as shown in SEQ ID NO:92;

3) a heavy chain variable region VH comprising an amino acid sequence having at least 90%, 91%, 92%, 93%, 94%, 95%, 96%, 97%, 98% or 99% identity to the amino acid sequence as shown in SEQ ID NO:57, and a light chain variable region VL comprising an amino acid sequence having at least 90%, 91%, 92%, 93%, 94%, 95%, 96%, 97%, 98% or 99% identity to the amino acid sequence as shown in SEQ ID NO:92;

4) a heavy chain variable region VH comprising an amino acid sequence having at least 90%, 91%, 92%, 93%, 94%, 95%, 96%, 97%, 98% or 99% identity to the amino acid sequence as shown in SEQ ID NO:58, and a light chain variable region VL comprising an amino acid sequence having at least 90%, 91%, 92%, 93%, 94%, 95%, 96%, 97%, 98% or 99% identity to the amino acid sequence as shown in SEQ ID NO:92;

5) a heavy chain variable region VH comprising an amino acid sequence having at least 90%, 91%, 92%, 93%, 94%, 95%, 96%, 97%, 98% or 99% identity to the amino acid sequence as shown in SEQ ID NO:59, and a light chain variable region comprising an amino acid sequence having at least 90%, 91%, 92%, 93%, 94%, 95%, 96%, 97%, 98% or 99% identity to the amino acid sequence as shown in SEQ ID NO:93;

6) a heavy chain variable region VH comprising an amino acid sequence having at least 90%, 91%, 92%, 93%, 94%, 95%, 96%, 97%, 98% or 99% identity to the amino acid sequence as shown in SEQ ID NO:60, and a light chain variable region VL comprising an amino acid sequence having at least 90%, 91%, 92%, 93%, 94%, 95%, 96%, 97%, 98% or 99% identity to the amino acid sequence as shown in SEQ ID NO:93;

7) a heavy chain variable region VH comprising an amino acid sequence having at least 90%, 91%, 92%, 93%, 94%, 95%, 96%, 97%, 98% or 99% identity to the amino acid sequence as shown in SEQ ID NO:61, and a light chain variable region VL comprising an amino acid sequence having at least 90%, 91%, 92%, 93%, 94%, 95%, 96%, 97%, 98% or 99% identity to the amino acid sequence as shown in SEQ ID NO:93;

8) a heavy chain variable region VH comprising an amino acid sequence having at least 90%, 91%, 92%, 93%, 94%, 95%, 96%, 97%, 98% or 99% identity to the amino acid sequence as shown in SEQ ID NO:62, and a light chain variable region VL comprising an amino acid sequence having at least 90%, 91%, 92%, 93%, 94%, 95%, 96%, 97%, 98% or 99% identity to the amino acid sequence as shown in SEQ ID NO:93;

9) a heavy chain variable region VH comprising an amino acid sequence having at least 90%, 91%, 92%, 93%, 94%, 95%, 96%, 97%, 98% or 99% identity to the amino acid sequence as shown in SEQ ID NO:63, and a light chain variable region VL comprising an amino acid sequence having at least 90%, 91%, 92%, 93%, 94%, 95%, 96%, 97%, 98% or 99% identity to the amino acid sequence as shown in SEQ ID NO:93;

10) a heavy chain variable region VH comprising an amino acid sequence having at least 90%, 91%, 92%, 93%, 94%, 95%, 96%, 97%, 98% or 99% identity to the amino acid sequence as shown in SEQ ID NO:64, and a light chain variable region VL comprising an amino acid sequence having at least 90%, 91%, 92%, 93%, 94%, 95%, 96%, 97%, 98% or 99% identity to the amino acid sequence as shown in SEQ ID NO:93;

11) a heavy chain variable region VH comprising an amino acid sequence having at least 90%, 91%, 92%, 93%, 94%, 95%, 96%, 97%, 98% or 99% identity to the amino acid sequence as shown in SEQ ID NO:65, and a light chain variable region VL comprising an amino acid sequence having at least 90%, 91%, 92%, 93%, 94%, 95%, 96%, 97%, 98% or 99% identity to the amino acid sequence as shown in SEQ ID NO:94;

12) a heavy chain variable region VH comprising an amino acid sequence having at least 90%, 91%, 92%, 93%, 94%, 95%, 96%, 97%, 98% or 99% identity to the amino acid sequence as shown in SEQ ID NO:66, and a light chain variable region VL comprising an amino acid sequence having at least 90%, 91%, 92%, 93%, 94%, 95%, 96%, 97%, 98% or 99% identity to the amino acid sequence as shown in SEQ ID NO:94;

13) a heavy chain variable region VH comprising an amino acid sequence having at least 90%, 91%, 92%, 93%, 94%, 95%, 96%, 97%, 98% or 99% identity to the amino acid sequence as shown in SEQ ID NO:67, and a light chain variable region VL comprising an amino acid sequence having at least 90%, 91%, 92%, 93%, 94%, 95%, 96%, 97%, 98% or 99% identity to the amino acid sequence as shown in SEQ ID NO:94;

14) a heavy chain variable region VH comprising an amino acid sequence having at least 90%, 91%, 92%, 93%, 94%, 95%, 96%, 97%, 98% or 99% identity to the amino acid sequence as shown in SEQ ID NO:68, and a light chain variable region VL comprising an amino acid sequence having at least 90%, 91%, 92%, 93%, 94%, 95%, 96%, 97%, 98% or 99% identity to the amino acid sequence as shown in SEQ ID NO:95;

15) a heavy chain variable region VH comprising an amino acid sequence having at least 90%, 91%, 92%, 93%, 94%, 95%, 96%, 97%, 98% or 99% identity to the amino acid sequence as shown in SEQ ID NO:69, and a light chain variable region VL comprising an amino acid sequence having at least 90%, 91%, 92%, 93%, 94%, 95%, 96%, 97%, 98% or 99% identity to the amino acid sequence as shown in SEQ ID NO:96;

16) a heavy chain variable region VH comprising an amino acid sequence having at least 90%, 91%, 92%, 93%, 94%, 95%, 96%, 97%, 98% or 99% identity to the amino acid sequence as shown in SEQ ID NO:70, and a light chain variable region VL comprising an amino acid sequence having at least 90%, 91%, 92%, 93%, 94%, 95%, 96%, 97%, 98% or 99% identity to the amino acid sequence as shown in SEQ ID NO:97;

17) a heavy chain variable region VH comprising an amino acid sequence having at least 90%, 91%, 92%, 93%, 94%, 95%, 96%, 97%, 98% or 99% identity to the amino acid sequence as shown in SEQ ID NO:71, and a light chain variable region VL comprising an amino acid sequence having at least 90%, 91%, 92%, 93%, 94%, 95%, 96%, 97%, 98% or 99% identity to the amino acid sequence as shown in SEQ ID NO:97; or

18) a heavy chain variable region VH comprising an amino acid sequence having at least 90%, 91%, 92%, 93%, 94%, 95%, 96%, 97%, 98% or 99% identity to the amino acid sequence as shown in SEQ ID NO:72, and a light chain variable region VL comprising an amino acid sequence having at least 90%, 91%, 92%, 93%, 94%, 95%, 96%, 97%, 98% or 99% identity to the amino acid sequence as shown in SEQ ID NO:97.

In some embodiments, the present invention provides an anti-CD73 antibody or antigen-binding fragment thereof comprising

1) a heavy chain variable region VH comprising the amino acid sequence as shown in SEQ ID NO:55, and a light chain variable region VL comprising the amino acid sequence as shown in SEQ ID NO:91;

2) a heavy chain variable region VH comprising the amino acid sequence as shown in SEQ ID NO:56, and a light chain variable region VL comprising the amino acid sequence as shown in SEQ ID NO:92;

3) a heavy chain variable region VH comprising the amino acid sequence as shown in SEQ ID NO:57, and a light chain variable region VL comprising the amino acid sequence as shown in SEQ ID NO:92;

4) a heavy chain variable region VH comprising the amino acid sequence as shown in SEQ ID NO:58, and a light chain variable region VL comprising the amino acid sequence as shown in SEQ ID NO:92;

5) a heavy chain variable region VH comprising the amino acid sequence as shown in SEQ ID NO:59, and a light chain variable region VL comprising the amino acid sequence as shown in SEQ ID NO:93;

6) a heavy chain variable region VH comprising the amino acid sequence as shown in SEQ ID NO:60, and a light chain variable region VL comprising the amino acid sequence as shown in SEQ ID NO:93;

7) a heavy chain variable region VH comprising the amino acid sequence as shown in SEQ ID NO:61, and a light chain variable region VL comprising the amino acid sequence as shown in SEQ ID NO:93;

8) a heavy chain variable region VH comprising the amino acid sequence as shown in SEQ ID NO:62, and a light chain variable region VL comprising the amino acid sequence as shown in SEQ ID NO:93;

9) a heavy chain variable region VH comprising the amino acid sequence as shown in SEQ ID NO:63, and a light chain variable region VL comprising the amino acid sequence as shown in SEQ ID NO:93;

10) a heavy chain variable region VH comprising the amino acid sequence as shown in SEQ ID NO:64, and a light chain variable region VL comprising the amino acid sequence as shown in SEQ ID NO:93;

11) a heavy chain variable region VH comprising the amino acid sequence as shown in SEQ ID NO:65, and a light chain variable region VL comprising the amino acid sequence as shown in SEQ ID NO:94;

12) a heavy chain variable region VH comprising the amino acid sequence as shown in SEQ ID NO:66, and a light chain variable region VL comprising the amino acid sequence as shown in SEQ ID NO:94;

13) a heavy chain variable region VH comprising the amino acid sequence as shown in SEQ ID NO:67, and a light chain variable region VL comprising the amino acid sequence as shown in SEQ ID NO:94;

14) a heavy chain variable region VH comprising the amino acid sequence as shown in SEQ ID NO:68, and a light chain variable region VL comprising the amino acid sequence as shown in SEQ ID NO:95;

15) a heavy chain variable region VH comprising the amino acid sequence as shown in SEQ ID NO:69, and a light chain variable region VL comprising the amino acid sequence as shown in SEQ ID NO:96;

16) a heavy chain variable region VH comprising the amino acid sequence as shown in SEQ ID NO:70, and a light chain variable region VL comprising the amino acid sequence as shown in SEQ ID NO:97;

17) a heavy chain variable region VH comprising the amino acid sequence as shown in SEQ ID NO:71, and a light chain variable region VL comprising the amino acid sequence as shown in SEQ ID NO:97; or

18) a heavy chain variable region VH comprising the amino acid sequence as shown in SEQ ID NO:72, and a light chain variable region VL comprising the amino acid sequence as shown in SEQ ID NO:97;

In some embodiments, the invention provides an anti-CD73 antibody or antigen-binding fragment thereof comprising a heavy chain and/or a light chain, wherein

(a) the heavy chain

(i) comprises or consists of an amino acid sequence having at least 85%, 90%, 91%, 92%, 93%, 94%, 95%, 96%, 97%, 98% or 99% identity to the amino acid sequence as shown in any one of SEQ ID Nos: 106-123 and comprises the corresponding CDR sequences of the sequence as shown in any one of SEQ ID Nos: 106-123;

(ii) comprises or consists of the amino acid sequence as shown in any one of SEQ ID NOs: 106-123; or

(iii) comprises an amino acid sequence having one or more (preferably no more than 20 or 10, more preferably no more than 5, 4, 3, 2, 1) amino acid changes (preferably amino acid substitutions, more preferably conservative amino acid substitutions) compared to the amino acid sequence as shown in any one of SEQ ID NOs: 106-123, preferably, the amino acid changes do not occur in the heavy chain CDR regions, more preferably, the amino acid changes do not occur in the heavy chain variable region;
and/or

(b) the light chain

(i) comprises or consists of an amino acid sequence having at least 85%, 90%, 91%, 92%, 93%, 94%, 95%, 96%, 97%, 98% or 99% identity to the amino acid sequence as shown in any one of SEQ ID Nos: 124-130 and comprises the corresponding CDR sequences of the sequence as shown in any one of SEQ ID Nos: 124-130;

(ii) comprises or consists of the amino acid sequence as shown in any one of SEQ ID NOs: 124-130; or

(iii) comprises an amino acid sequence having one or more (preferably no more than 20 or 10, more preferably no more than 5, 4, 3, 2, 1) amino acid changes (preferably amino acid substitutions, more preferably conservative amino acid substitutions) compared to the amino acid sequence as shown in any one of SEQ ID NOs: 124-130, preferably, the amino acid changes do not occur in the light chain CDR regions, more preferably, the amino acid changes do not occur in the light chain variable region.

In some embodiments, the present invention provides an anti-CD73 antibody or antigen-binding fragment thereof comprising

1) a heavy chain comprising an amino acid sequence having at least 85%, 90%, 91%, 92%, 93%, 94%, 95%, 96%, 97%, 98% or 99% identity to the amino acid sequence as shown in SEQ ID NO: 106, and a light chain comprising an amino acid sequence having at least 85%, 90%, 91%, 92%, 93%, 94%, 95%, 96%, 97%, 98% or 99% identity to the amino acid sequence as shown in SEQ ID NO:124;

2) a heavy chain comprising an amino acid sequence having at least 85%, 90%, 91%, 92%, 93%, 94%, 95%, 96%, 97%, 98% or 99% identity to the amino acid sequence as shown in SEQ ID NO:107, and a light chain comprising an amino acid sequence having at least 85%, 90%, 91%, 92%, 93%, 94%, 95%, 96%, 97%, 98% or 99% identity to the amino acid sequence as shown in SEQ ID NO:125;

3) a heavy chain comprising an amino acid sequence having at least 85%, 90%, 91%, 92%, 93%, 94%, 95%, 96%, 97%, 98% or 99% identity to the amino acid sequence as shown in SEQ ID NO:108, and a light chain comprising an amino acid sequence having at least 85%, 90%, 91%, 92%, 93%, 94%, 95%, 96%, 97%, 98% or 99% identity to the amino acid sequence as shown in SEQ ID NO:125;

4) a heavy chain comprising an amino acid sequence having at least 85%, 90%, 91%, 92%, 93%, 94%, 95%, 96%, 97%, 98% or 99% identity to the amino acid sequence as shown in SEQ ID NO:109, and a light chain comprising an amino acid sequence having at least 85%, 90%, 91%, 92%, 93%, 94%, 95%, 96%, 97%, 98% or 99% identity to the amino acid sequence as shown in SEQ ID NO:125;

5) a heavy chain comprising an amino acid sequence having at least 85%, 90%, 91%, 92%, 93%, 94%, 95%, 96%, 97%, 98% or 99% identity to the amino acid sequence as shown in SEQ ID NO:110, and a light chain comprising an amino acid sequence having at least 85%, 90%, 91%, 92%, 93%, 94%, 95%, 96%, 97%, 98% or 99% identity to the amino acid sequence as shown in SEQ ID NO:126;

6) a heavy chain comprising an amino acid sequence having at least 85%, 90%, 91%, 92%, 93%, 94%, 95%, 96%, 97%, 98% or 99% identity to the amino acid sequence as shown in SEQ ID NO:111, and a light chain comprising an amino acid sequence having at least 85%, 90%, 91%, 92%, 93%, 94%, 95%, 96%, 97%, 98% or 99% identity to the amino acid sequence as shown in SEQ ID NO:126;

7) a heavy chain comprising an amino acid sequence having at least 85%, 90%, 91%, 92%, 93%, 94%, 95%, 96%, 97%, 98% or 99% identity to the amino acid sequence as shown in SEQ ID NO: 112, and a light chain comprising an amino acid sequence having at least 85%, 90%, 91%, 92%, 93%, 94%, 95%, 96%, 97%, 98% or 99% identity to the amino acid sequence as shown in SEQ ID NO:126;

8) a heavy chain comprising an amino acid sequence having at least 85%, 90%, 91%, 92%, 93%, 94%, 95%, 96%, 97%, 98% or 99% identity to the amino acid sequence as shown in SEQ ID NO:113, and a light chain comprising an amino acid sequence having at least 85%, 90%, 91%, 92%, 93%, 94%, 95%, 96%, 97%, 98% or 99% identity to the amino acid sequence as shown in SEQ ID NO:126;

9) a heavy chain comprising an amino acid sequence having at least 85%, 90%, 91%, 92%, 93%, 94%, 95%, 96%, 97%, 98% or 99% identity to the amino acid sequence as shown in SEQ ID NO:114, and a light chain comprising an amino acid sequence having at least 85%, 90%, 91%, 92%, 93%, 94%, 95%, 96%, 97%, 98% or 99% identity to the amino acid sequence as shown in SEQ ID NO:126;

10) a heavy chain comprising an amino acid sequence having at least 85%, 90%, 91%, 92%, 93%, 94%, 95%, 96%, 97%, 98% or 99% identity to the amino acid sequence as shown in SEQ ID NO:115, and a light chain comprising an amino acid sequence having at least 85%, 90%, 91%, 92%, 93%, 94%, 95%, 96%, 97%, 98% or 99% identity to the amino acid sequence as shown in SEQ ID NO:126;

11) a heavy chain comprising an amino acid sequence having at least 85%, 90%, 91%, 92%, 93%, 94%, 95%, 96%, 97%, 98% or 99% identity to the amino acid sequence as shown in SEQ ID NO:116, and a light chain comprising an amino acid sequence having at least 85%, 90%, 91%, 92%, 93%, 94%, 95%, 96%, 97%, 98% or 99% identity to the amino acid sequence as shown in SEQ ID NO:127;

12) a heavy chain comprising an amino acid sequence having at least 85%, 90%, 91%, 92%, 93%, 94%, 95%, 96%, 97%, 98% or 99% identity to the amino acid sequence as shown in SEQ ID NO:117, and a light chain comprising an amino acid sequence having at least 85%, 90%, 91%, 92%, 93%, 94%, 95%, 96%, 97%, 98% or 99% identity to the amino acid sequence as shown in SEQ ID NO:127;

13) a heavy chain comprising an amino acid sequence having at least 85%, 90%, 91%, 92%, 93%, 94%, 95%, 96%, 97%, 98% or 99% identity to the amino acid sequence as shown in SEQ ID NO:118, and a light chain comprising an amino acid sequence having at least 85%, 90%, 91%, 92%, 93%, 94%, 95%, 96%, 97%, 98% or 99% identity to the amino acid sequence as shown in SEQ ID NO:127;

14) a heavy chain comprising an amino acid sequence having at least 85%, 90%, 91%, 92%, 93%, 94%, 95%, 96%, 97%, 98% or 99% identity to the amino acid sequence as shown in SEQ ID NO:119, and a light chain comprising an amino acid sequence having at least 85%, 90%, 91%, 92%, 93%, 94%, 95%, 96%, 97%, 98% or 99% identity to the amino acid sequence as shown in SEQ ID NO:128;

15) a heavy chain comprising an amino acid sequence having at least 85%, 90%, 91%, 92%, 93%, 94%, 95%, 96%, 97%, 98% or 99% identity to the amino acid sequence as shown in SEQ ID NO:120, and a light chain comprising an amino acid sequence having at least 85%, 90%, 91%, 92%, 93%, 94%, 95%, 96%, 97%, 98% or 99% identity to the amino acid sequence as shown in SEQ ID NO:129;

16) a heavy chain comprising an amino acid sequence having at least 85%, 90%, 91%, 92%, 93%, 94%, 95%, 96%, 97%, 98% or 99% identity to the amino acid sequence as shown in SEQ ID NO:121, and a light chain comprising an amino acid sequence having at least 85%, 90%, 91%, 92%, 93%, 94%, 95%, 96%, 97%, 98% or 99% identity to the amino acid sequence as shown in SEQ ID NO:130;

17) a heavy chain comprising an amino acid sequence having at least 85%, 90%, 91%, 92%, 93%, 94%, 95%, 96%, 97%, 98% or 99% identity to the amino acid sequence as shown in SEQ ID NO:122, and a light chain comprising an amino acid sequence having at least 85%, 90%, 91%, 92%, 93%, 94%, 95%, 96%, 97%, 98% or 99% identity to the amino acid sequence as shown in SEQ ID NO:130; or

18) a heavy chain comprising an amino acid sequence having at least 85%, 90%, 91%, 92%, 93%, 94%, 95%, 96%, 97%, 98% or 99% identity to the amino acid sequence as shown in SEQ ID NO:123, and a light chain comprising an amino acid sequence having at least 85%, 90%, 91%, 92%, 93%, 94%, 95%, 96%, 97%, 98% or 99% identity to the amino acid sequence as shown in SEQ ID NO:130;

In some embodiments, the present invention provides an anti-CD73 antibody or antigen-binding fragment thereof comprising

1) a heavy chain comprising the amino acid sequence as shown in SEQ ID NO: 106, and a light chain comprising the amino acid sequence as shown in SEQ ID NO: 124;

2) a heavy chain comprising the amino acid sequence as shown in SEQ ID NO: 107, and a light chain comprising the amino acid sequence as shown in SEQ ID NO: 125;

3) a heavy chain comprising the amino acid sequence as shown in SEQ ID NO: 108, and a light chain comprising the amino acid sequence as shown in SEQ ID NO: 125;

4) a heavy chain comprising the amino acid sequence as shown in SEQ ID NO: 109, and a light chain comprising the amino acid sequence as shown in SEQ ID NO: 125;

5) a heavy chain comprising the amino acid sequence as shown in SEQ ID NO: 110, and a light chain comprising the amino acid sequence as shown in SEQ ID NO: 126;

6) a heavy chain comprising the amino acid sequence as shown in SEQ ID NO: 111, and a light chain comprising the amino acid sequence as shown in SEQ ID NO: 126;

7) a heavy chain comprising the amino acid sequence as shown in SEQ ID NO: 112, and a light chain comprising the amino acid sequence as shown in SEQ ID NO: 126;

8) a heavy chain comprising the amino acid sequence as shown in SEQ ID NO: 113, and a light chain comprising the amino acid sequence as shown in SEQ ID NO: 126;

9) a heavy chain comprising the amino acid sequence as shown in SEQ ID NO: 114, and a light chain comprising the amino acid sequence as shown in SEQ ID NO: 126;

10) a heavy chain comprising the amino acid sequence as shown in SEQ ID NO: 115, and a light chain comprising the amino acid sequence as shown in SEQ ID NO: 126;

11) a heavy chain comprising the amino acid sequence as shown in SEQ ID NO: 116, and a light chain comprising the amino acid sequence as shown in SEQ ID NO: 127;

12) a heavy chain comprising the amino acid sequence as shown in SEQ ID NO:117, and a light chain comprising the amino acid sequence as shown in SEQ ID NO:127;

13) a heavy chain comprising the amino acid sequence as shown in SEQ ID NO: 118, and a light chain comprising the amino acid sequence as shown in SEQ ID NO: 127;

14) a heavy chain comprising the amino acid sequence as shown in SEQ ID NO: 119, and a light chain comprising the amino acid sequence as shown in SEQ ID NO: 128;

15) a heavy chain comprising the amino acid sequence as shown in SEQ ID NO: 120, and a light chain comprising the amino acid sequence as shown in SEQ ID NO: 129;

16) a heavy chain comprising the amino acid sequence as shown in SEQ ID NO: 121, and a light chain comprising the amino acid sequence as shown in SEQ ID NO: 130;

17) a heavy chain comprising the amino acid sequence as shown in SEQ ID NO: 122, and a light chain comprising the amino acid sequence as shown in SEQ ID NO: 130; or

18) a heavy chain comprising the amino acid sequence as shown in SEQ ID NO: 123, and a light chain comprising the amino acid sequence as shown in SEQ ID NO: 130;

In some embodiments, the anti-CD73 antibody of the invention is an IgGl-type antibody, an IgG2-type antibody, an IgG3-type antibody, or an IgG4-type antibody; preferably, the anti-CD73 antibody is an IgG4-type antibody.

In some embodiments, the anti-CD73 antibody is a monoclonal antibody.

In some embodiments, the anti-CD73 antibody or antigen-binding fragment thereof comprises framework sequences, wherein at least part of the framework sequences are human consensus framework sequences.

In some embodiments, the present application relates to antibodies that compete with the exemplary antibodies disclosed herein for binding to CD73, or antagonize, block the binding of the exemplary antibodies disclosed herein to CD73.

In some embodiments, the anti-CD73 antibody is a chimeric antibody, and in preferred embodiments, the anti-CD73 antibody is humanized. In some embodiments, the anti-CD73 antibody is a human antibody. The anti-CD73 antibodies of the present invention also encompass antibody fragments thereof, preferably antibody fragments selected from the group consisting of: Fab, Fab', Fab'-SH, F(ab')$_2$, Fv, single chain antibody (e.g. scFv), single domain antibody, diabody (dAb) or linear antibody.

In some embodiments, the present invention provides an isolated nucleic acid encoding the anti-CD73 antibody or antigen-binding fragment thereof of the present invention, a vector comprising the nucleic acid, and a host cell comprising the nucleic acid or the vector.

In some embodiments, the present invention provides a method of preparing the anti-CD73 antibody or antigen-binding fragment thereof of the present invention, the method comprising culturing the host cell of the present invention under conditions suitable for expressing the encoding nucleic acid of the present invention. In another embodiment, the present invention provides anti-CD73 antibodies and antigen-binding fragments thereof prepared by the method as described above.

In some embodiments, the present invention provides an immunoconjugate and a pharmaceutical composition and a pharmaceutical combination comprising the anti-CD73 antibody or antigen-binding fragment thereof of the present invention.

In some embodiments, the present invention also provides use of the anti-CD73 antibody or antigen-binding fragment thereof, immunoconjugate, pharmaceutical composition or pharmaceutical combination of the present invention in the manufacture of a medicament for the prevention and/or treatment of CD73-related diseases or disorders (*e.g.* tumors).

In some embodiments, the present invention also provides a method for preventing and/or treating CD73-related diseases or disorders (*e.g.*, tumors), the method comprising administering to a subject an effective amount of the CD73-binding antibody or antigen-binding fragment thereof, immunoconjugate, pharmaceutical composition or pharmaceutical combination of the present invention.

In some embodiments, the pharmaceutical combination of the present invention comprises the above-mentioned anti-CD73 antibody or antigen-binding fragment thereof and an anti-PD1 antibody, and the anti-PD1 antibody is preferably Sintilimab.

In some embodiments, the present invention also provides use of the anti-CD73 antibody or antigen-binding fragment thereof in the manufacture of a medicament for reversing the inhibition of T cell proliferation by CD73. In some embodiments, wherein the T cells are CD4+ T cells, CD8+ T cells.

In some embodiments, the present invention also provides use of the anti-CD73 antibody or antigen-binding fragment thereof in the manufacture of a medicament for activating the T cell activity.

In some embodiments, the present invention also provides an antibody-binding epitope of CD73, comprising a fragment of amino acids 159-170 shown in SEQ ID NO: 131, preferably, the epitope comprises amino acids 159, 161, 162, 163 and 170 shown in SEQ ID NO: 131.

The present invention also relates to a method for detecting the CD73 molecule in a sample, the method comprising (a) contacting the antibody or antigen-binding fragment thereof of the present invention with the sample; and (b) detecting whether a complex is formed by the antibody or antigen-binding fragment thereof and the CD73 molecule in the sample.

The anti-CD73 antibody or antigen-binding fragment thereof of the present invention has the following advantages:

1) binding to human or cynomolgus monkey CD73 with high affinity;

2) completely blocking the enzymatic activity of membrane-bound CD73 on the cell surface;

3) Binding to human CD73 expressed on the surface of CHO-S cells with high affinity;

4) blocking the enzymatic activity of the soluble CD73;

5) reversing the inhibition of CD4+ T cell proliferation by AMP-adenosine;

6) reversing the inhibition of CD8+ T cell proliferation by AMP-adenosine;

7) reversing the inhibition of T cell activity by AMP-adenosine;

8) excellent anti-tumor effect and safety.

## DESCRIPTION OF THE DRAWINGS

Preferred embodiments of the present invention, which are described in detail below, will be better understood when read in combination with the accompanying drawings. For the purpose of illustrating the invention, the presently preferred embodiments are shown in the drawings. It should be understood, however, that the invention is not intended to be limited to the precise arrangements and means of the embodiments shown in the drawings.

Figure 1. Expression of CD73 molecule on the cell surface;

Figure 2. Anti-CD73 antibody molecules inhibit the enzymatic activity of CD73 on the surface of Calu-6 cells;

Figure 3. Anti-CD73 antibody molecules inhibit the enzymatic activity of CD73 on the surface of NCI-H292 cells;

Figure 4. Inhibitory effects of the antibodies of the present invention on the enzymatic activity of soluble CD73;

Figure 5. Anti-CD73 antibodies bind to human CD73 expressed on the surface of CHO-S cells;

Figure 6. Anti-CD73 antibody molecules promote the proliferation activity of CD4+ T cells;

Figure 7. Results of CD73 expression in CD8+ T cells by Flow cytometry;

Figure 8. Anti-CD73 antibody molecules promote the proliferation activity of CD8+ T cells;

Figure 9. Activation of human T cells by anti-CD73 antibodies;

Figure 10. Activation of T cells by the anti-CD73 antibody in combination with an anti-PD-1 antibody;

Figure 11. Tumor inhibition activity of ADI37505 and ADI37506 in MDA-MB-231 tumor-bearing NOG mouse models;

Figure 12. Changes in body weights of mice;

Figure 13. Tumor inhibition rate of ADI37505 and anti-PD-1 antibody in A375 tumor-bearing humanized mouse models.

Figure 14. Changes in body weights of mice for each group.

Figure 15. Binding of anti-CD73 antibody to GS-CHO cells over-expressing full-length human CD73, N-terminal domain and C-terminal domain of CD73 by flow cytometry;

Figure 16. Binding of anti-CD73 antibody to GS-CHO cells over-expressing CD73 N-terminal mutant by flow cytometry.

## DETAILED DESCRIPTION OF THE INVENTION

### I. Definitions

Before describing the present invention in detail below, it should be understood that the present invention is not limited to the specific methodology, protocols, and reagents described herein, as these may vary. It should also be understood that the terms used herein are only for the purpose of describing specific embodiments, but not intended to limit the scope of the present invention, which will only be limited by the appended claims. Unless otherwise defined, all technical and scientific terms used herein have the same meaning as commonly understood by those skilled in the art to which the present invention belongs.

For the purpose of interpreting the specification, the following definitions will be used, and the terms used in the singular may also include the plural, and vice versa, if appropriate. It is to be understood that the terminology used herein is for the purpose of describing particular embodiments only and is not intended to be limiting.

The term "about" when used in connection with a numerical value is meant to encompass numerical values within the range between the lower limit of 5% less than the specified numerical value and the upper limit of 5% greater than the specified numerical value.

The term "and/or", when used in conjunction with two or more alternatives, should be understood to mean any one of the alternatives or any two or more of the alternatives.

The term "comprising" or "including" means to include the stated elements, integers or steps, but does not exclude any other elements, integers or steps. When the term "comprising" or "including" is used herein, unless otherwise specified, it also encompasses the situation of consisting of the stated elements, integers or steps. For example, when referring to an antibody variable region "comprising" a specific sequence, it is also intended to encompass an antibody variable region consisting of the specific sequence.

The term "antibody" is used in the broadest sense herein and encompasses a variety of antibody structures, including but not limited to monoclonal antibodies, polyclonal antibodies, recombinant antibodies, humanized antibodies, chimeric antibodies, multispecific antibodies (e.g., bispecific antibodies), single chain antibodies, intact antibodies or antibody fragments thereof showing desirable antigen-binding activity. An intact antibody will generally contain at least two full-length heavy chains and two full-length light chains, but may include fewer chains in some cases, for example, antibodies naturally occurring in camels may only contain heavy chain.

The terms "full length antibody" and "intact antibody" are used interchangeably herein to refer to an antibody having a structure substantially similar to that of a native antibody or having a heavy chain containing Fc region as defined herein .

The term"antibody fragment" include any portion of the above-mentioned antibodies, preferably antigen-binding fragments thereof or variable regions thereof.

The term "antigen-binding fragment" refers to a molecule other than an intact antibody, such molecule comprises a portion of the intact antibody and binds the antigen to which the intact antibody binds. Examples of antigen-binding fragments include, but are not limited to, Fv, Fab, Fab', Fab'-SH, $F(ab')_2$, diabodies, dAb, linear antibody, single-chain antibodies (e.g., scFv); single-domain antibodies; antigen-binding fragments of bivalent or bispecific antibodies; camelid antibodies; and other fragments with desired antigen (e.g. CD73)-binding ability.

The term "antigen" refers to a molecule that elicits an immune response. This immune response may involve either generation of antibodies or activation of specific immune cells, or both. The skilled artisan will understand that any macromolecule, substantially including any protein or peptide, can be used as an antigen. In addition, antigens can be derived from recombinant or genomic DNAs.

As used herein, the term "epitope" refers to the portion of an antigen (e.g., CD73) that specifically interacts with an antibody molecule.

As used herein, the term "monoclonal antibody" refers to a preparation of antibody molecules having a single molecular composition (i.e., they are produced by the same type of immune cells, all of immune cells are clones of a single parental cell, and thus the molecules are all identical ). Monoclonal antibodies or antigen-binding fragments thereof can be produced, for example, by hybridoma technology, recombinant technology, phage display technology, synthetic technology such as CDR grafting, or a combination of such or other techniques known in the art.

As used herein, the terms "binding" and "specific binding" mean that the binding of the antibody is selective for the antigen and can be distinguished from unwanted or nonspecific interactions. The ability of an antibody to bind to a specific antigen can be determined by Enzyme-Linked Immunosorbent Assay (ELISA), Surface Plasmon Resonance (SPR) or Biofilm Optical Interference Technology (ForteBio) or other conventional binding assays known in the art. As an example of the present invention, it is meant that an antibody or antigen-binding fragment thereof binds to an antigenic epitope by *in vitro* assay, preferably in Biofilm Optical Interference Assay with purified wild-type antigen. In certain embodiments, an antibody or antigen-binding fragment thereof is referred to specifically binding to an antigen, when it preferably recognizes its target antigen in a complex mixture of proteins and/or macromolecules.

Antibodies are divided into "classes" depending on the amino acid sequences of their heavy chain constant region: IgA, IgD, IgE, IgG, and IgM, and several of these classes can be further divided into subclasses, e.g., IgG1, IgG2, IgG3 and IgG4, IgA1 and IgA2. The heavy chain constant regions corresponding to the different classes of antibodies are called $\alpha$, $\delta$, $\varepsilon$, $\gamma$ and $\mu$, respectively. The light chain constant regions (CLs) that can be found in all five antibody classes are called $\kappa$ and $\lambda$. Within full-length light and heavy chains, typically the variable and constant regions are joined by a "J" region of about 12 or more amino acids, and the heavy chain also includes a "D" region of about 10 or more amino acids. See, e.g., Fundamental Immunology, Ch. 7 (Paul, W. ed., 2nd ed., Raven Press, NY (1989)) (which is incorporated herein by reference in its entirety for all purposes). The variable regions of each light/heavy chain pair typically form the antigen binding site.

The term "Fc region" is used herein to define the C-terminal region of an immunoglobulin heavy chain, and at least a portion of the constant region is comprised in such region. The term includes naturally occurring sequence of Fc region, or variant Fc region. In certain embodiments, the human IgG heavy chain Fc region generally extends from Cys226 or Pro230 to the carbonyl terminus of the heavy chain. However, the C-terminal lysine (Lys447) of the Fc region may or may not be present. Unless otherwise stated, the numbering of amino acid residues in the Fc region or constant region is according to the EU numbering system, which is also known as the EU index, as described in Kabat et al., Sequences of Proteins of Immunological Interest, 5th Ed. Public Health Service, National Institutes of Health, Bethesda, MD, 1991.

The term "variable region" or "variable domain" refers to the domain of an antibody heavy or light chain that is involved in antibody binding to an antigen. The heavy and light chain variable domains of naturally occurring antibodies generally have similar structures, wherein each domain comprises four conserved framework regions (FRs) and three complementarity determining regions (see, *e.g.,* Kindt et al. Kuby Immunology, 6th ed., WH Freeman and Co. page 91 (2007)). A single VH or VL domain may be sufficient to confer antigen binding specificity. In addition, antibodies that bind to a particular antigen can be isolated by screening libraries of complementary VL or VH domains, respectively with the VH or VL domains from antibodies binding to the antigen, see, *e.g.,* Portolano et al., J. Immunol. 150:880 -887 (1993); Clarkson et al., Nature 352:624-628 (1991).

The variable region is usually composed of three hypervariable regions, also known as complementarity determining regions or CDRs, connected by four relatively conserved framework regions (FRs). The CDRs from the two chains of each pair, which can specifically bind to an epitope, are typically aligned by the framework regions. Both light and heavy chain variable regions generally comprise the domains FR1, CDR1, FR2, CDR2, FR3, CDR3 and FR4 from the N-terminus to the C-terminus.

"Complementarity-determining regions" or "CDR regions" or "CDRs" or "hypervariable regions" (used interchangeably herein with hypervariable regions "HVRs"), are the regions in antibody variable domains, in which the sequences are highly variable and a structurally defined loop ("hypervariable loop") is formed, and/or antigen-contacting residues ("antigen-contacting points") are included. The CDRs are mainly responsible for binding to antigenic epitopes. The CDRs of the heavy and light chains are numbered sequentially from the N-terminus and are commonly referred to as CDR1, CDR2 and CDR3. The CDRs located within the antibody heavy chain variable domain are also referred to as HCDR1, HCDR2 and HCDR3, while the CDRs located within the antibody light chain variable domain are referred to as LCDR1, LCDR2 and LCDR3. CDR sequences can be determined from the amino acid sequences in a given light or heavy chain variable region by using various schemes well known in the art, such as Chothia based on the three-dimensional structure of the antibody and the topology of the CDR loops. (Chothia et al. (1989) Nature 342: 877-883, Al-Lazikani et al. "Standard conformations for the canonical structures of immunoglobulins", Journal of Molecular Biology, 273, 927-948 (1997)), Kabat based on the variability of antibody sequences (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (International Immunogenetics Information Systems, World Wide Web imgt.cines.fr/), and North CDR definition based on affinity propagation clustering by using a large number of crystal structures (North et al., "A New Clustering of Antibody CDR Loop Conformations", Journal of Molecular Biology, 406, 228-256 (2011)).

For example, different ranges of CDR regions are defined by Kabat and Chothia scheme, etc.

| CDRs | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia Numbering System) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

CDRs can also be determined based on having the same Kabat numbering positions as the reference CDR sequences.

Unless otherwise stated, in the present invention, the term "CDRs" or "CDR sequences" encompass CDR sequences determined in any of the ways described above.

Unless otherwise stated, in the present invention, when referring to the positions of antibody variable region residues (including both heavy and light chain variable region residues), it refers to the numbering positions according to the Kabat numbering system ( Kabat et al., Sequences of Proteins of Immunological Interest, 5th Ed. Public Health Service, National Institutes of Health, Bethesda, Md. (1991)).

In one embodiment, the boundaries of a HCDR1 are defined in the invention according to AbM scheme, the boundaries of a HCDR3 are defined according to IMGT scheme, and the boundaries of a HCDR2 and LCDR1-3 are defined according to Kabat scheme, *e.g.*, as shown in Table A below. In one embodiment, the boundaries of a CDR1 are defined in the invention according to Kabat scheme, *e.g.*, as shown in Table D below.

However, it should be noted that the CDR boundaries of the same antibody variable region may be varied depending on different assignment systems. That is, the CDR sequences of the same antibody variable region defined according to different assignment systems are different. Thus, when referring to an antibody defined with specific CDR sequences as defined in the present invention, the scope of said antibody also encompasses antibodies, for which said specific CDR sequences are comprised in their variable region sequences, whereas the alleged CDR boundaries are different from the specific CDR boundaries defined by the present invention due to the application of a different scheme (*e.g.* different assignment system criteria or combination).

Antibodies with different specificities (*i.e.*, different binding sites for different antigens) have different CDRs. However, although CDRs vary from antibody to antibody, only a limited number of amino acid positions within CDRs are directly involved in antigen binding. A minimum overlap region can be determined by using at least two of the Kabat, Chothia, AbM, Contact and North methods, thereby providing the "minimum binding unit" for antigen binding. The minimum binding unit can be a sub-portion of CDRs. The rest residues of the CDR sequences can be determined by the structure and protein folding of the antibody, as will be apparent to those skilled in the art. Accordingly, the present invention also contemplates variants of any of the CDRs presented herein. For example, in a CDR variant, the amino acid residues of the minimum binding unit may remain unchanged, while the rest of the CDR residues as defined by Kabat or Chothia may be replaced by conservative amino acid residues.

The term "antibody-dependent cell-mediated cytotoxicity" or "ADCC" means a form of cytotoxicity, wherein the secreted immunoglobulins bind to Fc receptors (FcRs) present on some cytotoxic cells (*e.g.*, NK cells, neutrophils and macrophages), enabling these cytotoxic effector cells to specifically bind to antigen-bearing target cells, and then to kill the target cells by cytotoxin. NK cells, the primary cells mediating ADCC, express $Fc\gamma RIII$ only, whereas monocytes express FcγRI, FcγRII and FcγRIII. FcR expression on hematopoietic cells are summarized in Table 3 on page 464 of Ravetch and Kinet, Annu. Rev. Immunol. 9:457-92 (1991). To assess ADCC activity of a molecule of interest, *in vitro* ADCC assays can be performed, such as those described in US Pat. No. 5,500,362 or 5,821,337 or US Pat. No. 6,737,056. Effector cells applicable for such assays include PBMCs and NK cells. Alternatively or additionally, ADCC activity of a molecule of interest can be assessed *in vivo, e.g.,* in animal models such as those disclosed in Clynes et al., PNAS (USA) 95:652-656 (1998). An exemplary assay for assessing ADCC activity is provided in the Examples herein.

The term "functional Fc region" refers to Fc region that possesses the "effector functions" of Fc region of a naturally occurring sequence. Exemplified "effector functions" include Clq binding; CDC; Fc receptor binding; ADCC; Phagocytosis; downregulation of cell-surface receptors (e.g., B-cell receptors; BCR), etc. For such effector functions, the association of Fc region with a binding domain *(e.g.,* an antibody variable domain) is generally necessary, and such functions can be assessed by using a variety of assays, such as those disclosed herein.

The term "therapeutic agent" as used herein encompasses any substance that is effective in preventing or treating tumors (*e.g.*, cancers), including chemotherapeutic agents, cytotoxic agents, vaccines, other antibodies, anti-infectious agents, small molecule drugs, or immunomodulatory agents.

The term " immunomodulatory agents" as used herein refers to a natural or synthetic active agent or drug that inhibits or modulates an immune response. The immune response can be a humoral response or a cellular response.

The term "effective amount" refers to an amount or dose of an antibody or fragment or conjugate or composition of the invention to produce the desired effect in a patient in need of treatment or prevention after being administered to a patient in single or multiple doses. For therapeutic or prophylactic purposes, an "effective amount" can be distinguished as a "therapeutically effective amount" and a "prophylactically effective amount". An effective amount can be readily determined by the attending physician skilled in the art, by taking into account various factors such as the species, size, age and general health of the mammals, the specific disease involved; the degree or severity of the disease; the response of the individual patient; the specific antibody to be administered; the mode of administration; the bioavailability profile of the preparation to be administered; the selected dosing regimen; and the use of any concomitant therapy.

In one embodiment, an effective amount of the CD73 antibody of the invention preferably inhibits a measurable parameter (*e.g.*, tumor growth rate, tumor volume, etc.) by at least about 20%, more preferably at least about 40%, even more preferably at least about 50%, 60% or 70% and still more preferably at least about 80% or 90%, compared to a control.

The terms "host cell", "host cell line" and "host cell culture" are used interchangeably and refer to cells into which exogenous nucleic acid has been introduced, including the progeny of such cells. Host cells include "transformants" and "transformed cells," which include the original primary transformed cells and progeny derived therefrom, regardless of the number of passages. Progeny may not be identical in nucleic acid content to the parent cell, but may contain mutations. Included herein are mutant progeny screened or selected for the same function or biological activity in the originally transformed cell.

As used herein, the term "multispecific" antibody refers to an antibody having at least two different antigen-binding sites, each of which binds to a different epitope of the same antigen or binds to a different epitope of different antigen. Multispecific antibodies are antibodies that have binding specificity for at least two different antigenic epitopes. In one embodiment, provided herein are bispecific antibodies that have binding specificity for a first antigen and a second antigen.

The term "effector function" refers to those biological activities attributable to the Fc region of an immunoglobulin that vary with the immunoglobulin isotype. Examples of immunoglobulin effector functions include: C1q binding and complement-dependent cytotoxicity (CDC), Fc receptor binding, antibody-dependent cell-mediated cytotoxicity (ADCC), antibody-dependent phagocytosis (ADCP), cytokine secretion, immune complex-mediated antigen uptake by antigen-presenting cells, downregulation of cell surface receptors (*e.g.*, B cell receptors), and B cell activation.

The term "cytokine" is a generic term for proteins released by a population of cells as intercellular mediators acting on another cell. Examples of such cytokines are lymphokines, monokines, interleukins (IL) such as IL-1, IL-1α, IL-2, IL-3, IL-4, IL-5, IL-6, IL- 7, IL-8, IL-9, IL-11, IL-12, IL-15; tumor necrosis factors, such as TNF-α or TNF-β; and other polypeptide factors, including LIF and kit ligand (KL) and Gamma-interferon. As used herein, the term cytokine includes proteins from natural sources or from recombinant cell cultures and biologically active equivalents of cytokines with native sequence, including synthetically produced small molecule entities, and pharmaceutically acceptable derivatives and salts thereof.

The term "chimeric antibody" is an antibody molecule in which (a) the constant regions or portions thereof are altered, replaced or exchanged such that the antigen binding site is connected to constant regions with a different or altered class, effector function and/or from different species, or connected to disparate molecules (*e.g.*, enzymes, toxins, hormones, growth factors, drugs) etc. that confer novel properties to the chimeric antibody; or (b) the variable regions or portions thereof are altered, replaced or exchanged with variable regions having different or altered antigen specificities. For example, mouse antibodies can be modified by replacing their constant regions with those from human immunoglobulins. Due to the replacement of human constant regions, the chimeric antibody can retain its specificity in recognizing the antigen while having reduced antigenicity in humans as compared to the original mouse antibody.

The term "human antibody" refers to an antibody having an amino acid sequence that corresponds to the amino acid sequence of an antibody produced by human or human cells or derived from a non-human source utilizing the human antibody library or other human antibody coding sequence. Such definition for human antibody specifically excludes humanized antibodies that contain non-human antigen-binding residue(s).

The term "humanized" antibody refers to a chimeric antibody comprising amino acid residues from non-human CDRs and amino acid residues from human FRs. In some embodiments, a humanized antibody will comprise substantially all of at least one, usually two, variable domains, wherein all or substantially all of the CDRs (*e.g.*, 6 CDRs) are corresponding to those of the non-human antibody, and all or substantially all of the FRs are corresponding to those of the human antibody. A humanized antibody may optionally contain at least a portion of an antibody constant region derived from a human antibody. The "humanized form" of an antibody (*e.g.*, a non-human antibody) refers to an antibody that has been humanized.

The term "immunoconjugate" refers to an antibody conjugated to one or more other substances, including but not limited to cytotoxic agents or labels.

The term "label" as used herein refers to a compound or composition that is directly or indirectly conjugated or fused to an agent, such as a polynucleotide probe or an antibody, and facilitates detection of the agent to which it is conjugated or fused. The label can itself be detectable (*e.g.*, a radioisotope label or a fluorescent label) or, in the case of an enzymatic label, it can catalyze a detectable chemical change of a substrate compound or composition. The term is intended to encompass direct labeling of a probe or antibody by coupling (*i.e.*, physically linking) a detectable substance to the probe or antibody and indirect labeling of a probe or antibody by reaction with another reagent that is directly labeled. Examples of indirect labeling include detection of primary antibodies using fluorescently labeled secondary antibodies and end-labeling of DNA probes with biotin so that they can be detected with fluorescently labeled streptavidin.

The term "individual" or "subject" includes mammals. Mammals include, but are not limited to, domestic animals (*e.g.*, cattle, sheep, cats, dogs, and horses), primates (*e.g.*, humans and non-human primates such as monkeys), rabbits, and rodents (*e.g.*, mice and rats). In some embodiments, the individual or subject is a human.

The term "isolated" antibody is an antibody that has been separated from components of its natural environment. In some embodiments, an antibody is purified to greater than 95% or 99% purity as determined by, for example, electrophoretic (*e.g.*, SDS-PAGE, isoelectric focusing (IEF), capillary electrophoresis) or chromatography (*e.g.*, ion exchange or reverse phase HPLC). For a review of methods for assessing the purity of an antibody, see, for example, Flatman et al., J. Chromatogr. B848:79-87 (2007).

"An isolated nucleic acid encoding an anti-CD73 antibody or antigen-binding fragment thereof" refers to one or more nucleic acid molecules encoding an antibody heavy or light chain (or antigen-binding fragment thereof), including nucleic acid molecules in a single vector or in separate vectors, as well as such nucleic acid molecules present at one or more locations in a host cell.

The sequence identity between sequences is calculated as follows.

To determine the percent identity of two amino acid sequences or two nucleic acid sequences, the sequences are aligned for optimal comparison purposes (*e.g.*, gaps can be introduced within one or both of the first and second amino acid sequences or nucleic acid sequences for optimal alignment, or non-homologous sequences can be discarded for comparison purposes). In a preferred embodiment, the length of the reference sequences to be aligned for comparison purposes is at least 30%, preferably at least 40%, more preferably at least 50%, 60% and even more preferably at least 70%, 80%, 90%, 100% of the reference sequence length. The amino acid residues or nucleotides at corresponding amino acid positions or nucleotide positions are then compared. The molecules are identical at a position when the position of the first sequence is occupied by the same amino acid residue or nucleotide as the one at the corresponding position in the second sequence.

Sequence comparison and percent identity calculation between two sequences can be accomplished using mathematical algorithms. In a preferred embodiment, percent identity between two amino acid sequences can be determined by the Needlema and Wunsch ((1970) J. Mol. Biol. 48:444-453) algorithm which has been integrated into the GAP program in the GCG software package (available at http://www.gcg.com), by using the Blossum 62 matrix or the PAM250 matrix and gap weight of 16, 14, 12, 10, 8, 6, or 4 and length weight of 1, 2, 3, 4, 5, or 6. In yet another preferred embodiment, percent identity between two nucleotide sequences can be determined by the GAP program in the GCG software package (available at http://www.gcg.com), by using the NWSgapdna.CMP matrix and gap weight of 40, 50, 60, 70 or 80 and length weight of 1, 2, 3, 4, 5, or 6. A particularly preferred set of parameters (and one that should be used unless otherwise indicated) is a Blossum 62 scoring matrix with a gap penalty of 12, a gap extension penalty of 4, and a shift gap penalty of 5.

It is also possible to determine the percent identity between two amino acid sequences or nucleotide sequences by the PAM120 Table of weighted residuals, gap length penalty of 12, gap penalty of 4, by using the E. Meyers and W. Miller algorithm that has been incorporated into the ALIGN program (version 2.0) ((1989) CABIOS, 4:11- 17) ).

Additionally or alternatively, the nucleic acid sequences and protein sequences described herein can be further used as "query sequences" to perform searches against public databases, e.g., to identify other family member sequences or related sequences.

The term "pharmaceutical excipients" refers to a diluent, adjuvant (e.g., Freund's adjuvant (complete and incomplete)), vehicle, carrier or stabilizer, etc., administered along with the active substance.

The term "pharmaceutical composition" refers to a composition that is in a form that allows the biological activity of the active ingredients contained therein to be effective and does not contain additional ingredients which are unacceptably toxic to a subject who would be administered with the composition.

The term "pharmaceutical combination" refers to a non-fixed combination product or a fixed combination product, including but not limited to kits, pharmaceutical compositions. The term "non-fixed combination" means that as separate entities, the active ingredients (e.g., (i) an anti-CD73 antibody or fragment thereof, and (ii) other therapeutic agents) are administered simultaneously, sequentially without specific time constraints, or at the same or different time intervals, administered to a patient, wherein such administration provides prophylactically or therapeutically effective levels of two or more active agents in the patient. In some embodiments, the anti-CD73 antibody or fragment thereof and other therapeutic agent used in the pharmaceutical combination are administered at levels no greater than when they are used alone. The term "fixed combination" means that the two or more active agents are administered to a patient simultaneously in the form of a single entity. The doses and/or time intervals of the two or more active agents are preferably selected so that the combination use of each part produces a greater effect in the treatment of a disease or condition than either component alone can achieve. Each ingredient may be in the same or different individual formulations.

The term "combination therapy" or "combining therapy" refers to the administration of two or more therapeutic agents to treat cancer or infection as described in the present disclosure. Such administration includes co-administration of these therapeutic agents in a substantially simultaneous manner, e.g., in a single capsule having a fixed ratio of active ingredients. Alternatively, such administration includes co-administration of the individual active ingredients in multiple or separate containers such as tablets, capsules, powders and liquids. The powders and/or liquids can be reconstituted or diluted to the desired dose prior to administration. In addition, such administration also includes use of each type of therapeutic agent in a sequential mode at approximately the same time or at different times. In either case, the treatment regimen will provide the beneficial effect of the drug combination in the treatment of the disorders or conditions described herein.

As used herein, "treatment" refers to slowing, interrupting, blocking, alleviating, stopping, reducing, or reversing the progression or severity of an existing symptom, disorder, condition, or disease.

As used herein, "prevention" includes the inhibition of the occurrence or progression of a disease or disorder or symptoms of a particular disease or disorder. In some embodiments, subjects with a family history of cancer are candidates for preventive regimens. Generally, in the context of cancer, the term "prevention" refers to the administration of a drug prior to the onset of signs or symptoms of cancer, particularly in subjects at risk of cancer.

The term "anti-infectious active agent" includes any molecule that specifically inhibits or eliminates the growth of microorganisms, such as viruses, bacteria, fungi, or protozoa, e.g., parasites, at an administration concentration and interval, but is not lethal to the host. As used herein, the term anti-infectious active agent includes antibiotics, antibacterial agents, antiviral agents, antifungal agents, and antiprotozoal agents. In a specific aspect, the anti-infectious active agent is nontoxic to the host at the administration concentration and interval.

Antibacterial anti-infectious active agents or antibacterial agents can be broadly classified as bactericidal (i.e., killing directly) or bacteriostatic (i.e., stopping division). Antibacterial anti-infectious active agents can be further sub-classified as narrow-spectrum antimicrobials (i.e., affecting only a small group of bacterial subtypes, *e.g.*, Gram-negative, *etc.*) or broad-spectrum antimicrobials (i.e., affecting a broad range of species).

The term "vector," as used herein, refers to a nucleic acid molecule capable of propagating another nucleic acid to which it is linked. The term includes vectors as self-replicating nucleic acid structure as well as vectors incorporated into the genome of a host cell into which they has been introduced. Certain vectors are capable of directing the expression of nucleic acids to which they are operatively linked. Such vectors are referred to herein as "expression vectors."

The term "subject/patient sample" refers to a collection of tissue or cell samples obtained from a patient or subject. The source of the tissue or cell sample can be solid tissue (*e.g.* from a fresh, frozen and/or preserved organ or tissue sample or biopsy or aspiration biopsy); blood or any blood component; body fluids (such as cerebrospinal fluid, amniotic fluid, peritoneal fluid (ascites), or interstitial fluid; cells from any time of pregnancy or development of the subject. Tissue samples may contain compounds that are not naturally mixed with the tissue in nature, such as preservatives, anticoagulants, buffers, fixatives, nutrients, antibiotics, and the like. Examples of tumor samples herein include, but are not limited to, tumor biopsy, fine needle aspirate, bronchial lavage, pleural fluid, sputum, urine, surgical specimen, circulating tumor cell, serum, plasma, circulating plasma protein, ascites, primary cell culture or cell line derived from tumors or exhibiting tumor-like properties, and preserved tumor samples such as formalin-fixed, paraffin-embedded tumor samples, or frozen tumor samples.

## II. Antibodies

Unless otherwise specified, the terms "Cluster of Differentiation 73", "extracellular-5'-nucleotidase", "extracellular-5'-NT" and "CD73" as used herein refer to any native CD73 derived from any vertebrate source (including mammals such as primates (*e.g.*, humans) and rodents (e.g., mice and rats)), unless otherwise specified. The term encompasses "full-length" unprocessed CD73 as well as any form of CD73 or any fragment thereof produced by intracellular processing. The term also includes naturally-occurring variants of CD73, *e.g.*, splice variants or allelic variants. In some embodiments, the CD73 is human or cynomolgus CD73.

The term "anti-CD73 antibody, "anti-CD73", "CD73 antibody" or "anti-CD73 antibody" as used herein refers to an antibody or antigen-binding fragment thereof, capable of binding to the CD73 protein with sufficient affinity. The antibodies can be used as diagnostic and/or therapeutic agents targeting CD73. In one embodiment, the anti-CD73 antibodies bind to non-CD73 proteins at an extent less than about 10% of its binding to CD73 protein, as measured, for example, by radioimmunoassay (RIA) or optical interferometry of biofilm layers (*e.g.*, Fortebio affinity assay) or MSD (Meso Scale Discovery) assay.

In some embodiments, the anti-CD73 antibodies or antigen-binding fragments thereof of the invention bind to CD73 (*e.g.*, human or cynomolgus monkey CD73) with a sufficient affinity, *e.g.,* bind to CD73 with an equilibrium dissociation constant ($K_D$) $\leq 1\mu M$, $\leq 100nM$, $\leq 10nM$, $\leq 1nM$, $<0.1nM$, $\leq 0.01nM$, or $\leq 0.001nM$ (*e.g.* $10^{-8}M$ or less, such as from $10^{-8}M$ to $10^{-13}M$, for example from $10^{-9}M$ to $10^{-13}M$). In some embodiments, CD73 is human or cynomolgus CD73. In some embodiments, antibody binding affinity is determined by using Biofilm Optical Interferometry.

In some embodiments, the antibodies or antigen-binding fragments thereof of the invention bind to CD73 expressed on the cell surface and inhibit the enzymatic function of CD73. In some embodiments, the antibodies or antigen-binding fragments thereof of the invention bind to soluble CD73 molecules and inhibit the enzymatic function of CD73.

In some embodiments, the antibodies or antigen-binding fragments thereof of the invention can reverse the inhibition of CD4+ T cell proliferation caused by accumulation of adenosine. In some embodiments, the antibodies or antigen-binding fragments thereof of the invention can completely reverse the inhibition of CD4+ T cell proliferation caused by accumulation of adenosine.

In some embodiments, the antibodies or antigen-binding fragments thereof of the invention can reverse the inhibition of CD8+ T cell proliferation caused by accumulation of adenosine. In some embodiments, the antibodies or antigen-binding fragments thereof of the invention can completely reverse the inhibition of CD8+ T cell proliferation caused by accumulation of adenosine.

In some embodiments, the antibodies or antigen-binding fragments thereof of the invention can reverse the inhibition of T cell activation caused by accumulation of adenosine.

In some embodiments, the CD73-binding antibody or antigen-binding fragment thereof of the invention comprises a heavy chain variable region (VH) and/or a light chain variable region (VL), wherein the VH and VL comprise a combination of six CDRs selected from those as shown in Table A or Table D.

In a preferred embodiment, the combination of HCDR1, HCDR2, HCDR3, LCDR1, LCDR2 and LCDR3 comprised in the CD73-binding antibody or antigen-binding fragment thereof provided by the present invention is shown in Table A or Table D.

In one embodiment of the invention, the amino acid change described herein includes amino acid substitution, insertion or deletion. Preferably, the amino acid change described herein is amino acid substitution, preferably conservative substitution.

In preferred embodiments, the amino acid change described herein occurs in regions outside the CDRs (e.g., in FRs). More preferably, the amino acid change described in the present invention occurs in regions outside the heavy chain variable region and/or outside the light chain variable region.

In some embodiments, the substitution is conservative substitution. Conservative substitution refers to the substitution of an amino acid by another amino acid belonging to the same category, for example, an acidic amino acid is substituted by another acidic amino acid, a basic amino acid is substituted by another basic amino acid, or a neutral amino acid is substituted by another neutral amino acid. Exemplary substitutions are shown in the following table:

| Original residue | Exemplary substitution | Preferred conservative amino acid substitution |
|---|---|---|
| Ala (A) | Val, Leu, Ile | Val |
| Arg (R) | Lys, Gln, Asn | Lys |
| Asn (N) | Gln, His, Asp, Lys, Arg | Gin |
| Asp (D) | Glu, Asn | Glu |
| Cys (C) | Ser, Ala | Ser |
| Gin (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Norleucine | Leu |

In some embodiments, the substitution occurs in the CDR regions of the antibody. Typically, the resulting variant has been modified (*e.g.*, improved) in some biological properties (*e.g.*, increased affinity) relative to the parent antibody and/or will have some substantially retained parent antibody's biological properties. Exemplary substitution variants are affinity matured antibodies.

In certain embodiments, the antibodies provided herein have been altered to increase or decrease the degree of the antibody's glycosylation. Addition or deletion of glycosylation sites to an antibody is conveniently accomplished by altering the amino acid sequence so as to create or remove one or more glycosylation sites. When an antibody contains Fc region, the carbohydrate attached to it can be altered. In some applications, modifications to remove unwanted glycosylation sites may be useful, such as removal of fucose moieties to enhance antibody-dependent cell-mediated cytotoxicity (ADCC) function (see Shield et al. (2002) ) JBC277:26733). In other applications, galactosylation modifications can be made to modify complement-dependent cytotoxicity (CDC).

In certain embodiments, one or more amino acid modifications can be introduced into the Fc region of the antibodies provided herein, thereby generating Fc region variants. Fc region variants can include human Fc region sequences (*e.g.*, human IgG1, IgG2, IgG3, or IgG4 Fc regions) comprising amino acid modifications (*e.g.*, substitutions) at one or more amino acid positions. The examples of Fc variants can be found in US Pat. No. 7,332,581, US Pat. No. 6,737,056, US Pat. No. 6,737,056; WO 2004/056312 and Shields et al., J. Biol. Chem. 9(2):6591-6604 (2001), US Pat. No. 6,194,551, WO 99/51642 and Idusogie et al. J. Immunol. 164: 4178-4184 (2000), US Pat. No. 7,371,826, Duncan & Winter, Nature 322: 738-40 (1988); US Pat. No. 5,648,260; US Pat. No. 5,624,821; and WO 94/29351.

In some embodiments, the Fc region of the antibodies provided by the present invention has point mutations S228, F234 and/or L235, preferably S228P, F234A and/or L235A.

In some embodiments, it may be desirable to generate cysteine-engineered antibody, *e.g.*, "thioMAbs," in which one or more residues of the antibody are replaced with cysteine residues. Cysteine-engineered antibodies can be generated as described, *e.g.*, in US Pat. No. 7,521,541.

In some embodiments, the antibodies provided herein can be further modified to contain other non-proteinaceous moieties known and readily available in the art . Moieties suitable for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, dextran, polyvinyl alcohol, polyvinylpyrrolidone, poly-1,3-dioxane, poly-1,3,6-trioxane, ethylene/maleic anhydride copolymer, polyamino acid (homopolymers or random copolymers), and dextran or poly(n-ethylene pyrrolidone) polyethylene glycol, propylene glycol homopolymer, polypropylene oxide/ethylene oxide copolymer, polyoxyethylated polyol (e.g., glycerol), polyvinyl alcohol, and mixtures thereof.

### III. Nucleic acids of the invention and vectors and host cells containing the same

In one aspect, the present invention provides nucleic acids encoding any of the above anti-CD73 antibodies or antigen-binding fragments thereof. Exemplary nucleic acid sequences encoding the antibody heavy chain variable region include nucleic acid sequence having at least 90%, 91%, 92%, 93%, 94%, 95%, 96%, 97%, 98% or 99% identity to any one of SEQ ID NOs: 73-90, or include nucleic acid sequence selected from any one of SEQ ID Nos: 73-90. Exemplary nucleic acid sequences encoding the antibody light chain variable region include nucleic acid sequence having at least 90%, 91%, 92%, 93%, 94%, 95%, 96%, 97%, 98% or 99% identity to any one of SEQ ID NOs: 98-105, or include nucleic acid sequence selected from any one of SEQ ID Nos: 98-105. The invention also encompasses nucleic acids that hybridize to the above-described nucleic acids under stringent conditions or have one or more substitutions (*e.g.*, conservative substitutions), deletions or insertions compared to the above-described nucleic acids.

In one embodiment, the present invention provides one or more vectors comprising the nucleic acid. In one embodiment, the vector is an expression vector, such as a eukaryotic expression vector. Vectors include, but are not limited to, viruses, plasmids, cosmids, lambda phages, or yeast artificial chromosomes (YACs). In one embodiment, the vector is pV120 vector.

Once the expression vector or DNA sequence for expression has been prepared, the expression vector can be transfected or introduced into a suitable host cell. This can be achieved by various techniques, *e.g.*, protoplast fusion, calcium phosphate precipitation, electroporation, retroviral transduction, viral transfection, genegun, lipid-based transfection, or other conventional techniques. In the case of protoplast fusion, cells are grown in culture medium and screened for appropriate activity. Methods and conditions for culturing the resulting transfected cells and for recovering the resulting antibody molecules are known to those skilled in the art and can be based on methods in this specification and known in the prior art, depending on the particular expression vector used and mammalian host cell modification or optimization.

Additionally, cells with DNAs stably incorporated into their chromosomes can be selected by introducing one or more markers that allow selection of the transfected host cells. Markers can, for example, provide prototrophy, biocide resistance (*e.g.*, antibiotics), or heavy metal (*e.g.*, copper) resistance, etc. to an auxotrophic host. The selectable marker gene can introduced into the same cell with the DNA sequence to be expressed by direct ligation or by co-transformation. Additional elements may also be required for optimal mRNA synthesis. These elements can include splicing signals, as well as transcriptional promoters, enhancers, and termination signals.

In one embodiment, the present invention provides a host cell comprising the nucleic acid or the vector. Suitable host cells for cloning or expressing the nucleic acid encoding the antibody or the vector include prokaryotic or eukaryotic cells as described herein. In one embodiment, the host cell is eukaryotic. In another embodiment, the host cell is selected from yeast cells, mammalian cells (e.g., CHO cells or HEK 293 cells) or other cells suitable for the production of antibodies or antigen-binding fragments thereof.

### IV. Production and purification of antibody molecules of the invention

In one embodiment, the present invention provides a method of preparing the anti-CD73 antibody or fragment thereof (preferably antigen-binding fragment), wherein the method comprises culturing the host cell under a condition suitable for expressing the nucleic acid encoding the antibody or fragment thereof (preferably antigen-binding fragment), and optionally isolating the antibody or fragment thereof. In a certain embodiment, the method further comprises recovering the anti-CD73 antibody or fragment thereof from the host cell.

In one embodiment, a method of preparing the CD73-binding antibody is provided, wherein the method comprises culturing host cells comprising nucleic acid(s) encoding the antibody (*e.g.*, any one polypeptide chain and/or multiple polypeptide chains) or expression vector(s) comprising the nucleic acid(s) under a condition suitable for expression of the antibody, and optionally recovering the antibody from the host cell (or the culture medium for the host cell).

For recombinant production of the CD73-binding antibody, nucleic acid(s) encoding the antibody (*e.g.*, the antibody as described above, *e.g.*, any one polypeptide chain and/or multiple polypeptide chains) is(are) isolated and inserted into one or more vectors for use in further cloning and/or expression in host cells. Such nucleic acids are readily isolated and sequenced using conventional procedures (*e.g.*, by using oligonucleotide probes capable of binding specifically to genes encoding the antibody heavy and light chains).

Antibody molecules prepared as described herein can be purified by techniques known prior art, such as high performance liquid chromatography, ion exchange chromatography, gel electrophoresis, affinity chromatography, size exclusion chromatography, and the like. The actual conditions used to purify a particular protein will also be dependent on factors such as net charge, hydrophobicity, hydrophilicity, etc., and these will be apparent to those skilled in the art. The purity of the antibody molecules of the invention can be determined by any of a variety of well-known analytical methods, including size exclusion chromatography, gel electrophoresis, high performance liquid chromatography, and the like.

## V. Assays

CD73-binding antibodies provided herein can be identified, screened, or characterized for their physical/chemical properties and/or biological activity by a variety of assays known in the art. In one aspect, the antibodies of the invention are tested for their antigen-binding activity, *e.g.*, by known methods such as ELISA, Western blotting, and the like. The binding to CD73 can be determined using methods known in the art, exemplary methods are disclosed herein. In some embodiments, Biofilm Layer Optical Interferometry or MSD assay is used.

It will be appreciated that any of the above assays can be performed with the immunoconjugates of the invention in place of or in addition to the CD73-binding antibodies.

It will be appreciated that any of the above assays can be performed with CD73-binding antibodies and other active agents.

## VI. Immunoconjugates

In some embodiments, the present invention provides immunoconjugates comprising any of the anti-CD73 antibodies and other substances provided herein. In some embodiments, other substances are *e.g.*, therapeutic agents, such as cytotoxic or chemotherapeutic agents. Cytotoxic agents include any agent that is detrimental to cells. Examples of cytotoxic agents suitable for forming immunoconjugates are known in the art.

In some embodiments, the immunoconjugate is used for the prevention or treatment of tumors. In some embodiments, the tumor is cancer.

## VII. Pharmaceutical Compositions

In some embodiments, the present invention provides a composition comprising any of the anti-CD73 antibody described herein, or antigen-binding fragment thereof, or immunoconjugate thereof, preferably the composition is a pharmaceutical composition. In one embodiment, the composition further comprises a pharmaceutical excipients. In one embodiment, the composition (*e.g.*, the pharmaceutical composition) comprises the anti-CD73 antibody or antigen-binding fragment or immunoconjugate thereof of the invention, and a combination of one or more other therapeutic agents (e.g., chemotherapeutic agents, cytotoxic agents, vaccines, other antibodies, anti-infectious active agents, small molecule drugs or immunomodulators).

In some embodiments, the composition is used to prevent or treat cancer. In one embodiment, the cancer is breast cancer, lung cancer.

The present invention also includes a composition (including a pharmaceutical composition) comprising the anti-CD73 antibody or immunoconjugate thereof and a composition (including a pharmaceutical composition) comprising the polynucleotide encoding the anti-CD73 antibody. In certain embodiments, the composition comprises one or more anti-CD73 antibodies or antigen-binding fragments thereof or one or more polynucleotides encoding one or more anti-CD73 antibodies or antigen-binding fragments thereof. These compositions may also contain suitable pharmaceutical excipients, such as pharmaceutical carriers and pharmaceutical vehicles known in the art. For the use of pharmaceutical excipients, see, for example, "Handbook of Pharmaceutical Excipients", Fifth Edition, R.C. Rowe, P. J. Seskey and S.C. Owen, Pharmaceutical Press, London, Chicago.

The pharmaceutical composition of the present invention may also contain one or more other active ingredients as required for the particular indication being treated, preferably may contain those active ingredients that do not adversely affect the activity of each other. For example, it would be desirable to also provide other anti-cancer active ingredients such as chemotherapeutic agents, cytotoxic agents, vaccines, other antibodies, anti-infectious active agents, small molecule drugs or immunomodulators, and the like. The active ingredients are suitably present in a combination in amounts effective for the intended application.

**VIII. Pharmaceutical combination**

The present invention provides the pharmaceutical combination of the present invention as described above, which comprises the anti-CD73 antibody or antigen-binding fragment thereof and an anti-PD1 antibody, further the anti-PD1 antibody is sintilimab.
The present invention provides use of the pharmaceutical combination of the present invention as described above in the preparation of a medicament for treating cancers or tumors.
The cancers or tumors of the present invention include breast cancer, lung cancer and melanoma.
The pharmaceutical combination of the present invention may be used in combination simultaneously, separately or sequentially.
The present invention provides a method of preventing or treating cancers, including administering to an individual in need thereof an effective amount of the pharmaceutical combination of the present invention. The effective amount includes a prophylactically effective amount and a therapeutically effective amount.

**IX Application**

In one aspect, the present invention provides a method of preventing and/or treating CD73-related diseases or disorders (*e.g.*, cancers), comprising administering to a subject an effective amount of the anti-CD73 antibody or antigen-binding fragment, immunoconjugate, or the pharmaceutical composition or combination of the present invention.
The subject may be a mammal, *e.g.*, a primate, preferably a higher order primate, *e.g.,* a human. In one embodiment, the subject has or is at risk of having a disease described herein . In certain embodiments, the subject is receiving or has received other treatments, such as chemotherapy treatment and/or radiation therapy.
In some embodiments, the cancer described herein is, *e.g.*, human breast cancer, lung cancer.
In other aspects, the present invention provides use of the anti-CD73 antibody or antigen-binding fragment, immunoconjugate, pharmaceutical composition or pharmaceutical combination thereof in the production or manufacture of a medicament for the prevention and/or treatment of the CD73-related diseases or disorders described herein.
In some embodiments, the antibody or antigen-binding fragment or immunoconjugate or composition or pharmaceutical combination or product thereof delays the onset of the disorders and/or symptoms associated with the disorders.

**X. Methods and compositions for diagnosis and detection**

In certain embodiments, any of the anti-CD73 antibodies or antigen-binding fragments thereof provided herein can be used to detect the presence of CD73 in a biological sample. As used herein, the term "detection" includes quantitative or qualitative detection, and exemplary detection methods may involve immunohistochemistry, immunocytochemistry, flow cytometry (*e.g.*, FACS), magnetic beads complexed with antibody molecules, ELISA assays, PCR-techniques (*e.g.* RT-PCR). In certain embodiments, the biological sample is blood, serum, or other fluid sample of biological origin. In certain embodiments, the biological sample comprises cells or tissues. In some embodiments, the biological sample is from a hyperproliferative or cancerous lesion.
In one embodiment, a method of detecting the presence or absence of CD73 in a biological sample is provided. In certain embodiments, the CD73 is human CD73. In certain embodiments, the method comprises contacting the biological sample with the anti-CD73 antibody described herein under conditions that allow binding of the antibody to CD73, and detecting whether a complex of the anti-CD73 antibody and CD73 is formed. The method can be an *in vitro* or *in vivo* method.
In some embodiments, a method of treating CD73-related diseases or disorders (*e.g.*, cancers or tumors) is provided, the method comprising: administering to a subject a therapeutically effective amount of the anti-CD73 antibody. In another embodiment, the method further comprises administering to the subject one or more other therapies.

**XI. Exemplary anti-CD73 antibodies of the invention**

Table A. Amino acid sequences of CDRs of exemplary antibodies of the invention

| Antibody name | VH CDR1 (ABM scheme) | VH CDR2 (Kabat scheme) | VH CDR3 (IMGT scheme) | VL CDR1 (Kabat scheme) | VL CDR2 (Kabat scheme) | VL CDR3 (Kabat scheme) |
|---|---|---|---|---|---|---|
| ADI-34071 | GFTFDDYAMH (SEQ ID NO:1) | GISWSSGSIGYADSVKG (SEQ ID NO:16) | AKDRRYGLTYGMDV (SEQ ID NO:31) | QASQDITNYLN (SEQ ID NO:38) | DASNLET (SEQ ID NO: 44) | QQSVVLPFT (SEQ ID NO: 49) |
| ADI-34074 | | | AKGKYLPTGSVDY (SEQ ID NO:32) | RASQSVSSSYLA (SEQ ID NO:39) | GASSRAT (SEQ ID NO: 45) | QQYYDYPPIT (SEQ ID NO: 50) |
| ADI-37496 | GFTFDDFYMH (SEQ ID NO:2) | GISWSSTSIDYADSVKG (SEQ ID NO:17) | | | | |
| ADI-37497 | GFTFDDYFMH (SEQ ID NO:3) | GISWSSGPIDYADSVKG (SEQ ID NO: 18) | | | | |
| ADI-34086 | GFTFSNYAMS (SEQ ID NO:4) | AISGSGGSTYYADSVKG (SEQ ID NO:19) | ARGGPYGSGSYWDTFDY (SEQ ID NO:33) | RASQSVSSNLA (SEQ ID NO:40) | GASTRAT (SEQ ID NO: 46) | QQYTNHYT (SEQ ID NO: 51) |
| ADI-37503 | | AISGRASSTYYADSVKG (SEQ ID NO:20) | | | | |
| ADI-37504 | GFTFHNYAMS (SEQ ID NO:5) | AISGTGRATYYADSVKG (SEQ ID NO:21) | | | | |
| ADI-37505 | GFTFSNYAMT (SEQ ID NO:6) | AISGRGTATYYADSVKG (SEQ ID NO:22) | | | | |
| ADI-37506 | GFTFSNLAMS (SEQ ID NO:7) | AISGRATSTYYADSVKG (SEQ ID NO:23) | | | | |
| ADI-37507 | GFTFLNYAMA (SEQ ID NO:8) | | | | | |
| ADI-34095 | GFTFSSYGMH (SEQ ID NO:9) | VISYEGSNKYYADSVKG (SEQ ID NO:24) | ARGGARYPIAFDI (SEQ ID NO:34) | RASQDISSWLA (SEQ ID NO:41) | AASSLQS (SEQ ID NO: 47) | QQANDFPIT (SEQ ID NO: 52) |
| ADI-37513 | GFTFNFYGMH (SEQ ID NO: 10) | VINDEGINKYYADSVKG (SEQ ID NO:25) | | | | |
| ADI-37522 | GFTFSHYGMH (SEQ ID NO:11) | VISDEGSNKYYADSVKG (SEQ ID NO:26) | ARGWLRYPIAFDI (SEQ ID NO:35) | | | |

| Antibody name | VH CDR1 (ABM scheme) | VH CDR2 (Kabat scheme) | VH CDR3 (IMGT scheme) | VL CDR1 (Kabat scheme) | VL CDR2 (Kabat scheme) | VL CDR3 (Kabat scheme) |
|---|---|---|---|---|---|---|
| ADI-34101 | GFTFSSYGMH (SEQ ID NO:9) | VISYEGSNKYYADSVKG (SEQ ID NO:24) | ARSRYDWSLFDI (SEQ ID NO: 36) | RASQSVSSSYLA (SEQ ID NO:39) | GASSRAT (SEQ ID NO: 45) | QQYVLYPFT (SEQ ID NO: 53) |
| ADI-37532 | GFTFSSYSMH (SEQ ID NO:12) | HI-VEGSDKYYADSVKG (SEQ ID NO:27) | | GASQSVSSSYLA (SEQ ID NO:42) | | |
| consensus sequence | GFTFX$_1$X$_2$X$_3$X$_4$MX$_5$<br><br>Preferably, X$_1$ is D/S/H/L/N<br>Preferably, X$_2$ is D/N/S/F/H<br>Preferably, X$_3$ is Y/F/L<br>Preferably, X$_4$ is A/Y/F/G/S<br>Preferably, X$_5$ is H/S/T/A<br><br>(SEQ ID NO:132) | X$_1$IX$_2$X$_3$X$_4$X$_5$X$_6$X$_7$X$_8$X$_9$Y ADSVKG<br>Preferably, X$_1$ is G/A/V/H<br><br>Preferably, X$_2$ is S/N/Deletion<br><br>Preferably, X$_3$ is W/G/Y/D/V<br><br>Preferably, X$_4$ is S/R/T/E<br><br>Preferably, X$_5$ is S/G/A<br>Preferably, X$_6$ is G/T/S/R/I<br><br>Preferably, X$_7$ is S/P/A/N/D<br><br>Preferably, X$_8$ is I/T/K<br>Preferably, X$_9$ is G/D/Y (SEQ ID NO:134) | | | | |
| | | | | | | |
| ADI-34104 | GYSISSGYYWG (SEQ ID NO:13) | SIYHSGSTYYNPSLKS (SEQ ID NO:28) | | | | |
| ADI-37542 | GYSISEGYYWG (SEQ ID NO:14) | SIYHTGFTYYNPSLKS (SEQ ID NO:29) | ARASIYTPPPEYFQH (SEQ ID NO:37) | RASQSISSWLA (SEQ ID NO:43) | DASSLES (SEQ ID NO: 48) | QQSRIYST (SEQ ID NO: 54) |
| ADI-37543 | GYSISSGYYWA (SEQ ID NO:15) | SIFHSGFTFYNPSLKS (SEQ ID NO:30) | | | | |

EP 4 169 948 A1

27

| Antibody name | VH CDR1 (ABM scheme) | VH CDR2 (Kabat scheme) | VH CDR3 (IMGT scheme) | VL CDR1 (Kabat scheme) | VL CDR2 (Kabat scheme) | VL CDR3 (Kabat scheme) |
|---|---|---|---|---|---|---|
| consensus sequence | GYSISX$_1$GYYWX$_2$<br>X$_1$ is S/E<br>Preferably, X$_2$ is G/A<br>Preferably, (SEQ ID NO:133) | SIX$_1$HX$_2$GX$_3$TX$_4$YNPSLKS<br><br>Preferably, X$_1$ is Y/F<br><br>Preferably, X$_2$ is S/T<br><br>Preferably, X$_3$ is S/F<br><br>Preferably, X$_4$ is Y/F (SEQ ID NO: 135) | | | | |

Table B. Light chain variable region (VL) and heavy chain variable region (VH) of the exemplary antibodies of the invention

| Antibody name | VH protein | VH DNA | VL protein | VL DNA |
|---|---|---|---|---|
| ADI-3407 1 | EVQLVESGGGLVQPG RSLRLSCAASGFTFDD YAMHWVRQAPGKGL EWVSGISWSSGSIGYA DSVKGRFTISRDNAKN SLYLQMNSLRAEDTA VYYCAKDRRYGLTYG MDVWGQGTTVTVSS (SEQ ID NO:55) | GAAGTGCAGCTGGTGGAGTCTGGGGGAG GCTTGGTACAGCCTGGCAGGTCCCTGAGA CTCTCCTGTGCAGCCTCTGGATTCACCTTT GATGATTATGCCATGCACTGGGTCCGGCA AGCTCCAGGGAAGGGCCTGGAGTGGGTC TCAGGTATTAGTTGGAGTAGTGGTAGCATA GGCTATGCGGACTCTGTGAAGGGCCGATT CACCATCTCCAGAGACAACGCCAAGAACT CCCTGTATCTGCAAATGAACAGTCTGAGA GCTGAGGACACGGCGGTGTACTACTGCGC CAAGGACAGAAGATACGGCTTAACATACG GAATGGACGTATGGGGCCAGGGAACAAC TGTCACCGTCTCCTCA (SEQ ID NO:73) | DIQMTQSPSSLSASVGDR VTITCQASQDITNYLNWY QQKPGKAPKLLIYDASNL ETGVPSRFSGSGSGTDFT FTISSLQPEDIATYYCQQS VVLPFTFGGGTKVEIK (SEQ ID NO:91) | GACATCCAGATGACCCAGTCTCCAT CCTCCCTGTCTGCATCTGTAGGAGA CAGAGTCACCATCACTTGCCAGGCG AGTCAGGACATTACCAACTATTTAAA TTGGTATCAGCAGAAACCAGGGAAA GCCCCTAAGCTCCTGATCTACGATGC ATCCAATTTGGAAACAGGGGTCCCA TCAAGGTTCAGTGGAAGTGGATCTG GGACAGATTTTACTTTCACCATCAGC AGCCTGCAGCCTGAAGATATTGCAA CATATTACTGTCAGCAGTCCGTCGTC CTCCCTTTCACTTTTGGCGGAGGGA CCAAGGTTGAGATCAAA (SEQ ID NO:98) |
| ADI-3407 4 | EVQLVESGGGLVQPG RSLRLSCAASGFTFDD YAMHWVRQAPGKGL EWVSGISWSSGSIGYA DSVKGRFTISRDNAKN SLYLQMNSLRAEDTA VYYCAKGKYLPTGSV DYWGQGTLVTVSS (SEQ ID NO:56) | GAAGTGCAGCTGGTGGAGTCTGGGGGAG GCTTGGTACAGCCTGGCAGGTCCCTGAGA CTCTCCTGTGCAGCCTCTGGATTCACCTTT GATGATTATGCCATGCACTGGGTCCGGCA AGCTCCAGGGAAGGGCCTGGAGTGGGTC TCAGGTATTAGTTGGAGTAGTGGTAGCATA GGCTATGCGGACTCTGTGAAGGGCCGATT CACCATCTCCAGAGACAACGCCAAGAACT CCCTGTATCTGCAAATGAACAGTCTGAGA GCTGAGGACACGGCGGTGTACTACTGCGC AAAGGGCAAGTATCTACCAACCGGAAGC GTGGACTACTGGGGACAGGGTACATTGGT AACCGTCTCCTCA (SEQ ID NO:74) | EIVLTQSPGTLSLSPGERA TLSCRASQSVSSSYLAWY QQKPGQAPRLLIYGASSR ATGIPDRFSGSGSGTDFTL TISRLEPEDFAVYYCQQY YDYPPITFGGGTKVEIK (SEQ ID NO:92) | GAAATTGTGTTGACGCAGTCTCCAG GCACCCTGTCTTTGTCTCCAGGGGA AAGAGCCACCCTCTCCTGCAGGGCC AGTCAGAGTGTTAGCAGCAGCTACT TAGCCTGGTACCAGCAGAAACCTGG CCAGGCTCCCAGGCTCCTCATCTATG GTGCATCCAGCAGGGCCACTGGCAT CCCAGACAGGTTCAGTGGCAGTGG GTCTGGGACAGACTTCACTCTCACC ATCAGCAGACTGGAGCCTGAAGATT TTGCAGTGTATTACTGTCAGCAGTAC TACGACTACCCTCCTATCACTTTTGG CGGAGGGACCAAGGTTGAGATCAA A (SEQ ID NO:99) |
| ADI-3749 6 | EVQLVESGGGLVQPG RSLRLSCAASGFTFDD FYMHWVRQAPGKGL EWVSGISWSSTSIDYA | GAAGTGCAGCTGGTGGAGTCTGGGGGAG GCTTGGTACAGCCTGGCAGGTCCCTGAGA CTCTCCTGTGCAGCCTCTGGATTCACCTTT GATGATTTCTACATGCACTGGGTCCGGCA | | |

| | Amino acid sequence | Nucleotide sequence | | |
|---|---|---|---|---|
| | DSVKGRFTISRDNAKN SLYLQMNSLRAEDTA VYYCAKGKYLPTGSV DYWGQGTLVTVSS (SEQ ID NO:57) | AGCTCCAGGGAAGGGCCTGGAGTGGGTC TCAGGTATTAGTTGGAGTAGTACCAGCATA GACTATGCGGACTCTGTGAAGGGCCGGTT CACCATCTCCAGAGACAACGCCAAGAACT CCCTGTATCTGCAAATGAACAGCCTGAGA GCCGAGGACACGGCGGTGTACTACTGCGC AAAGGGCAAGTATCTACCAACGGAAGC GTGGACTACTGGGGACAGGGTACATTGGT AACCGTCTCCTCA (SEQ ID NO:75) | | |
| ADI-3749 7 | EVQLVESGGGLVQPG GSLRLSCAASGFTFDD YFMHWVRQAPGKGL EWVSGISWSSGPIDYA DSVKGRFTISRDNAKN SLYLQMNSLRAEDTA VYYCAKGKYLPTGSV DYWGQGTLVTVSS (SEQ ID NO:58) | GAGGTGCAGCTGGTTGGAGTCTGGGGGAG GCTTGGTGCAGCCTGGGGGGTCCCTGAGA CTCTCCTGTGCAGCCTCTGGATTCACCTTT GATGATTATTTCATGCACTGGGTCCGGCAA GCTCCAGGGAAGGGCCTGGAGTGGGTCT CAGGTATTAGTTGGAGTAGTGGTCCCATA GACTATGCGGACTCTGTGAAGGGCCGATT CACCATCTCCAGAGACAACGCCAAGAACT CCCTGTATCTGCAAATGAACAGTCTGAGA GCTGAGGACACGGCGGTGTACTACTGCGC AAAGGGCAAGTATCTACCAACGGAAGC GTGGACTACTGGGGACAGGGTACATTGGT AACCGTCTCCTCA (SEQ ID NO:76) | | |
| ADI-3408 6 | EVQLLESGGGLVQPG GSLRLSCAASGFTFSN YAMSWVRQAPGKGL EWVSAISGSGGSTYYA DSVKGRFTISRDNSKN TLYLQMNSLRAEDTA VYYCARGGPYGSGSY WDTFDYWGQGTLVT VSS (SEQ ID NO:59) | GAGGTGCAGCTGTTGGAGTCTGGGGGAG GCTTGGTACAGCCTGGGGGGTCCCTGAGA CTCTCCTGTGCAGCCTCTGGATTCACCTTT AGCAATTATGCCATGAGCTGGGTCCGCCA GGCTCCAGGGAAGGGCCTGGAGTGGGTC TCAGCTATTAGTGGTAGTGGTGGTAGCAC ATACTACGCAGACTCCGTGAAGGGCCGGT TCACCATCTCCAGAGACAATTCCAAGAAC ACGCTGTATCTGCAAATGAACAGCCTGAG AGCCGAGGACACGGCGGTGTACTACTGC GCCAGAGGTGGGCCTTACGGAAGCGGAA GCTACTGGGATACCTTTGATTATTGGGGAC AGGGTACATTGGTCACCGTCTCCTCA | EIVMTQSPATLSVSPGERA TLSCRASQSVSSNLAWY QQKPGQAPRLLIYGASTR ATGIPARFSGSGSGTEFTL TISSLQSEDFAVYYCQQY TNHYTFGGGTKVEIK (SEQ ID NO:93) | GAAATAGTGATGACGCAGTCTCCAG CCACCCTGTCTGTGTCTCCAGGGGA AAGAGCCACCCTCTCCTGCAGGGCC AGTCAGAGTGTTAGCAGCAACTTAG CCTGGTACCAGCAGAAACCTGGCCA GGCTCCCAGGCTCCTCATCTATGGTG CATCCACCAGGGCCACTGGTATCCC AGCCAGGTTCAGTGGCAGTGGGTCT GGGACAGAGTTCACTCTCACCATCA GCAGCCTGCAGTCTGAAGATTTTGC AGTTTATTACTGTCAGCAGTACACC AATCACTACACTTTTGGCGGAGGGA CCAAGGTTGAGATCAAA |

| | Amino acid | Nucleotide |
|---|---|---|
| | | (SEQ ID NO:100) |
| ADI-3750 3 | EVQLLESGGGLVQPGGSLRLSCAASGFTFSNYAMSWVRQAPGKGLEWVSAISGRASSTYYADSVKGRFTISRDNSKNTLYLQMNSLRAEDTAVYYCARGGPYGSGSYWDTFDYWGQGTLVTVSS (SEQ ID NO:60) | GAGGTGCAGCTGTTGGAGTCTGGGGGAGGCTTGGTACAGCCTGGGGGGTCCCTGAGACTCTCCTGTGCAGCCTCTGGATTCACCTTTAGCAATTATGCCATGAGCTGGGTCCGCCAGGCTCCAGGGAAAGGGCTGGAGTGGGTCTCAGCTATTAGTGGACGAGCATCCAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGGTGTGTACTACTGCGCCAGAGGTGGGCCTTACGGAAGCGGAAGCTACTGGGATACCTTTGATTATTGGGGACAGGGTACATTGGTCACCGTCTCCTCA (SEQ ID NO:77) |
| ADI-3750 4 | EVQLLESGGGLVQPGGSLRLSCAASGFTFHNYAMSWVRQAPGKGLEWVSAISGTGRATYYADSVKGRFTISRDNSKNTLYLQMNSLRAEDTAVYYCARGGPYGSGSYWDTFDYWGQGTLVTVSS (SEQ ID NO:61) | GAGGTGCAGCTGTTGGAGTCTGGGGGAGGCTTGGTACAGCCTGGGGGGTCCCTGAGACTCTCCTGTGCAGCCTCTGGATTCACCTTTCACAACTATGCCATGAGCTGGGTCCGCCAGGCTCCAGGGAAAGGGCTGGAGTGGGTCTCAGCTATTAGTGGTACAGGTAGAGCAAACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGGTGTGTACTACTGCGCCAGAGGTGGGCCTTACGGAAGCGGAAGCTACTGGGATACCTTTGATTATTGGGGACAGGGTACATTGGTCACCGTCTCCTCA (SEQ ID NO:78) |
| ADI-3750 5 | QVQLVESGGGLVQPGGSLRLSCAASGFTFSNYAMTWVRQAPGKGLEWVSAISGRGTATYYADSVKGRFTISRDNSKNTLYLQMNSLRAEDTAVYYCARGGPYGSGSYWDTFDYWGQGTLVT | CAGGTGCAGCTGGTGGAGTCTGGGGGAGGCTTGGTACAGCCTGGGGGGTCCCTGAGACTCTCCTGTGCAGCCTCTGGATTCACCTTTAGCAATTATGCCATGACCTGGGTCCGCCAGGCTCCAGGGAAAGGGCTGGAGTGGGTCTCAGCTATTAGTGGTAGAGGTACAGCAACATACTACGCAGACTCCGTGAAGGGCCGATTCACCATCTCCAGAGACAATTCCAAGAAC (SEQ ID NO:79) |

| | | | | |
|---|---|---|---|---|
| | VSS<br>(SEQ ID NO:62) | ACGCTGTATCTGCAAATGAACAGCCTGAG<br>AGCCGAGGACACGGCGGTGTACTACTGC<br>GCCAGAGGTGGGGCCTTACGGAAGCGGAA<br>GCTACTGGGATACCTTTGATTATTGGGGAC<br>AGGGTACATTGGTCACCGTCTCCTCA<br>(SEQ ID NO:80) | | |
| ADI-37506 | EVQLLESGGGLVQPG<br>GSLRLSCAASGFTFSN<br>LAMSWVRQAPGKGL<br>EWVSAISGRATSTYYA<br>DSVKGRFTISRDNSKN<br>TLYLQMNSLRAEDTA<br>VYYCARGGPYGSGSY<br>WDTFDYWGQGTLVT<br>VSS<br>(SEQ ID NO:63) | GAGGTGCAGCTGTTGGAGTCTGGGGGAG<br>GCTTGGTACAGCCTGGGGGGGTCCCTGAGA<br>CTCTCCTGTGCAGCCTCTGGATTCACCTTT<br>AGCAACCTCGCCATGTCCTGGGTCCGCCA<br>GGCTCCAGGGAAGGGGCTGGAGTGGGTC<br>TCAGCTATTAGTGGACGAGCAACAAGCAC<br>ATACTACGCAGACTCCGTGAAGGGCCGGT<br>TCACCATCTCCAGAGACAATTCCAAGAAC<br>ACGCTGTATCTGCAAATGAACAGCCTGAG<br>AGCCGAGGACACGGCGGTGTACTACTGC<br>GCCAGAGGTGGGGCCTTACGGAAGCGGAA<br>GCTACTGGGATACCTTTGATTATTGGGGAC<br>AGGGTACATTGGTCACCGTCTCCTCA<br>(SEQ ID NO:81) | | |
| ADI-37507 | EVQLLESGGGLVQPG<br>GSLRLSCAASGFTFLN<br>YAMAWVRQAPGKGL<br>EWVSAISGRATSTYYA<br>DSVKGRFTISRDNSKN<br>TLYLQMNSLRAEDTA<br>VYYCARGGPYGSGSY<br>WDTFDYWGQGTLVT<br>VSS<br>(SEQ ID NO:64) | GAGGTGCAGCTGTTGGAGTCTGGGGGAG<br>GCTTGGTACAGCCTGGGGGGGTCCCTGAGA<br>CTCTCCTGTGCAGCCTCTGGATTCACCTTT<br>CTCAACTATGCCATGGCCTGGGTCCGCCA<br>GGCTCCAGGGAAGGGGCTGGAGTGGGTC<br>TCAGCTATTAGTGGAAGAGCAACAAGCAC<br>ATACTACGCAGACTCCGTGAAGGGCCGGT<br>TCACCATCTCCAGAGACAATTCCAAGAAC<br>ACGCTGTATCTGCAAATGAACAGCCTGAG<br>AGCCGAGGACACGGCGGTGTACTACTGC<br>GCCAGAGGTGGGGCCTTACGGAAGCGGAA<br>GCTACTGGGATACCTTTGATTATTGGGGAC<br>AGGGTACATTGGTCACCGTCTCCTCA<br>(SEQ ID NO:82) | | |
| ADI-34095 | QVQLVESGGGVVQPG<br>RSLRLSCAASGFTFSS<br>YGMHWVRQAPGKGL | CAGGTGCAGCTGGTGGAGTCTGGGGGAG<br>GCGTGGTCCAGCCTGGGAGGTCCCTGAG<br>ACTCTCCTGTGCAGCGTCTGGATTCACCTT | DIQLTQSPSSVSASVGDR<br>VTITCRASQDISSWLAWY<br>QQKPGKAPKLLIYAASSL | GACATCCAGTTGACCCAGTCTCCAT<br>CTTCCGTGTCTGCATCTGTAGGAGA<br>CAGAGTCACCATCACTTGTCGGGCG |

32

| | | | |
|---|---|---|---|
| | EWVAVISYEGSNKYY ADSVKGRFTISRDNSK NTLYLQMNSLRAEDT AVYYCARGGARYPIA FDIWGQGTMVTVSS (SEQ ID NO:65) | CAGTAGCTATGGCATGCACTGGGTCCGCC AGGCTCCAGGCAAGGGGCTGGAGTGGGT GGCAGTTATATCGTATGAGGGAAGTAATAA ATACTATGCAGACTCCGTGAAGGGCCGAT TCACCATCTCCAGAGACAATTCCAAGAAC ACGCTGTATCTGCAAATGAACAGCCTGAG AGCCGAGGACACGGCGGTGTACTACTGC GCCAGAGGAGGTGCTAGATACCCAATAGC ATTCGACATATGGGGTCAGGGTACAATGG TCACCGTCTCCTCA (SEQ ID NO:83) | QSGVPSRFSGSGSGTDFT LTISSLQPEDFATYYCQQA NDFPITFGGGTKVEIK (SEQ ID NO:94) | AGTCAGGATATTAGCAGCTGGTTAG CCTGGTATCAGCAGAAACCAGGGAA AGCCCCTAAGCTCCTGATCTATGCTG CATCCAGTTTGCAAAGTGGGGTCCC ATCAAGGTTCAGCGGCAGTGGATCT GGGACAGATTTCACTCTCACCATCA GCAGCCTGCAGCCTGAAGATTTTGC AACTTACTACTGTCAGCAGGCAAAT GACTTCCCTATCACTTTTGGCGGAG GGACCAAGGTTGAGATCAAA (SEQ ID NO:101) |
| ADI-3751 3 | EVQLLESGGGVVQPG RSLRLSCAASGFTFNF YGMHWVRQAPGKGL EWVAVINDEGINKYYA DSVKGRFTISRDNSKN TLYLQMNSLRAEDTA VYYCARGGARYPIAF DIWGQGTMVTVSS (SEQ ID NO:66) | GAGGTGCAGCTGTTGGAGTCTGGGGGAG GCGTGGTCCAGCCTGGGAGGTCCCTGAG ACTCTCCTGTGCAGCGTCTGGATTCACCTT CAACTTCTATGGCATGCACTGGGTCCGCC AGGCTCCAGGCAAGGGGCTGGAGTGGGT GGCAGTTATAAACGACGAGGGAATCAATA AATACTATGCAGACTCCGTGAAGGGCCGA TTCACCATCTCCAGAGACAATTCCAAGAA CACGCTGTATCTGCAAATGAACAGCCTGA GAGCCGAGGACACGGCGGTGTACTACTG CGCCAGAGGAGGTGCTAGATACCCAATAG CATTCGACATATGGGGTCAGGGTACAATG GTCACCGTCTCCTCA (SEQ ID NO:84) | | |
| ADI-3752 2 | QVQLVESGGGVVQPG RSLRLSCAASGFTFSH YGMHWVRQAPGKGL EWVAVISDEGSNKYY ADSVKGRFTISRDNSK NTLYLQMNSLRAEDT AVYYCARGWLRYPIA FDIWGQGTMVTVSS (SEQ ID NO:67) | CAGGTGCAGCTGGTGGAGTCTGGGGGAG GCGTGGTCCAGCCTGGGAGGTCCCTGAG ACTCTCCTGTGCAGCGTCTGGATTCACCTT CAGTCACTATGGCATGCACTGGGTCCGCC AGGCTCCAGGCAAGGGGCTGGAGTGGGT GGCAGTTATATCGGACGAGGGATCCAATA AATACTATGCAGACTCCGTGAAGGGCCGA TTCACCATCTCCAGAGACAATTCCAAGAA CACGCTGTATCTGCAAATGAACAGCCTGA GAGCCGAGGACACGGCGGTGTACTACTG CGCCAGAGGATGGCTTAGATACCCAATAG CATTCGACATATGGGGTCAGGGTACAATG | | |

33

EP 4 169 948 A1

| | | GTCACCGTCTCCTCA<br>(SEQ ID NO:85) | | |
|---|---|---|---|---|
| ADI-3410<br>1 | QVQLVESGGGVVQPG<br>RSLRLSCAASGFTFSS<br>YGMHWVRQAPGKGL<br>EWWAVISYEGSNKYY<br>ADSVKGRFTISRDNSK<br>NTLYLQMNSLRAEDT<br>AVYYCARSRYDWSLF<br>DIWGQGTMVTVSS<br>(SEQ ID NO:68) | CAGGTGCAGCTGGTGGAGTCTGGGGGAG<br>GCGTGGTCCAGCCTGGGAGGTCCCTGAG<br>ACTCTCCTGTGCAGCGTCTGGATTCACCTT<br>CAGTAGCTATGGCATGCACTGGGTCCGCC<br>AGGCTCCAGGCAAGGGGCTGGAGTGGGT<br>GGCAGTTATATCGTATGAGGGAAGTAATAA<br>ATACTATGCAGACTCCGTGAAGGGCCGAT<br>TCACCATCTCCAGAGACAATTCCAAGAAC<br>ACGCTGTATCTGCAAATGAACAGCCTGAG<br>AGCCGAGGACACGGCGGTGTACTACTGC<br>GCTAGATCAAGATACGACTGGAGTCTATTC<br>GACATATGGGGTCAGGGTACAATGGTCAC<br>CGTCTCCTCA<br>(SEQ ID NO:86) | EIVLTQSPGTLSLSPGERA<br>TLSCRASQSVSSSYLAWY<br>QQKPGQAPRLLIYGASSR<br>ATGIPDRFSGSGSGTDFTL<br>TISRLEPEDFAVYYCQQY<br>VLYPFTFGGGTKVEIK<br>(SEQ ID NO:95) | GAAATTGTGTTGACGCAGTCTCCAG<br>GCACCCTGTCTTTGTCTCCAGGGGA<br>AAGAGCCACCCTCTCCTGCAGGGCC<br>AGTCAGAGTGTTAGCAGCAGCTACT<br>TAGCCTGGTACCAGCAGAAACCTGG<br>CCAGGCTCCCAGGCTCCTCATCTATG<br>GTGCATCCAGCAGGGCCACTGGCAT<br>CCCAGACAGGTTCAGTGGCAGTGG<br>GTCTGGGACAGACTTCACTCTCACC<br>ATCAGCAGACTGGAGCCTGAAGATT<br>TTGCAGTGTATTACTGTCAGCAGTAC<br>GTCCTCTACCCTTTCACTTTTGGCGG<br>AGGGACCAAGGTTGAGATCAAA<br>(SEQ ID NO:102) |
| ADI-3753<br>2 | QVQLVESGGGVVQPG<br>RSLRLSCAASGFTFSS<br>YSMHWVRQAPGKGL<br>EWWAHIVEGSDKYYA<br>DSVKGRFTISRDNSKN<br>TLYLQMNSLRAEDTA<br>VYYCARSRYDWSLFD<br>IWGQGTMVTVSS<br>(SEQ ID NO:69) | CAGGTGCAGCTGGTGGAGTCTGGGGGAG<br>GCGTGGTCCAGCCTGGGAGGTCCCTGAG<br>ACTCTCCTGTGCAGCGTCTGGATTCACCTT<br>CTCCAGCTATTCCATGCACTGGGTCCGCC<br>AGGCTCCAGGCAAGGGGCTGGAGTGGGT<br>GGCACATATCGTAGAGGGAAGTGACAAAT<br>ACTATGCAGACTCCGTGAAGGGCCGATTC<br>ACCATCTCCAGAGACAATTCCAAGAACAC<br>GCTGTATCTGCAAATGAACAGCCTGAGAG<br>CCGAGGACACGGCGGTGTACTACTGCGCT<br>AGATCAAGATACGACTGGAGTCTATTCGA<br>CATATGGGGTCAGGGTACAATGGTCACCG<br>TCTCCTCA<br>(SEQ ID NO:87) | EIVLTQSPGTLSLSPGERA<br>TLSCGASQSVSSSYLAWY<br>QQKPGQAPRLLIYGASSR<br>ATGIPDRFSGSGSGTDFTL<br>TISRLEPEDFAVYYCQQY<br>VLYPFTFGGGTKVEIK<br>(SEQ ID NO:96) | GAAATTGTGTTGACGCAGTCTCCAG<br>GCACCCTGTCTTTGTCTCCAGGGGA<br>AAGAGCCACCCTCTCCTGCGGGGCC<br>AGTCAGAGTGTTAGCAGCAGCTACT<br>TAGCCTGGTACCAGCAGAAACCTGG<br>CCAGGCTCCCAGGCTCCTCATCTATG<br>GTGCATCCAGCAGGGCCACTGGCAT<br>CCCAGACAGGTTCAGTGGCAGTGG<br>GTCTGGGACAGACTTCACTCTCACC<br>ATCAGCAGACTGGAGCCTGAAGATT<br>TTGCAGTGTATTACTGTCAGCAGTAC<br>GTCCTCTACCCTTTCACTTTTGGCGG<br>AGGGACCAAGGTTGAGATCAAA<br>(SEQ ID NO:103) |
| ADI-3410<br>4 | QVQLQESGPGLVKPSE<br>TLSLTCAVSGYSISSGY<br>YWGWIRQPPGKGLE<br>WIGSIYHSGSTYYNPS<br>LKSRVTISVDTSKNQF<br>SLKLSSVTAADTAVYY | CAGGTGCAGCTGCAGGAGTCGGGCCCAG<br>GACTGGTGAAGCCTTCGGAGACCCTGTCC<br>CTCACCTGCGCTGTCTCTGGTTACTCCATC<br>AGCAGTGGTTACTACTGGGGCTGGATCCG<br>GCAGCCCCAGGGAAGGGGCTGGAGTGG<br>ATTGGGAGTATCTATCATAGTGGGAGCACC | DIQMTQSPSTLSASVGDR<br>VTITCRASQSISSWLAWY<br>QQKPGKAPKLLIYDASSL<br>ESGVPSRFSGSGSGTEFTL<br>TISSLQPDDFATYYCQQS<br>RIYSTFGGGTKVEIK | GACATCCAGATGACCCAGTCTCCTT<br>CCACCCTGTCTGCATCTGTAGGAGA<br>CAGAGTCACCATCACTTGCCGGGCC<br>AGTCAGAGTATTAGTAGCTGGTTGG<br>CCTGGTATCAGCAGAAACCAGGGAA<br>AGCCCCTAAGCTCCTGATCTATGATG |

34

| Clone | Amino acid / Nucleotide sequences | | |
|---|---|---|---|
| | CARASIYTPPPEYFQH WGQGTLVTVSS (SEQ ID NO:70) | TACTACAACCCGTCCCTCAAGAGTCGAGT CACCATATCAGTAGACACGTCCAAGAACC AGTTCTCCCTGAAGCTGAGTTCTGTGACC GCCGCAGACACGGCGGGTGTACTACTGCGC TAGGGCATCTATATACACTCCACCTCCAGA ATACTTCCAACACTGGGGACAGGGTACAT TGGTCACCGTCTCCTCA (SEQ ID NO:88) | (SEQ ID NO:97) | CCTCCAGTTTGGAAAGTGGGGTCCC ATCAAGGTTCAGCGGCAGTGGATCT GGGACAGAATTCACTCTCACCATCA GCAGCCTGCAGCCTGATGATTTTGC AACTTATTACTGCCAGCAGTCCAGA ATCTACTCTACTTTTGGCGGAGGGA CCAAGGTTGAGATCAAA (SEQ ID NO:104) |
| ADI-3754 2 | QVQLQESGPGLVKPSE TLSLTCAVSGYSISEGY YWGWIRQPPGKGLE WIGSIYHTGFTYYNPS LKSRVTISVDTSKNQF SLKLSSVTAADTAVYY CARASIYTPPPEYFQH WGQGTLVTVSS (SEQ ID NO:71) | CAGGTGCAGCTGCAGGAGAGTCGGGCCCAG GACTGGTGAAGCCTTCGGAGACCCTGTCC CTCACCTGCGCTGTCTCTGGTTACTCCATC AGCGAAGGTTACTACTGGGGCTGGATCCG GCAGCCCCCAGGGAAGGGGCTGGAGTGG ATTGGGAGTATCTATCATACAGGGTTCACC TACTACAACCCGTCCCTCAAGAGTCGAGT CACCATATCAGTAGACACGTCCAAGAACC AGTTCTCCCTGAAGCTGAGTCTGTGACC GCCGCAGACACGGCGGGTGTACTACTGCGC TAGGGCATCTATATACACTCCACCTCCAGA ATACTTCCAACACTGGGGACAGGGTACAT TGGTCACCGTCTCCTCA (SEQ ID NO:89) | | |
| ADI-3754 3 | QVQLQESGPGLVKPSE TLSLTCAVSGYSISSGY YWAWIRQPPGKGLEW IGSIFHSGFTFYNPSLK SRVTISVDTSKNQFSL KLSSVTAADTAVYYC ARASIYTPPPEYFQHW GQGTLVTVSS (SEQ ID NO:72) | CAGGTGCAGCTGCAGGAGAGTCGGGCCCAG GACTGGTGAAGCCTTCGGAGACCCTGTCC CTCACCTGCGCTGTCTCTGGTTACTCCATC TCCAGTGGTTACTACTGGGCCTGGATCCG GCAGCCCCCAGGGAAGGGGCTGGAGTGG ATTGGGAGTATCTTCCATAGTGGTTCACC TTCTACAACCCGTCCCTCAAGAGTCGAGT CACCATATCAGTAGACACGTCCAAGAACC AGTTCTCCCTGAAGCTGAGTTCTGTGACC GCCGCAGACACGGCGGGTGTACTACTGCGC TAGGGCATCTATATACACTGGGGACAGGGTACAT ATACTTCCAACACTGGGGACAGGGTACAT TGGTCACCGTCTCCTCA (SEQ ID NO:90) | | GACATCCAGATGACCCAGTCTCCTT CCACCCTGTCTGCATCTGTAGGAGA CAGAGTCACCATCACTTGCCGGCCA AGTCAGAGTATTAGTAGCTGGTTGG CCTGGTATCAGCAGAAAACCAGGGAA AGCCCCTAAGCTCCTGATCTATGATG CCTCCAGTTTGGAAAGTGGGGTCCC ATCAAGGTTCAGCGGCAGTGGATCT GGGACAGAATTCACTCTCACCATCA GCAGCCTGCAGCCTGATGATTTTGC AACTTATTACTGCCAGCAGTCCAGA ATCTACTCTACTTTTCGGCGGAGGGA CCAAGGTGGAGATCAAA (SEQ ID NO:105) |

Table C. The SEQ ID NO. corresponding to the partial sequences in the sequence listing of the present invention.

| ADI name | Heavy chain | | | | | Light chain | | | | | Heavy chain HC | Light chain LC |
| | Heavy chain variable region VH | | | | | Light chain variable region VL | | | | | | |
| | VH CDR1 (SEQ ID NO) (**ABM**) | VH CDR2 (SEQ ID NO) (**Kabat**) | VH CDR3 (SEQ ID NO) (**IMGT**) | VH Pro. (SEQ ID NO) | VH DNA (SEQ ID NO) | VL CDR1 (SEQ ID NO) (**Kabat**) | VL CDR2 (SEQ ID NO) (**Kabat**) | VL CDR3 (SEQ ID NO) (**Kabat**) | VL Pro. (SEQ ID NO) | VL DNA (SEQ ID NO) | | |
|---|---|---|---|---|---|---|---|---|---|---|---|---|
| ADI-34071 | 1 | 16 | 31 | 55 | 73 | 38 | 44 | 49 | 91 | 98 | 106 | 124 |
| ADI-34074 | | | 32 | 56 | 74 | 39 | 45 | 50 | 92 | 99 | 107 | 125 |
| ADI-37496 | 2 | 17 | | 57 | 75 | | | | | | 108 | |
| ADI-37497 | 3 | 18 | | 58 | 76 | | | | | | 109 | |
| ADI-34086 | 4 | 19 | 33 | 59 | 77 | 40 | 46 | 51 | 93 | 100 | 110 | 126 |
| ADI-37503 | | 20 | | 60 | 78 | | | | | | 111 | |
| ADI-37504 | 5 | 21 | | 61 | 79 | | | | | | 112 | |
| ADI-37505 | 6 | 22 | | 62 | 80 | | | | | | 113 | |
| ADI-37506 | 7 | 23 | | 63 | 81 | | | | | | 114 | |
| ADI-37507 | 8 | | | 64 | 82 | | | | | | 115 | |

EP 4 169 948 A1

36

(continued)

| ADI name | Heavy chain | | | | | Light chain | | | | | Heavy chain HC | Light chain LC |
| | Heavy chain variable region VH | | | | | Light chain variable region VL | | | | | | |
| | VH CDR1 (SEQ ID NO) (ABM) | VH CDR2 (SEQ ID NO) (Kabat) | VH CDR3 (SEQ ID NO) (IMGT) | VH Pro. (SEQ ID NO) | VH DNA (SEQ ID NO) | VL CDR1 (SEQ ID NO) (Kabat) | VL CDR2 (SEQ ID NO) (Kabat) | VL CDR3 (SEQ ID NO) (Kabat) | VL Pro. (SEQ ID NO) | VL DNA (SEQ ID NO) | | |
|---|---|---|---|---|---|---|---|---|---|---|---|---|
| ADI-34095 | 9 | 24 | 34 | 65 | 83 | 41 | 47 | 52 | 94 | 101 | 116 | 127 |
| ADI-37513 | 10 | 25 | | 66 | 84 | | | | | | 117 | |
| ADI-37522 | 11 | 26 | 35 | 67 | 85 | | | | | | 118 | |
| ADI-34101 | 9 | 24 | 36 | 68 | 86 | 39 | 45 | 53 | 95 | 102 | 119 | 128 |
| ADI-37532 | 12 | 27 | | 69 | 87 | 42 | | | 96 | 103 | 120 | 129 |
| ADI-34104 | 13 | 28 | 37 | 70 | 88 | 43 | 48 | 54 | 97 | 104 | 121 | 130 |
| ADI-37542 | 14 | 29 | | 71 | 89 | | | | | | 122 | |
| ADI-37543 | 15 | 30 | | 72 | 90 | | | | | 105 | 123 | |

EP 4 169 948 A1

Table D. **Heavy and light chain sequences of the exemplary anti-CD73 antibodies of the invention:**

| Antibody name | VH CDR1 (Kabat scheme) | VH CDR2 (Kabat scheme) | VH CDR3 (Kabat scheme) | VL CDR1 (Kabat scheme) | VL CDR2 (Kabat scheme) | VL CDR3 (Kabat scheme) |
|---|---|---|---|---|---|---|
| ADI-34071 | DYAMH (SEQ ID NO: 136) | GISWSSGSIGYADSVKG (SEQ ID NO:16) | DRRYGLTYGMDV (SEQ ID NO: 150) | QASQDITNYLN (SEQ ID NO:38) | DASNLET (SEQ ID NO:44) | QQSVVLPFT (SEQ ID NO: 49) |
| ADI-34074 | DFYMH (SEQ ID NO: 137) | GISWSSTSIDYADSVKG (SEQ ID NO:17) | GKYLPTGSVDY (SEQ ID NO: 151) | RASQSVSSSYLA (SEQ ID NO:39) | GASSRAT (SEQ ID NO:45) | QQYYDYPPIT (SEQ ID NO: 50) |
| ADI-37496 | DFYMH (SEQ ID NO: 137) | GISWSSTSIDYADSVKG (SEQ ID NO:17) | GKYLPTGSVDY (SEQ ID NO: 151) | RASQSVSSSYLA (SEQ ID NO:39) | GASSRAT (SEQ ID NO:45) | QQYYDYPPIT (SEQ ID NO: 50) |
| ADI-37497 | DYFMH (SEQ ID NO: 138) | GISWSSGPIDYADSVKG (SEQ ID NO:18) | GKYLPTGSVDY (SEQ ID NO: 151) | RASQSVSSSYLA (SEQ ID NO:39) | GASSRAT (SEQ ID NO:45) | QQYYDYPPIT (SEQ ID NO: 50) |
| ADI-34086 | NYAMS (SEQ ID NO: 139) | AISGSGGSTYYADSVKG (SEQ ID NO:19) | GGPYGSGSYWDTFDY (SEQ ID NO:152) | RASQSVSSNLA (SEQ ID NO:40) | GASTRAT (SEQ ID NO:46) | QQYTNHYT (SEQ ID NO: 51) |
| ADI-37503 | NYAMS (SEQ ID NO: 139) | AISGRASSTYYADSVKG (SEQ ID NO:20) | GGPYGSGSYWDTFDY (SEQ ID NO:152) | RASQSVSSNLA (SEQ ID NO:40) | GASTRAT (SEQ ID NO:46) | QQYTNHYT (SEQ ID NO: 51) |
| ADI-37504 | NYAMS (SEQ ID NO: 139) | AISGTGRATYYADSVKG (SEQ ID NO:21) | GGPYGSGSYWDTFDY (SEQ ID NO:152) | RASQSVSSNLA (SEQ ID NO:40) | GASTRAT (SEQ ID NO:46) | QQYTNHYT (SEQ ID NO: 51) |
| ADI-37505 | NYAMT (SEQ ID NO: 140) | AISGRGTATYYADSVKG (SEQ ID NO:22) | GGPYGSGSYWDTFDY (SEQ ID NO:152) | RASQSVSSNLA (SEQ ID NO:40) | GASTRAT (SEQ ID NO:46) | QQYTNHYT (SEQ ID NO: 51) |
| ADI-37506 | NLAMS (SEQ ID NO: 141) | AISGRATSTYYADSVKG (SEQ ID NO:23) | GGPYGSGSYWDTFDY (SEQ ID NO:152) | RASQSVSSNLA (SEQ ID NO:40) | GASTRAT (SEQ ID NO:46) | QQYTNHYT (SEQ ID NO: 51) |
| ADI-37507 | NYAMA (SEQ ID NO: 142) | AISGRATSTYYADSVKG (SEQ ID NO:23) | GGPYGSGSYWDTFDY (SEQ ID NO:152) | RASQSVSSNLA (SEQ ID NO:40) | GASTRAT (SEQ ID NO:46) | QQYTNHYT (SEQ ID NO: 51) |
| ADI-34095 | SYGMH (SEQ ID NO: 143) | VISYEGSNKYYADSVKG (SEQ ID NO:24) | GGARYPIAFDI (SEQ ID NO:153) | RASQDISSWLA (SEQ ID NO:41) | AASSLQS (SEQ ID NO:47) | QQANDFPIT (SEQ ID NO: 52) |
| ADI-37513 | FYGMH (SEQ ID NO: 144) | VINDEGINKYYADSVKG (SEQ ID NO:25) | GGARYPIAFDI (SEQ ID NO:153) | RASQDISSWLA (SEQ ID NO:41) | AASSLQS (SEQ ID NO:47) | QQANDFPIT (SEQ ID NO: 52) |
| ADI-37522 | HYGMH (SEQ ID NO: 145) | VISDEGSNKYYADSVKG (SEQ ID NO:26) | GWLRYPIAFDI (SEQ ID NO: 154) | RASQDISSWLA (SEQ ID NO:41) | AASSLQS (SEQ ID NO:47) | QQANDFPIT (SEQ ID NO: 52) |

| Antibody name | VH CDR1 (Kabat scheme) | VH CDR2 (Kabat scheme) | VH CDR3 (Kabat scheme) | VL CDR1 (Kabat scheme) | VL CDR2 (Kabat scheme) | VL CDR3 (Kabat scheme) |
|---|---|---|---|---|---|---|
| ADI-34101 | SYGMH (SEQ ID NO: 143) | VISYEGSNKYYADSVKG (SEQ ID NO:24) | SRYDWSLFDI (SEQ ID NO:155) | RASQSVSSSYLA (SEQ ID NO:39) | GASSRAT (SEQ ID NO:45) | QQYVLYPFT (SEQ ID NO: 53) |
| ADI-37532 | SYSMH (SEQ ID NO: 146) | HI-VEGSDKYYADSVKG (SEQ ID NO:27) | | GASQSVSSSYLA (SEQ ID NO:42) | | |
| ADI-34104 | SGYYWG (SEQ ID NO: 147) | SIYHSGSTYYNPSLKS (SEQ ID NO: 28) | ASIYTPPPEYFQH (SEQ ID NO: 156) | RASQSISSWLA (SEQ ID NO:43) | DASSLES (SEQ ID NO:48) | QQSRIYST (SEQ ID NO: 54) |
| ADI-37542 | EGYYWG (SEQ ID NO: 148) | SIYHTGFTYYNPSLKS (SEQ ID NO: 29) | | | | |
| ADI-37543 | SGYYWA (SEQ ID NO: 149) | SIFHSGFTFYNPSLKS (SEQ ID NO: 30) | | | | |

1) ADI-34071

**Heavy chain** (SEQ ID NO: 106)

EVQLVESGGGLVQPGRSLRLSCAASGFTFDDYAMHWVRQAPGKGLEWVSGISWSSGSIGYA
DSVKGRFTISRDNAKNSLYLQMNSLRAEDTAVYYCAKDRRYGLTYGMDVWGQGTTVTVSS
ASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPPCPAPEAAGGPSVFLFPPK
PKDTLMISRTPEVTCVVVDVSQEDPEVQFNWYVDGVEVHNAKTKPREEQFNSTYRVVSVLT
VLHQDWLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCSVMHEA
LHNHYTQKSLSLSLG*

**Light chain(SEQ** ID NO: 124)

DIQMTQSPSSLSASVGDRVTITCQASQDITNYLNWYQQKPGKAPKLLIYDASNLETGVPSRFS
GSGSGTDFTFTISSLQPEDIATYYCQQSVVLPFTFGGGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGEC*

2) ADI-34074

**Heavy chain (SEQ ID NO: 107)**

EVQLVESGGGLVQPGRSLRLSCAASGFTFDDYAMHWVRQAPGKGLEWVSGISWSSGSIGYA
DSVKGRFTISRDNAKNSLYLQMNSLRAEDTAVYYCAKGKYLPTGSVDYWGQGTLVTVSSAS
TKGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPPCPAPEAAGGPSVFLFPPKPK
DTLMISRTPEVTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFSCSVMHEAL
HNHYTQKSLSLSLG*

Light chain(SEQ ID NO: 125)

EIVLTQSPGTLSLSPGERATLSCRASQSVSSSYLAWYQQKPGQAPRLLIYGASSRATGIPDRFS GSGSGTDFTLTISRLEPEDFAVYYCQQYYDYPPITFGGGTKVEIKRTVAAPSVFIFPPSDEQLKS GTASVVCLLNNFYPREAKVQWKVDNALQSGNSQESVTEQDSKDSTYSLSSTLTLSKADYEK HKVYACEVTHQGLSSPVTKSFNRGEC*

3) ADI-37496

**Heavy chain** (SEQ ID NO: 108)

EVQLVESGGGLVQPGRSLRLSCAASGFTFDDFYMHWVRQAPGKGLEWVSGISWSSTSIDYA DSVKGRFTISRDNAKNSLYLQMNSLRAEDTAVYYCAKGKYLPTGSVDYWGQGTLVTVSSAS TKGPSVFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPPCPAPEAAGGPSVFLFPPKPK DTLMISRTPEVTCVVVDVSQEDPEVQFNWYVDGVEVHNAKTKPREEQFNSTYRVVSVLTVL HQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEAL HNHYTQKSLSLSLG*

**Light chain(SEQ** ID NO: 125)

4) ADI-37497

**Heavy chain(SEQ** ID NO: 109)

EVQLVESGGGLVQPGGSLRLSCAASGFTFDDYFMHWVRQAPGKGLEWVSGISWSSGPIDYA DSVKGRFTISRDNAKNSLYLQMNSLRAEDTAVYYCAKGKYLPTGSVDYWGQGTLVTVSSAS TKGPSVFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPPCPAPEAAGGPSVFLFPPKPK DTLMISRTPEVTCVVVDVSQEDPEVQFNWYVDGVEVHNAKTKPREEQFNSTYRVVSVLTVL HQDWLNGKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSCSVMHEAL HNHYTQKSLSLSLG*

Light chain(SEQ ID NO: 125)

5) ADI-34086

**Heavy chain(SEQ** ID NO: 110)

EVQLLESGGGLVQPGGSLRLSCAASGFTFSNYAMSWVRQAPGKGLEWVSAISGSGGSTYYA

DSVKGRFTISRDNSKNTLYLQMNSLRAEDTAVYYCARGGPYGSGSYWDTFDYWGQGTLVT

VSSASTKGPSVFPLAPCSRSTSESTAALGCLVKDYFPEPVTVSWNSGALTSGVHTFPAVLQSS

GLYSLSSVVTVPSSSLGTKTYTCNVDHKPSNTKVDKRVESKYGPPCPPCPAPEAAGGPSVFLF

PPKPKDTLMISRTPEVTCVVVDVSQEDPEVQFNWYVDGVEVHNAKTKPREEQFNSTYRVVS

VLTVLHQDWLNGKEYKCKVSNKGLPSSIEKTISKAKGQPREPQVYTLPPSQEEMTKNQVSLT

CLVKGFYPSDIAVEWESNGQPENNYKTTPPVLDSDGSFFLYSRLTVDKSRWQEGNVFSCSVM

HEALHNHYTQKSLSLSLG*

**Light chain**(SEQ ID NO: 126)

EIVMTQSPATLSVSPGERATLSCRASQSVSSNLAWYQQKPGQAPRLLIYGASTRATGIPARFSG

SGSGTEFTLTISSLQSEDFAVYYCQQYTNHYTFGGGTKVEIKRTVAAPSVFIFPPSDEQLKSGT

ASVVCLLNNFYPREAKVQWKVDNALQSGNSQESVTEQDSKDSTYSLSSTLTLSKADYEKHK

VYACEVTHQGLSSPVTKSFNRGEC*

6) ADI-37503

**Heavy chain**(SEQ ID NO: 111)

EVQLLESGGGLVQPGGSLRLSCAASGFTFSNYAMSWVRQAPGKGLEWVSAISGRASSTYYA

DSVKGRFTISRDNSKNTLYLQMNSLRAEDTAVYYCARGGPYGSGSYWDTFDYWGQGTLVT

VSSASTKGPSVFPLAPCSRSTSESTAALGCLVKDYFPEPVTVSWNSGALTSGVHTFPAVLQSS

GLYSLSSVVTVPSSSLGTKTYTCNVDHKPSNTKVDKRVESKYGPPCPPCPAPEAAGGPSVFLF

PPKPKDTLMISRTPEVTCVVVDVSQEDPEVQFNWYVDGVEVHNAKTKPREEQFNSTYRVVS

VLTVLHQDWLNGKEYKCKVSNKGLPSSIEKTISKAKGQPREPQVYTLPPSQEEMTKNQVSLT

CLVKGFYPSDIAVEWESNGQPENNYKTTPPVLDSDGSFFLYSRLTVDKSRWQEGNVFSCSVM

HEALHNHYTQKSLSLSLG*

Light chain(SEQ ID NO: 126)

7) ADI-37504

**Heavy chain**(SEQ ID NO: 112)

42

EVQLLESGGGLVQPGGSLRLSCAASGFTFHNYAMSWVRQAPGKGLEWVSAISGTGRATYYA
DSVKGRFTISRDNSKNTLYLQMNSLRAEDTAVYYCARGGPYGSGSYWDTFDYWGQGTLVT
VSSASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEAAGGPSVFLF
PPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSLT
CLVKGFYPSDIAVEWESNGQPENNYKTTPPVLDSDGSFFLYSRLTVDKSRWQEGNVFSCSVM
HEALHNHYTQKSLSLSLG*

**Light chain**(SEQ ID NO: 126)

8) ADI-37505

**Heavy chain**(SEQ ID NO: 113)

QVQLVESGGGLVQPGGSLRLSCAASGFTFSNYAMTWVRQAPGKGLEWVSAISGRGTATYYA
DSVKGRFTISRDNSKNTLYLQMNSLRAEDTAVYYCARGGPYGSGSYWDTFDYWGQGTLVT
VSSASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEAAGGPSVFLF
PPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSLT
CLVKGFYPSDIAVEWESNGQPENNYKTTPPVLDSDGSFFLYSRLTVDKSRWQEGNVFSCSVM
HEALHNHYTQKSLSLSLG*

**Light chain**(SEQ ID NO: 126)

9) ADI-37506

**Heavy chain**(SEQ ID NO: 114)

EVQLLESGGGLVQPGGSLRLSCAASGFTFSNLAMSWVRQAPGKGLEWVSAISGRATSTYYA
DSVKGRFTISRDNSKNTLYLQMNSLRAEDTAVYYCARGGPYGSGSYWDTFDYWGQGTLVT
VSSASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEAAGGPSVFLF
PPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSLT
CLVKGFYPSDIAVEWESNGQPENNYKTTPPVLDSDGSFFLYSRLTVDKSRWQEGNVFSCSVM
HEALHNHYTQKSLSLSLG*

**Light chain**(SEQ ID NO: 126)

10) ADI-37507

**Heavy chain**(SEQ ID NO: 115)

EVQLLESGGGLVQPGGSLRLSCAASGFTFLNYAMAWVRQAPGKGLEWVSAISGRATSTYYA
DSVKGRFTISRDNSKNTLYLQMNSLRAEDTAVYYCARGGPYGSGSYWDTFDYWGQGTLVT
VSSASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEAAGGPSVFLF
PPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSLT
CLVKGFYPSDIAVEWESNGQPENNYKTTPPVLDSDGSFFLYSRLTVDKSRWQEGNVFSCSVM
HEALHNHYTQKSLSLSLG*

**Light chain**(SEQ ID NO: 126)

11) ADI-34095

**Heavy chain**(SEQ ID NO: 116)

QVQLVESGGGVVQPGRSLRLSCAASGFTFSSYGMHWVRQAPGKGLEWVAVISYEGSNKYY

ADSVKGRFTISRDNSKNTLYLQMNSLRAEDTAVYYCARGGARYPIAFDIWGQGTMVTVSSA

STKGPSVFPLAPCSRSTSESTAALGCLVKDYFPEPVTVSWNSGALTSGVHTFPAVLQSSGLYSL

SSVVTVPSSSLGTKTYTCNVDHKPSNTKVDKRVESKYGPPCPPCPAPEAAGGPSVFLFPPKPK

DTLMISRTPEVTCVVVDVSQEDPEVQFNWYVDGVEVHNAKTKPREEQFNSTYRVVSVLTVL

HQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEAL

HNHYTQKSLSLSLG*

**Light chain**(SEQ ID NO: 127)

DIQLTQSPSSVSASVGDRVTITCRASQDISSWLAWYQQKPGKAPKLLIYAASSLQSGVPSRFS

GSGSGTDFTLTISSLQPEDFATYYCQQANDFPITFGGGTKVEIKRTVAAPSVFIFPPSDEQLKSG

TASVVCLLNNFYPREAKVQWKVDNALQSGNSQESVTEQDSKDSTYSLSSTLTLSKADYEKH

KVYACEVTHQGLSSPVTKSFNRGEC*

12) ADI-37513

**Heavy chain**(SEQ ID NO: 117)

EVQLLESGGGVVQPGRSLRLSCAASGFTFNFYGMHWVRQAPGKGLEWVAVINDEGINKYY

ADSVKGRFTISRDNSKNTLYLQMNSLRAEDTAVYYCARGGARYPIAFDIWGQGTMVTVSSA

STKGPSVFPLAPCSRSTSESTAALGCLVKDYFPEPVTVSWNSGALTSGVHTFPAVLQSSGLYSL

SSVVTVPSSSLGTKTYTCNVDHKPSNTKVDKRVESKYGPPCPPCPAPEAAGGPSVFLFPPKPK

DTLMISRTPEVTCVVVDVSQEDPEVQFNWYVDGVEVHNAKTKPREEQFNSTYRVVSVLTVL

HQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEAL

HNHYTQKSLSLSLG*

**Light chain**(SEQ ID NO: 127)

13) ADI-37522

**Heavy chain**(SEQ ID NO: 118)

QVQLVESGGGVVQPGRSLRLSCAASGFTFSHYGMHWVRQAPGKGLEWVAVISDEGSNKYY
ADSVKGRFTISRDNSKNTLYLQMNSLRAEDTAVYYCARGWLRYPIAFDIWGQGTMVTVSSA
STKGPSVFPLAPCSRSTSESTAALGCLVKDYFPEPVTVSWNSGALTSGVHTFPAVLQSSGLYSL
SSVVTVPSSSLGTKTYTCNVDHKPSNTKVDKRVESKYGPPCPPCPAPEAAGGPSVFLFPPKPK
DTLMISRTPEVTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFSCSVMHEAL
HNHYTQKSLSLSLG*

**Light chain**(SEQ ID NO: 127)

14) ADI-34101

**Heavy chain**(SEQ ID NO: 119)

QVQLVESGGGVVQPGRSLRLSCAASGFTFSSYGMHWVRQAPGKGLEWVAVISYEGSNKYY
ADSVKGRFTISRDNSKNTLYLQMNSLRAEDTAVYYCARSRYDWSLFDIWGQGTMVTVSSAS
TKGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPPCPAPEAAGGPSVFLFPPKPK
DTLMISRTPEVTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFSCSVMHEAL
HNHYTQKSLSLSLG*

**Light chain**(SEQ ID NO: 128)

EIVLTQSPGTLSLSPGERATLSCRASQSVSSSYLAWYQQKPGQAPRLLIYGASSRATGIPDRFS
GSGSGTDFTLTISRLEPEDFAVYYCQQYVLYPFTFGGGTKVEIKRTVAAPSVFIFPPSDEQLKS
GTASVVCLLNNFYPREAKVQWKVDNALQSGNSQESVTEQDSKDSTYSLSSTLTLSKADYEK

HKVYACEVTHQGLSSPVTKSFNRGEC*

15) ADI-37532

**Heavy chain**(SEQ ID NO: 120)

QVQLVESGGGVVQPGRSLRLSCAASGFTFSSYSMHWVRQAPGKGLEWVAHIVEGSDKYYA
DSVKGRFTISRDNSKNTLYLQMNSLRAEDTAVYYCARSRYDWSLFDIWGQGTMVTVSSAST
KGPSVFPLAPCSRSTSESTAALGCLVKDYFPEPVTVSWNSGALTSGVHTFPAVLQSSGLYSLSS
VVTVPSSSLGTKTYTCNVDHKPSNTKVDKRVESKYGPPCPPCPAPEAAGGPSVFLFPPKPKD
TLMISRTPEVTCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCSVMHEALH
NHYTQKSLSLSLG*

**Light chain** (SEQ ID NO: 129)

EIVLTQSPGTLSLSPGERATLSCGASQSVSSSYLAWYQQKPGQAPRLLIYGASSRATGIPDRFS
GSGSGTDFTLTISRLEPEDFAVYYCQQYVLYPFTFGGGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC*

16) ADI-34104

**Heavy chain** (SEQ ID NO: 121)

QVQLQESGPGLVKPSETLSLTCAVSGYSISSGYYWGWIRQPPGKGLEWIGSIYHSGSTYYNPS
LKSRVTISVDTSKNQFSLKLSSVTAADTAVYYCARASIYTPPPEYFQHWGQGTLVTVSSASTK
GPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEAAGGPSVFLFPPKPKDTL
MISRTPEVTCVVVDVSQEDPEVQFNWYVDGVEVHNAKTKPREEQFNSTYRVVSVLTVLHQ
DWLNGKEYKCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSVMHEALHN
HYTQKSLSLSLG*

**Light chain** (SEQ ID NO: 130)

DIQMTQSPSTLSASVGDRVTITCRASQSISSWLAWYQQKPGKAPKLLIYDASSLESGVPSRFS
GSGSGTEFTLTISSLQPDDFATYYCQQSRIYSTFGGGTKVEIKRTVAAPSVFIFPPSDEQLKSGT
ASVVCLLNNFYPREAKVQWKVDNALQSGNSQESVTEQDSKDSTYSLSSTLTLSKADYEKHK
VYACEVTHQGLSSPVTKSFNRGEC*

17) ADI-37542

**Heavy chain**(SEQ ID NO: 122)

QVQLQESGPGLVKPSETLSLTCAVSGYSISEGYYWGWIRQPPGKGLEWIGSIYHTGFTYYNPS
LKSRVTISVDTSKNQFSLKLSSVTAADTAVYYCARASIYTPPPEYFQHWGQGTLVTVSSASTK
GPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEAAGGPSVFLFPPKPKDTL
MISRTPEVTCVVVDVSQEDPEVQFNWYVDGVEVHNAKTKPREEQFNSTYRVVSVLTVLHQ
DWLNGKEYKCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSVMHEALHN
HYTQKSLSLSLG*

**Light chain**(SEQ ID NO: 130)

18) ADI-37543

**Heavy chain**(SEQ ID NO: 123)

QVQLQESGPGLVKPSETLSLTCAVSGYSISSGYYWAWIRQPPGKGLEWIGSIFHSGFTFYNPSL
KSRVTISVDTSKNQFSLKLSSVTAADTAVYYCARASIYTPPPEYFQHWGQGTLVTVSSASTKG
PSVFPLAPCSRSTSESTAALGCLVKDYFPEPVTVSWNSGALTSGVHTFPAVLQSSGLYSLSSVV
TVPSSSLGTKTYTCNVDHKPSNTKVDKRVESKYGPPCPPCPAPEAAGGPSVFLFPPKPKDTL
MISRTPEVTCVVVDVSQEDPEVQFNWYVDGVEVHNAKTKPREEQFNSTYRVVSVLTVLHQ
DWLNGKEYKCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSVMHEALHN

HYTQKSLSLSLG*

**Light chain** (SEQ ID NO: 130)

**CD73 protein wild-type sequence** (SEQ ID NO: 131)

MCPRAARAPATLLLALGAVLWPAAGAWELTILHTNDVHSRLEQTSEDSSKCVNASRCMGGV

ARLFTKVQQIRRAEPNVLLLDAGDQYQGTIWFTVYKGAEVAHFMNALRYDAMALGNHEFD

NGVEGLIEPLLKEAKFPILSANIKAKGPLASQISGLYLPYKVLPVGDEVVGIVGYTSKETPFLS

NPGTNLVFEDEITALQPEVDKLKTLNVNKIIALGHSGFEMDKLIAQKVRGVDVVVGGHSNTF

LYTGNPPSKEVPAGKYPFIVTSDDGRKVPVVQAYAFGKYLGYLKIEFDERGNVISSHGNPILL

NSSIPEDPSIKADINKWRIKLDNYSTQELGKTIVYLDGSSQSCRFRECNMGNLICDAMINNNL

RHTDEMFWNHVSMCILNGGGIRSPIDERNNGTITWENLAAVLPFGGTFDLVQLKGSTLKKAF

EHSVHRYGQSTGEFLQVGGIHVVYDLSRKPGDRVVKLDVLCTKCRVPSYDPLKMDEVYKVI

LPNFLANGGDGFQMIKDELLRHDSGDQDINVVSTYISKMKVIYPAVEGRIKFSTGSHCHGSFS

LIFLSLWAVIFVLYQ

**EXAMPLES**

**Example 1. Achievement, Expression and Purification of Anti-CD73 Antibodies of the Invention**

1. Achievement of parental antibodies by yeast-based antibody display library:

Amplification was performed by the yeast-based antibody display library available from Adimab according to the current method (WO2009036379; WO 2010105256; WO2012009568), wherein the diversity of each library reached $1 \times 10^9$. Briefly, the first two rounds of screening were performed by magnetic activated cell sorting using the MACS system available from Miltenyi. First, the yeast cells of each library (about $1 \times 10^{10}$ cells/library) were incubated for 15 min at room temperature in FACS washing buffer (Phosphate Buffer Solution containing 0.1% bovine serum albumin) containing 100 nM biotin-labeled human CD73 antigen (R&D Systems, 5795-EN) . Cells were washed once with 50 ml of pre-cooling FACS washing buffer, re-suspended in 40 ml of the same washing buffer, added with 500 $\mu$l of streptomycin beads (Miltenyi LS) and incubated at 4°C for 15 minutes. The supernatant was discarded after centrifugation at 1000 rpm for 5 min at room temperature, the cells were resuspended with 5 ml of FACS washing buffer, and the cell solution was added to the Miltenyi LS column. After loading, the column was washed three times with FACS washing buffer, 3 ml each time. The Miltenyi LS column was removed from the magnetic area, eluted with 5 ml of growth medium, and the eluted yeast cells were collected and grown overnight at 37 °C.

The next round of sorting was performed by flow cytometry: about $1 \times 10^8$ yeast cells obtained by screening with MACS system were washed three times with FACS buffer, and incubated with a low concentration of biotin ($100^{-1}$ nM) labeled human CD73 antigen at room temperature. The culture medium was discarded, the cells were washed twice with FACS washing buffer, and then were mixed with LC-FITC (FITC-labeled goat anti-human immunoglobulin F(ab') kappa chain antibody, Southern Biotech) (1:100 dilution), further mixed with SA-633 (streptavidin-633, Molecular Probes) (1:500 dilution) or SA-PE (streptavidin-phycoerythrin, Sigma) (1:50 dilution) reagent, and incubated at 4°C for 15 minutes. The cells were rinsed twice with pre-cooling FACS washing buffer, resuspended in 0.4 ml buffer, and transfered to a separation tube with filter. The cells were sorted by using FACS ARIA (BD Biosciences).

The yeast cells expressing anti-CD73 antibody obtained by screening were shaken at 30°C for 48 hours to induce the expression of the anti-CD73 antibody. After induction, the yeast cells were removed by centrifugation at 1300 rpm for 10 min at room temperature, and the supernatant was harvested. The anti-CD73 antibody in the supernatant was purified using Protein A, eluted with acetic acid solution, pH 2.0, and the anti-CD73 antibody was harvested. After detection, the antibody purity was >95%.

A total of 6 strains of anti-human CD73 antibodies were obtained in this screening, named as: ADI-34086, ADI-34101, ADI-34071, ADI-34095, ADI-34104 and AD-34074, respectively. The properties of the antibodies are shown in Table 1 below.

2. Affinity maturation of anti-human CD73 antibody:

To obtain anti-human CD73 antibodies with higher affinity, the antibodies ADI-34086, ADI-34101, ADI-34071, ADI-34095, ADI-34104 and AD-34074 were optimized by the following methods.

VHmut screening

Mutations were introduced into the antibody heavy chain region using conventional mismatch PCR methods in this method. Specifically, the base mismatch probability during the PCR process was increased to about 0.01 bp by using 1 uM hypermutated base analogs dPTP and 8-oxo-dGTP.

The resulting mismatched PCR product was constructed into a vector containing a heavy chain constant region by homologous recombination. A secondary library with a library capacity of $1 \times 10^7$ was obtained under screening pressures including CD73 antigen titers, competition with unlabeled antigen, and competition with parent antibodies. Three rounds of successful screening were performed by the FACS method.

CDRH1/CDRH2 screening

The heavy chain CDR3 gene of the progeny antibody obtained by the VHmut method was constructed into CDRH1/CDRH2 gene library with diversity of $1 \times 10^8$, and 3 rounds of screening were carried out thereon. The first round was carried out by MACS method, while the second and third rounds were carried out by FACS method. The antibody-antigen conjugates were subjected to affinity pressure to screen out the antibodies with the highest affinity.

After the process of the above affinity maturation, 12 strains of anti-human CD73 monoclonal antibodies with improved affinity relative to the parent antibody were obtained, which were named as: ADI-37503, ADI-37504, ADI-37505, ADI-37506, ADI-37507, ADI-37532, ADI-37513, ADI-37522, ADI-37497, ADI-37496, ADI-37542 and ADI-37543, respectively.

Therefore, the amino acid sequences and corresponding sequence numbers (SEQ ID NO.) of the CDRs, light and heavy chain variable regions, and light and heavy chains of the 18 antibodies exemplified in the present invention are listed in Tables A, B, D and the Sequence listing of the present application. Antibodies exemplified in the invention is IgG4 format.

3. Expression and purification of the antibody of the present invention in CHO-S cells:

CHO-S cell lines expressing the antibodies were generated by using Freedom® CHO-S® Kit (Invitrogen) according to the manufacturer's instructions. The DNA sequences of the heavy and light chains of the antibody molecule were first inserted into the same pCHO1.0 plasmid, with the heavy chain upstream of the light chain. Then, the constructed pCHO1.0 plasmid was transferred into the CHO-S cell line by chemical transfection or electrotransfection, and the antibody production was detected by ForteBio 48 hours after transfection to determine the transfection efficiency. The transfected cells were subjected to two rounds of pressure screening, resulting in a pool of cells with high antibody expression. After that, the cell pool was expanded, the antibody was expressed in large quantities, and the cell supernatant was collected and purified with Protein A to make the antibody purity >95%.

4. Expression and purification of the antibody of the present invention in 293HEK cells:

The vector pV120 was used for transient expression of antibody in 293HEK cells. The heavy and light chains of the antibody were first cloned into separate pV120 vectors. The pV120 vector with the heavy or light chain of the antibody molecule was transferred into 293HEK cells by chemical transfection. The cultured 293HEK were transiently transfected with chemical transfection reagent polyethyleneimine PEI (available from Polysciences) according to the protocol provided by the manufacturer. First, plasmid DNAs and the transfection reagent were prepared, F17 medium (Gibco) (with volume of 1/5 of the transfection volume) was added into two 50ml centrifuge tubes, one was added with the filtered plasmid (130 $\mu$g/100 ml), the other was added with the filtered PEI (1g/L, Polysciences) (mass ratio (plasmid:PEI)=1:3), mixed gently for 20 times, 5 min, and let stand for 15-30 min. The DNA/PEI mixture was gently poured into the 293HEK cells, mixed well and cultured at 37°C, 8% $CO_2$ for 7 days. Fresh medium was added every 48 hours. After 7 days or continuously culturing the cells until the cell viability reached ≤60%, centrifuged at 13,000 rpm for 20 min at room temperature. The supernatant was taken out and purified with Protein A to make the antibody purity >95%.

In the same way, each of the following control antibodies used in the examples was expressed in 293HEK cells and purified:

Control antibody Oleclumab (Omab), the sequence of which is shown in the sequence of antibody "MEDI9447" disclosed in US Pat. No. 9,938,356 B2.
Control antibody BMS 4-2, the sequence of which is shown in the sequence of antibody "CD73.4-2" disclosed in WO2016/081748A2.

Control antibody Surface, the sequence of which is shown in the sequence of antibody "373.A" disclosed in WO2018/237157A1.

Control antibody CPI-006, the sequence of which is shown in the sequence of antibody "CPX-006" disclosed in WO2017/100670A1.

Control antibody Imab hu101-28, the sequence of which is shown in the sequence of antibody "hu101-28" disclosed in 2018/137598A1.

Control antibody Innate 6E1, the sequence of which is shown in the sequence of antibody "6E1" disclosed in WO2016/131950A1.

## Example 2. Detection of affinity of anti-CD73 antibody of the present invention

The equilibrium dissociation constants ($K_D$) of the 18 anti-CD73 antibodies described herein binding to human CD73 were determined by Biofilm Layer Optical Interferometry (ForteBio).

The bivalent affinity determination was carried out herein on six antibodies before affinity optimization by ForteBio affinity determination method according to the current method (Estep, P et al., High throughput solution Based measurement of antibody-antigen affinity and epitope binning. MAbs, 2013.5(2): p. 270-8). Briefly, the sensor was equilibrated off-line in assay buffer for 30 min, then on-line detection was carried out for 60 sec to establish a baseline. The purified antibody obtained as described above was loaded on-line to the AHQ sensor (ForteBio) for ForteBio affinity determination. The sensor loaded with the antibody was then exposed to 100 nM of human or cynomolgus monkey antigen for 5 minutes, after which the sensor was transferred to assay buffer for dissociation for 5 minutes to determine the dissociation rate. Kinetic analysis was performed by using a 1:1 binding model.

The ability of the six antibodies before affinity optimization to inhibit the enzymatic activity of human CD73 expressed on the surface of Calu-6 tumor cells was measured based on CellTiter-Glo® luminescence method, and the ability to inhibit the enzymatic activity was determined by comparing the inhibition curves of different antibodies for AMP degradation. The experimental process in detail was as follows: (1) Calu-6 cells were inoculated into a 96-well flat bottom plate and cultured overnight. (2) On the next day, 3-fold serially diluted solutions of a candidate antibody (concentration range from 0 to 10000ng/ml) were mixed with Calu-6 cells and incubated in an incubator at 37°C, 5% $CO_2$ for 30 minutes. (3) AMP solution (Sigma, 01930) was prepared and was added at a final concentration of 2 mM to the cells that have been incubated with the antibody. The mixture was incubated in an incubator at 37°C, 5% $CO_2$ for 3 hours. (4) ATP solution (Sigma, A6419) was prepared and mixed at a final concentration of 200 $\mu$M with cell culture supernatant and CellTiter-Glo substrate (Promega, G7572). After shaking at room temperature for 10 minutes, the fluorescence values were read by a Multimode Reader (Molecular Divices, SpectraMax i3) (1000 ms). The IC50 values were calculated by fitting the curve.

The affinities and enzymatic activities of the 6 antibodies before affinity optimization in the assay performed as described above are shown in Table 1 below.

Table 1: Summary of the properties of the six antibodies before affinity optimization

|  | CD73 enzymatic activity assay IC 50 (ng/mL) | Bin code | IgG $K_D$ Human CD73 (M) Bivalence | IgG $K_D$ Cynomolgus monkey CD73 (M) Bivalence |
|---|---|---|---|---|
| Omab | 167 | 1 | 9.48E-10 | 9.60E-10 |
| ADI-34086 | 89 | 1 | 1.23E-09 | 1.90E-09 |
| ADI-34101 | 153 | 1 | 1.07E-09 | 1.57E-09 |
| ADI-34071 | 171 | 1 | 8.92E-10 | 1.34E-09 |
| ADI-34095 | 279 | 1 | 9.44E-10 | 1.48E-09 |
| ADI-34104 | 142 | 3 | 8.47E-10 | 1.24E-09 |
| ADI-34074 | 476 | 2 | 2.12E-09 | 3.13E-09 |

Due to the presence of CD73 dimerization, the monovalent (scfv) affinity of 12 antibodies after affinity optimization were determined using the ForteBio affinity assay as follows: the sensor was equilibrated off-line in the assay buffer for 30 minutes, and then the on-line detection was carried out for 60 sec to establish the baseline. Human or cynomolgus monkey CD73 antigen protein was loaded on-line to the AHQ sensor (ForteBio) for ForteBio affinity measurement. The sensor loaded with antigen was then exposed to 100 nM of the scfv fragment of the antibody obtained by affinity maturation for 5 minutes, after which the sensor was transferred to assay buffer for dissociation for 5 minutes to determine the dissociation rate. Kinetic analysis was performed by using a 1:1 binding model.

The affinities of the 12 antibodies after affinity optimization are shown in Table 2 below.

Table 2: Affinities of the antibodies of the present invention after affinity optimization were measured by Biofilm Layer Optical Interferometry

| Samples | Fortebio scfv KD Biotinylated Human CD73 (M) Monovalent | Fortebio scfv KD Biotinylated cynomolgus monkey CD73(M) monovalent |
|---|---|---|
| ADI-37503 | 7.95E-09 | 6.61E-09 |
| ADI-37504 | 1.54E-08 | 2.02E-08 |
| ADI-37506 | 5.41E-09 | 4.77E-09 |
| ADI-37505 | 5.56E-09 | 5.34E-09 |
| ADI-37507 | 9.21E-09 | 2.37E-08 |
| ADI-37532 | 5.81E-10 | 5.30E-10 |
| ADI-37513 | 2.84E-09 | 2.26E-09 |
| ADI-37522 | 2.43E-09 | 2.93E-09 |
| ADI-37497 | 1.02E-08 | 8.82E-09 |
| ADI-37496 | 1.38E-08 | 1.37E-08 |
| ADI-37542 | 6.04E-09 | 5.47E-09 |
| ADI-37543 | 3.85E-09 | 2.99E-09 |

It can be seen that the above 12 exemplary antibodies of the present invention all show extremely high affinity to human and cynomolgus monkey CD73.

**Example 3. The anti-CD73 antibodies of the present invention inhibit the enzymatic activity of CD73 at the cellular level**

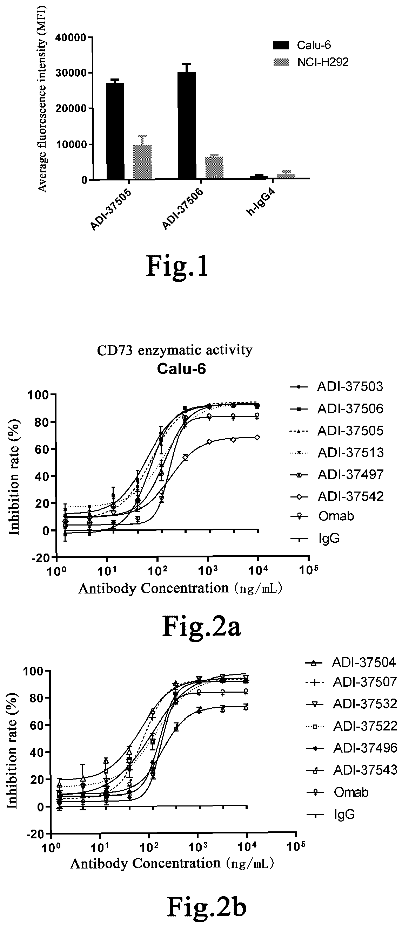

CD73 molecule is expressed on the surface of various tumor cells. In this study, the expression of CD73 molecules on the surface of NCI-H292 and Calu-6 tumor cells was detected by flow cytometry. As shown in Figure 1, the expression of CD73 molecule on the surface of Calu-6 tumor cells was higher than that on NCI-H292 cells.

1. Affinity-optimized anti-CD73 antibodies inhibit the enzymatic activity of cell membrane CD73

The ability of the above exemplary 12 affinity-optimized antibodies of the present invention to inhibit the enzymatic activity of human CD73 expressed on the surface of Calu-6 tumor cells was measured based on CellTiter-Glo® luminescence method, wherein Omab was used as a positive control, and irrelevant IgG was used as a negative control. The ability to inhibit the enzymatic activity was determined by comparing the inhibition curves of different antibodies for AMP degradation. The experimental process in detail was as follows: (1) Calu-6 cells were inoculated into a 96-well flat bottom plate and cultured overnight. (2) On the next day, 3-fold serially diluted solutions of a candidate antibody with a concentration range from 0 to 10000ng/ml were mixed with Calu-6 cells and incubated in an incubator at 37°C, 5% $CO_2$ for 30 minutes. (3) AMP solution (Sigma, 01930) was prepared and was added at a final concentration of 2 mM to the cells that have been incubated with the antibody. The mixture was incubated in an incubator at 37°C, 5% $CO_2$ for 3 hours. (4) ATP solution (Sigma, A6419) was prepared and mixed at a final concentration of 200 $\mu$M with cell culture supernatant and CellTiter-Glo substrate (Promega, G7572). After shaking at room temperature for 10 minutes, the fluorescence values were read by a Multimode Reader (Molecular Divices, SpectraMax i3) (1000 ms). The results are shown in Figure 2.

Table 3 below summarizes the IC50 of 12 exemplary affinity-optimized anti-CD73 antibodies of the present invention for inhibiting the enzymatic activity of cell membrane-bound CD73, with Omab as a positive control.

Table 3: IC50 values of anti-CD73 antibody molecules for inhibiting the enzymatic activity of CD73 on cell membrane

| Sample | IC50 for inhibiting the enzymatic activity of CD73 (ng/mL) |
|---|---|
| Omab | 177 |
| ADI-37503 | 62 |
| ADI-37504 | 70 |
| ADI-37506 | 73 |
| ADI-37505 | 76 |
| ADI-37507 | 78 |
| ADI-37532 | 114 |
| ADI-37513 | 143 |
| ADI-37522 | 146 |
| ADI-37497 | 147 |
| ADI-37496 | 173 |
| ADI-37542 | 189 |
| ADI-37543 | 196 |

From the above experimental results, it can be concluded that: (1) All the anti-CD73 antibodies after affinity maturation can effectively inhibit the enzymatic activity of CD73 on the cell surface. (2) The affinity-matured progeny antibodies ADI-37503, ADI-37504, ADI-37506, ADI-37505, ADI-37507, ADI-37532, ADI-37513, ADI-37522, ADI-37497 and ADI-37496 show stronger ability to inhibit the enzymatic activity of CD73 on cell surface relative to the control antibody Oleclumab.

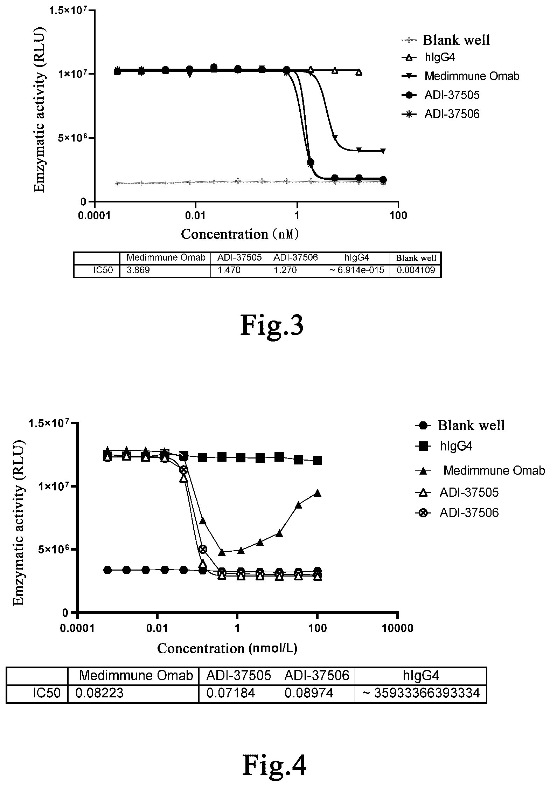

In NCI-H292 cells which have lower CD73 expression, anti-CD73 antibodies ADI-37505 and ADI-37506 of the present invention and control antibodies were measured for the ability to inhibit the enzymatic activity of human CD73 expressed on the surface of NCI-H292 cells by CellTiter-Glo® luminescence method, wherein Omab was used as a positive control, and irrelevant hIgG4 was used as a negative control. The ability to inhibit the enzymatic activity was determined by comparing the inhibition curves of different antibodies for AMP degradation. The experimental process in detail was as follows: (1) NCI-H292 cells were inoculated into a 96-well flat bottom plate and cultured overnight. (2) On the next day, 3-fold serially diluted solutions of a candidate antibody with the concentration range from 0 to 50 mM were mixed with NCI-H292 cells and incubated in an incubator at 37°C, 5% $CO_2$ for 30 minutes. (3) AMP solution (Sigma, 01930) was prepared and was added at a final concentration of 2 mM to the cells that have been incubated with the antibody. The mixture was incubated in an incubator at 37°C, 5% $CO_2$ for 7 hours. (4) ATP solution (Sigma, A6419) was prepared and mixed at a final concentration of 200 μM with cell culture supernatant and CellTiter-Glo substrate (Promega, G7572). After shaking at room temperature for 10 minutes, the fluorescence values were read by a Multimode Reader (Molecular Divices, SpectraMax i3) (1000 ms). The results are shown in Figure 3.

From the above experimental results, it can be concluded that: (1) Antibodies ADI-37505 and ADI-37506 can both effectively inhibit the enzymatic activity of CD73 on the surface of NIC-H292 cells. (2) Both ADI-37505 and ADI-37506 inhibit the enzymatic activity of CD73 with IC50 of 1.47 nM and 1.27 nM, respectively, lower than that of the control antibody Medimmune Omab. (3) The maximum enzyme activity inhibition rate has not been achieved for Medimmune Omab at the highest concentrations, while both ADI-37505 and ADI-37506 completely inhibited CD73 enzyme activity at high concentrations. Therefore, both ADI-37505 and ADI-37506 show higher ability to inhibit the enzymatic activity of CD73 at the cellular level compared to the control antibody.

2. Affinity-optimized anti-CD73 antibodies inhibit the enzymatic activity of soluble CD73

CD73 molecules on the surface of the cell membrane are separated from the cell membrane under the action of phospholipase and enter the intercellular substance and blood to become soluble CD73 molecules with enzymatic activity. The ability of the exemplary antibodies of the present invention to inhibit the enzymatic activity of human CD73 in solution was measured based on the Cell Titer-Glo® luminescence method. The ability to inhibit the enzymatic activity was determined by comparing the inhibition curves of different antibodies for AMP degradation. The experimental process in detail was as follows: (1) Serum-free EMEM medium (ATCC, 30-2003) was preheated at 37°C as a dilution medium in this experiment; (2) 50ul CD73 protein (ACRO, CD3-H52H7) was added into a 96-well round bottom plate at a concentration of 2 nM; (3) The antibody solution was 3-fold serially diluted with EMEM in another 96-well plate, with the highest antibody final concentration of 200 nM (12 gradient solutions); (4) 50ul of each of the antibody gradient solutions of (3) was added into each well of the 96-well round bottom plate of (2), mixed by pipetting; and incubated at 37°C for 30 minutes; (5) The stock solutions of ATP (Sigma, A6419) and AMP (Sigma, 01930) were added into the EMEM medium to make the concentration of ATP = 600 uM, AMP = 2 mM, 50ul of the above solution was added to each well of 96-well round bottom plate of (4), and mixed by pipetting; (6) Incubated in an incubator at 37 °C for 1 hour. (7) 50ul of Cell-Titer Glo (Promega, G7572) substrate was added to a 96-well opaque white culture plate, added with 50ul of each solution of (6), mixed and incubated at room temperature for 5 minutes. Fluorescence values were read by an i3 Multimode Reader (Molecular Divices, SpectraMax i3) (1000ms).

The results are shown in Figure 4: the anti-CD73 antibodies of the present invention can effectively inhibit the enzymatic activity of soluble CD73, thereby inhibiting the conversion of AMP to adenosine. For inhibition of the enzymatic activity, ADI-37505 and ADI-37506 molecules have IC50 of 0.07184 nM and 0.08974 nM at 1 hour, respectively, and the inhibitory effects exhibit dose-dependent. The Medimmune Omab antibody shows a poor inhibitory effect at high concentrations, and exhibits a hook effect.

**Example 4. Anti-CD73 antibodies of the invention bind to human CD73 overexpressed on CHO-S cells**

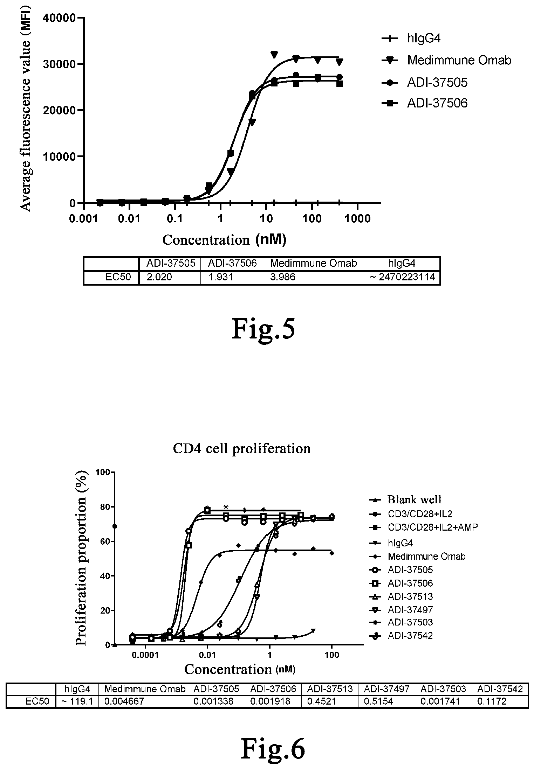

The binding ability of the antibodies of the invention to human CD73 overexpressed on the surface of CHO-S cells was measured based on flow cytometry assay. The binding ability of different antibodies was determined by comparing their binding curves to human CD73 expressed on the surface of CHO-S cells. The experimental process in detail was as follows: (1) The CHO-S cells were thawed and sub-cultured according to the conventional method to obtain cells in good condition. (2) 3-fold serially diluted candidate antibody with the concentration range from 0 to 400 mM were incubated with the CHO-S cells at 4°C for 30 minutes. After washing three times with PBS, incubated with PE-labeled goat anti-human IgG fluorescent secondary antibody (Southern biotech, 2040-09) at 4°C for 30 minutes. After washing three more times with PBS, the CHO-S cells were resuspended in 100ul PBS. (3) The median fluorescence value was measured in PE fluorescence channel by flow cytometer (BD, Celesta). (4) EC50 and the peak value of the curve was compared, and the results are shown in Figure 5.

From the above experimental results, it can be concluded that: antibodies ADI-37505 and ADI-37506 have comparable binding ability to human CD73 expressed on the surface of CHO-S cells, and their EC50 are better than thta of Medimmune Omab.

**Example 5. Anti-CD73 antibodies of the invention reverse the inhibition of T cell proliferation by CD73 and the anti-CD73 antibodies activate T cells *in vitro***

1. The anti-CD73 antibodies of the present invention reverse the inhibition of CD4+ T cell proliferation by CD73

CD73 antibody molecules can inhibit enzymatic activity of CD73 and block the conversion of AMP to adenosine, which has an immunosuppressive function, thereby reversing the inhibition of T cell proliferation caused by adenosine accumulation. Based on this principle, in this study, IL-2 and CD3/CD28 dynabeads were added to the CD4+ T cell culture system to promote T cell proliferation, and AMP was further added to inhibit the T cell proliferation. In this system, the activity of different CD73 antibody molecules in promoting T cell proliferation can be evaluated. The experimental process in detail was as follows: (1) PBMC cells were thawed and cultured overnight, on the next day, CD4+ T cells were isolated and purified by the CD4+ T Cell Enrichment Kit (stem cell, 19052) according to the manufacturer's instructions. (2) CD4+ T cells were labeled by using the Cell-Trace Far Red Live Cell Staining kit (Thermo, C34564) according to the manufacturer's instructions. (3) 20 ng/ml IL-2 (R&D, 202-IL-500) and beads: cells = 1:5 CD3/CD28 dynabeads (Gibco, 11131D) were mixed with labeled CD4+ T cells, and was then transferred to a 96-well round bottom plate and cultured still for 1 hour. (4) 0-20 nM serial dilutions of the candidate antibody molecule were added to the cells, mixed and incubated for 30 minutes. (5) AMP solution (Sigma, 01930) was prepared and added at a final concentration of 300 uM to CD4+ T cells. After mixing, placed in an incubator for 3 days. (6) The proportion of cell proliferation was detected by flow cytometry (BD, Celesta). (7) Cell proliferation curve was plotted and the EC50 and peak value of the curve were compared.

The results are shown in Figure 6: ADI-370503, ADI-37505, ADI-37506, ADI-37513, ADI-37497 and ADI-37542 can all reverse the inhibition of CD4+ T cell proliferation caused by AMP/adenosine, among which ADI-37503, ADI-37505 and ADI-37506 have a higher platform than the control antibody Omab, and their EC50 is also much lower than that of the control antibody Omab, indicating that ADI-37503, ADI-37505 and ADI-37506 have an activity superior to the control antibody Omab.

2. The anti-CD73 antibodies of the present invention reverse the inhibition of CD8+ T cell proliferation by CD73

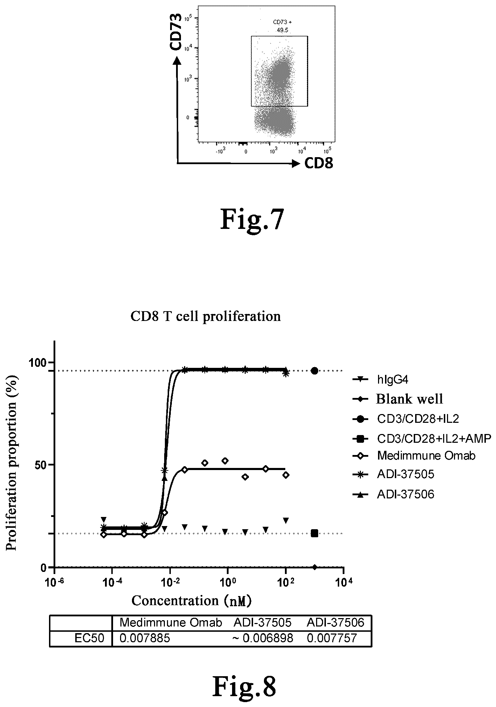

The antibody ADI-37505 of the present invention was used to detect the expression of CD73 molecule on the surface of CD8+ T cells. The experimental process in detail was as follows: (1) Human peripheral blood mononuclear cells were thawed according to the conventional method. (2) 20nM of antibody ADI-37505 and CD3 (Biolegend) and CD8 (Biolegend) antibodies were used to incubate with peripheral blood mononuclear cells at 4°C for 30 minutes. After washing three times with PBS, incubated with PE-labeled goat anti-human IgG fluorescent secondary antibody (Southern biotech) at 4°C for 30 minutes. After washing three more times with PBS, the human peripheral blood mononuclear cells were resuspended in 100ul PBS. (3) Flow cytometry (BD) assay. The results are shown in Figure 7. In CD8+ T cell population of the human peripheral blood mononuclear cell tested, 49.5% of the CD8+ T cells express CD73 molecule on the surface.

CD73 antibodies can inhibit enzymatic activity of CD73 and block the conversion of AMP to adenosine, which has an immunosuppressive function, thereby reversing the inhibition of T cell proliferation caused by adenosine accumulation. Based on this principle, in this study, 20 ng/ml IL-2 and $1 \times 10^4$ CD3/CD28 dynabeads:cells were added to the CD8+ T cell ($5 \times 10^4$/well) culture system to promote CD8+ T cell proliferation, and 1000μM AMP was further added to inhibit the T cell proliferation. In this system, the activity of different CD73 antibody molecules in promoting T cell proliferation can be evaluated. The experimental process in detail was as follows: (1) PBMC cells were thawed and cultured overnight according to the conventional method, on the next day, CD8+ T cells were isolated and purified by the CD8+ T Cell Enrichment Kit (stem cell, 19053) according to the manufacturer's instructions. (2) CD8+ T cells were labeled by using the Cell-Trace Far Red Live Cell Staining kit (Thermo) according to the manufacturer's instructions. (3) 20 ng/ml IL-2 (R&D, 202-IL-500) and beads: cells = 1:5 CD3/CD28 dynabeads (Gibco, 11131D) were mixed with labeled CD8+ T cells, and was then transferred to a 96-well round bottom plate and cultured still for 1 hour. (4) 0-100 nM serial dilutions of the candidate antibody molecule were added to the cells, mixed and incubated for 30 minutes. (5) AMP solution (Sigma, 01930) was prepared and added at a final concentration of 1000 uM to CD8+ T cells. After mixing, placed in an incubator for 3 days. (6) The proportion of cell proliferation was detected by flow cytometry (BD, Celesta). (7) Cell proliferation curve was plotted and the EC50 of each antibody and the peak value of the curve were compared.

The results are shown in Figure 8. The antibodies ADI-37505, ADI-37506 of the present invention and the control antibody Medimmune Omab can all reverse the inhibition of CD8+ T cell proliferation by AMP/adenosine. The antibodies of the present invention promote CD8+ T cell proliferation with EC50 lower than that of the control antibody Omab, and the maximum cell proliferation ratios in the treatment groups of ADI-37505 and ADI-37506 are higher than that of the control antibody Omab. Therefore, antibodies ADI-37505 and ADI-37506 have more excellent activity to promote CD8+ T cell proliferation than the control antibody Omab, wherein CD3/CD28+IL2 was used as positive control, and CD3/CD28+IL2+AMP was used as negative control.

3. Activation of T cells by the anti-CD73 antibodies of the present invention

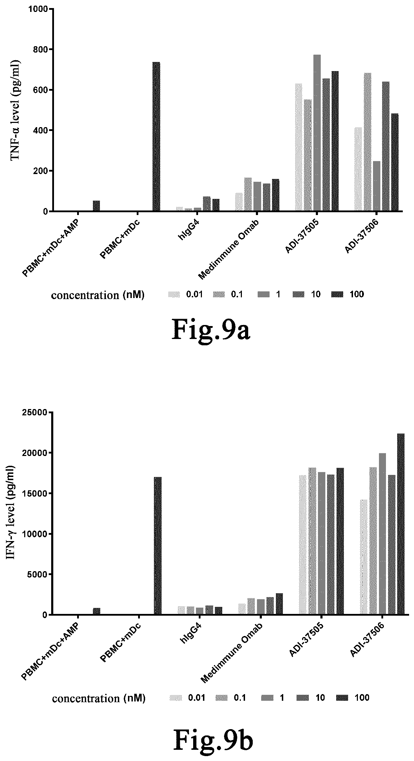

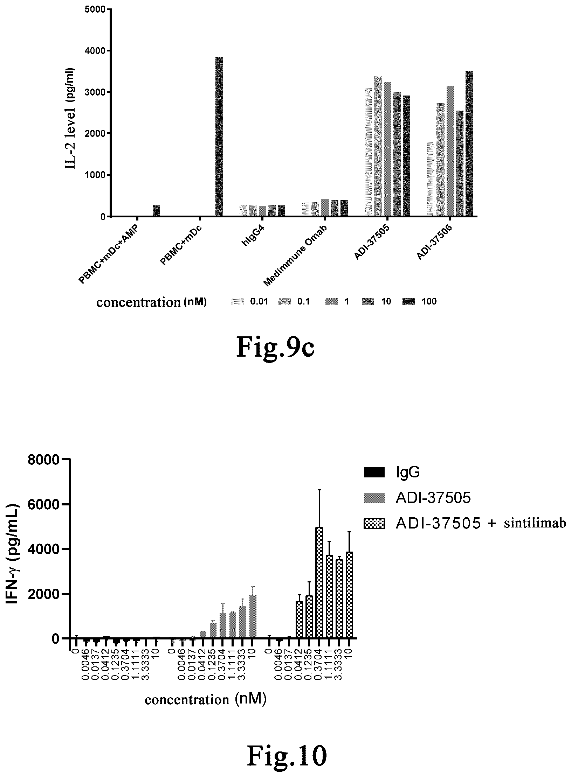

The *in vitro* effects of the antibodies ADI-37505 and ADI-37506 of the present invention on activating human T cells were detected. The experimental process in detail was as follows: the mature dendritic cells (moDC, Allcells) were thawed according to the conventional method, and mixed with peripheral blood mononuclear cells from another donor (moDC:PBMC=1:10); various gradient concentrations of the anti-CD73 antibody were then added (the concentrations are shown in Figure 9), and AMP (Sigma, 01930) was added at a final concentration of 2000 μM to the culture system. The cells were mixed and cultured for 4 days, after that, the levels of cytokines secreted in the cell culture supernatant were detected by Cytokine Detection kits TNF-α (Cisbio, 62HTNFAPEG), IFN-γ (Cisbio, 62HIFNGPEG) and IL-2 (Cisbio, 62HIL02PEG). The degree of T cell activation was assessed according to amount of the expression of cytokines.

The results are shown in Figure 9. Co-incubation of mature DC cells with PBMC cells can activate T cells to secrete cytokines TNF-α (Fig. 9a), IFN-γ (Fig. 9B) and IL-2 (Fig. 9c). When AMP was added to the system, the adenosine generated by the action of CD73 inhibited the activity of T cells. The antibodies ADI-37505 and ADI-37506 of the present invention can relieve the inhibitory effect of adenosine at different concentrations, thereby activating T cells to secrete cytokines. Various concentrations of the control antibody Medimmune Omab have poorer effect on activating T cells compared to the antibodies of the present invention.

**Example 6. Activation of antigen-specific T cells by the anti-CD73 antibody of the present invention in combination with an anti-PD-1 antibody**

The effect of the antibody ADI-37505 in combination with anti-PD-1 antibody (sintilimab, Cinda) on activating human memory T cells was detected. The experimental process in detail was as follows: the peripheral blood mononuclear cells from donors who have been infected with mumps or have been vaccinated with mumps vaccine were thawed according to the conventional method; various concentrations (starting from 10 nM, 3-fold serially diluted) of the anti-CD73 antibody and anti-PD-1 antibody sintilimab at final concentration of 10 nM were then added, and AMP (Sigma, 01930) at a final concentration of 1000 μM and Mumps (NOVUS, NBP2-62509) at a final concentration of 2 μg/Ml were added to the culture system. Mixed and cultured for 5 days. The level of cytokine secreted in the cell culture supernatant was detected by Cytokine Detection kits IFN-γ (Cisbio, 62HIFNGPEG) . The degree of T cell activation was assessed according to amount of the expression of cytokine.

The results are shown in Figure 10, co-incubation of PBMCs with Mumps can activate T cells to secrete IFN-γ cytokine. When AMP was added to the system, the adenosine generated by the action of CD73 inhibited the activity of T cells. Various concentrations of the antibody ADI-37505 of the present invention can all relieve the inhibitory effect of adenosine on T cell activation, thereby activating T cells to secrete cytokines; The activation of T cells are further increased by combining with anti-PD-1 antibody.

**Example 7. Anti-tumor activity of the anti-CD73 antibodies of the present invention**

In this experiment, the anti-tumor effect of the anti-CD73 antibody of the present invention was determined by using NOG mice (Beijing Weitong Lihua Laboratory Animal Technology Co., Ltd.) inoculated with MDA-MB-231 cells (ATCC, HTB-26).

Cells:

The peripheral blood mononuclear cells were thawed according to the conventional method, counted, collected by centrifugation, and diluted to $1\times10^7$ cells/ml with PBS. Each mouse was inoculated with 0.2ml of PBMC suspension intravenously, *i.e.* $2\times10^6$ cells/mouse.

MDA-MB-231 cells were routinely subcultured. 5 days after inoculation of peripheral blood mononuclear cells, MDA-MB-231 cells were collected by trypsinization and centrifugation, and the cells were dispersed with PBS + Matrigel (CORNING, 356231) (1:1) to a concentration of $2.5\times10^7$ cells/ml. Each mouse was subcutaneously inoculated with 0.2 ml of MDA-MB-231 cell suspension (*i.e.*, $5\times10^6$ per mouse) into the right abdominal region of NOG mice.

Dosing:

On day 1, the mice were randomly divided into groups (7 mice in each group) after tumor cells were inoculated. The drugs were administered on days 1, 8, 12, 15, and 19 after inoculation (the adopted antibodies, the positive control antibody, the negative control antibody, and the concentrations thereof are shown in Table 4), and the tumor volume and body weight of mice were monitored twice a week. The monitoring was ended until day 29. The relative tumor inhibition rate (TGI%) was calculated on day 29 after inoculation, and the calculation formula was as follows:

$$TGI\% = 100\% \text{ (tumor volume in the h-IgG control group – tumor volume in the treatment group)/(tumor volume in the h-IgG control group – initial tumor volume in the h-IgG control group)}.$$

Measurement of tumor volume: the largest long axis (L) and largest wide axis (W) of the tumor were measured by vernier caliper, and the tumor volume was calculated according to the following formula: $V = L\times W^2/2$. Body weights were measured using an electronic balance. Mice were euthanized when they lost >20% body weight throughout the study period.

Statistical results and analysis:

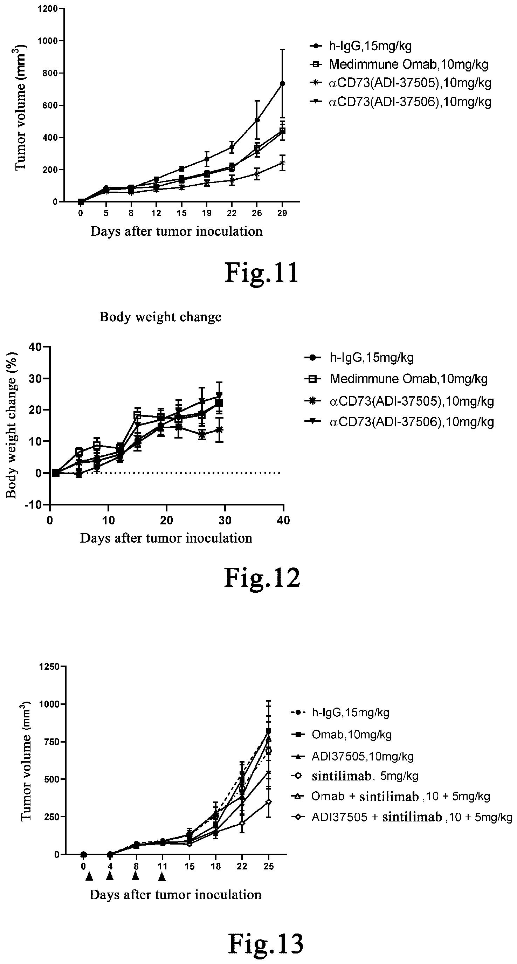

The results of tumor inhibition rate are shown in Figure 11 and Table 4: the tumor size was summarized on day 29, and the tumor inhibition rate was calculated. In contrast with the h-IgG, the tumor inhibition rates of the control antibody Medimmune Omab, ADI-37505 and ADI-37506 are 45%, 76% and 46%, respectively. The tumor inhibitory effect of the antibody ADI-37505 is significantly excellent than that of the control antibody. At the same time, the body weights of the mice were detected, and the results are shown in Figure 12. No significant weight loss was found in the mice of dosing group.

Table 4. Tumor inhibition rate on day 29 of inoculation

| Group | Tumor volume (mm$^3$) | Tumor inhibition rate (%) |
|---|---|---|
| h-IgG, 15 mg/kg | 735 | N/A |
| Medimmune Omab, 10 mg/kg | 442 | 45 |
| 37505, 10 mg/kg | 242 | 76 |
| 37506, 10 mg/kg | 435 | 46 |

**Example 8. Anti-tumor efficacy of the anti-CD73 antibody of the present invention in combination with an anti-PD-1 antibody in an A375 tumor-bearing humanized mouse model**

The peripheral blood mononuclear cells were thawed according to the conventional method, counted, collected by centrifugation, and diluted to $1\times10^7$ cells/ml with PBS. Each mouse was inoculated with 0.2ml of PBMC suspension intravenously, *i.e.* $2\times10^6$ cells/mouse.

Human malignant melanoma cell A375 (ATCC, CRL-1619™) were routinely subcultured. 5 days after inoculation of peripheral blood mononuclear cells, A375 cells were collected by trypsinization and centrifugation, and the cells were dispersed with PBS to $3\times10^7$ cells/ml. Each mouse was subcutaneously inoculated with 0.2 ml of A375 cell suspension (*i.e.*, $6\times10^6$ per mouse) into the right abdominal region of NOG mice.

Dosing:

On day 1, the mice were randomly divided into groups (8 mice in each group) after melanoma cells were inoculated. The drugs were administered on days 1, 4, 8, and 11 after inoculation (the adopted antibody, the positive control antibody, the negative control antibody, and the concentrations thereof are shown in Table 5), and the tumor volume and body weight of mice were monitored twice a week. The monitoring was ended until day 25. The relative tumor inhibition rate (TGI%) was calculated on day 25 after inoculation, and the calculation formula was as follows:

$$TGI\% = 100\% \text{ (tumor volume in the hIgG control group – tumor volume in the treatment group)/(tumor volume in the h-IgG control group – initial tumor volume in the h-IgG control group)}.$$

Measurement of tumor volume: the largest long axis (L) and largest wide axis (W) of the tumor were measured by vernier caliper, and the tumor volume was calculated according to the following formula: $V = L\times W^2/2$. Body weights were measured using an electronic balance. Mice were euthanized when they lost >20% body weight throughout the study period.

Statistical results and analysis:

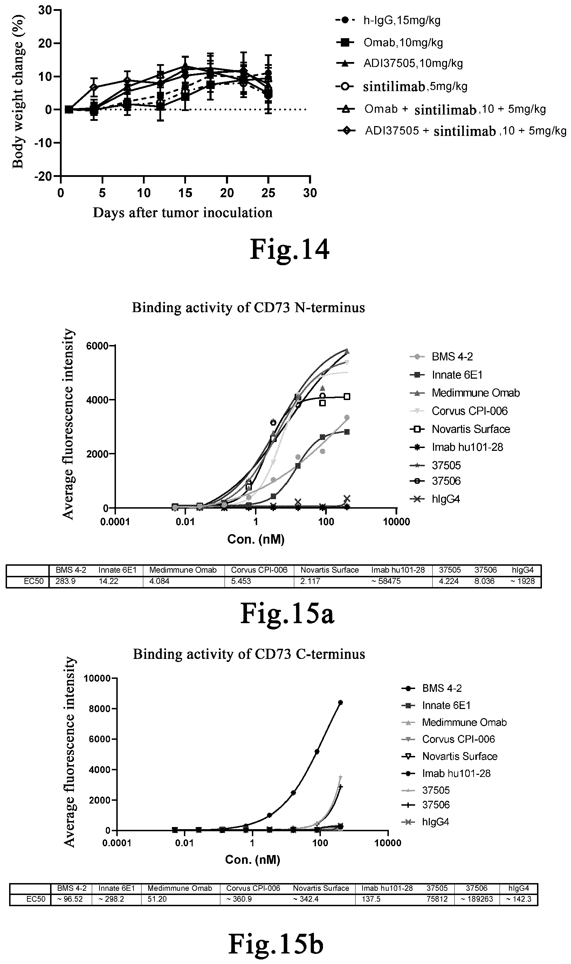

The results of tumor inhibition rate are shown in Figure 13 and Table 5: the tumor size was summarized on day 25, and the tumor inhibition rate was calculated. In contrast to h-IgG, 10 mg/kg control antibody Omab does not show antitumor efficacy against A375; the tumor inhibition rate in the ADI-37505 treatment group is 33%; the tumor inhibition of sintilimab is 15%; the tumor inhibition rate of the combination therapy of ADI-37505 and PD-1 antibody (sintilimab) is 61%, which is significantly excellent than that of each single drug. At the same time, the body weights of the mice were detected, and the results are shown in Figure 14. No significant change in body weight was found in the mice of dosing group.

Table 5. Tumor inhibition rate on day 25 after inoculation

| Group | Tumor volume (mm3) | Tumor inhibition rate (%) |
|---|---|---|
| h-IgG, 15 mg/kg | 817 | N/A |
| Omab, 10 mg/kg | 823 | no |
| ADI37505, 10 mg/kg | 551 | 33 |
| $\alpha$PD-1 (sintilimab), 5 mg/kg | 691 | 15 |
| ADI37505 + $\alpha$PD-1 (sintilimab), 10 + 5 mg/kg | 350 | 61 |

**Example 9. Study on the binding site of the anti-CD73 antibodies of the present invention to human CD73 molecule**

Compared with the control antibody, the CD73 antibodies of the present invention exhibit excellent *in vitro* function, and the CD73 antibodies of the present invention are speculated to have a unique binding site with the CD73 molecule. Fortebio results show that the CD73 antibodies of the present invention (ADI-37505 and ADI-37506), Innate 6E1, BMS 4-2 and Surface bind to the same bin of CD73 molecule as Omab, while CPI-006 and Imab hu101-28 bind to different bins respectively (Table 5). The binding of various CD73 antibodies to CHO-S cells over-expressing N-terminus (amino acids 27-317) and C-terminus (amino acids 337-549) of CD73 was further examined in the study.

The binding ability of the exemplary antibodies of the invention to the N-terminal or C-terminal domain of human CD73 expressed on the surface of GS-CHO cells was measured based on flow cytometry assay. The specific experimental process in detail was as follows: (1) Construction of pLVX-IRES-puro-human-CD plasmid (Suzhou Hongxun Biotechnology Co., Ltd.) expressing N-terminus (27-317 amino acids) and C-terminus (337-549 amino acids) of CD 73 for transient transfection of GS CHO cells; (2) After 48h, various concentrations of ADI-37505, ADI-37506, and control antibodies were incubated with the transiently transfected cells at 4°C for 30 minutes. (3) After washing three times with PBS (Gibco, 10010-023), incubated with PE-labeled goat anti-human secondary antibody (SouthernBiotech, 2040-09) at 4°C for 30 minutes. After washing three more times with PBS, the cells were resuspended in 100ul PBS. (4) The average fluorescence value was measured in PE channel by flow cytometer (BD, Celesta). The Logic of antibody concentration was used as the horizontal coordinate and the average fluorescence value of the PE channel was used as the vertical coordinate to plot a curve, and the EC50 and the peak of the curve were compared.

The results are shown in Figure 15, the CD73 antibodies of the present invention (ADI-37505 and ADI-37506), Innate 6E1, BMS 4-2, Surface, Omab and CPI-006 bind to the N-terminus of CD73 molecule, while Imab hu101-28 binds to the C-terminus of CD73 molecule.

According to this result, 25 N-terminal mutants of CD73 molecule were constructed (Table 6) in the study to further confirm which amino acid mutations can affect the binding of CD73 antibody to CD73 molecule. The specific experimental process in detail was as follows: (1) Construction of pLVX-IRES-puro-human-CD plasmids of N-terminal specific site mutations of CD73 molecule as indicated in Figure 6, for transient transfection of GS CHO cells; (2) After 48h, various concentrations of candidate antibody were incubated with the transiently transfected cells at 4°C for 30 minutes. (3) After washing three times with PBS (Gibco, 10010-023), incubated with PE-labeled goat anti-human secondary antibody (SouthernBiotech, 2040-09) at 4°C for 30 minutes. After washing three more times with PBS, the cells were resuspended in 100ul PBS. (4) The average fluorescence value was measured in PE channel by flow cytometer. The Logic of antibody concentration was used as the horizontal coordinate and the average fluorescence value of the PE channel was used as the vertical coordinate to plot a curve, and the EC50 and the peak of the curve were compared.

Table 6. N-terminal mutants of CD73 molecule

|    | CD73 mutants           |
|----|------------------------|
| 1  | K145A/K147A            |
| 2  | S152A/Q153A            |
| 3  | Y161A/K162A/V170A      |
| 4  | K179A/E180A            |
| 5  | F183A/L184A/N186A      |
| 6  | E194A/E196A            |
| 7  | Q201A/E203A            |
| 8  | K206A/N211A            |
| 9  | E224A/K227A            |
| 10 | K232A/R234A            |
| 11 | K291A/E293A            |
| 12 | E296A/R297A            |
| 13 | K136A/Y161A/K162A/V170A |
| 14 | V163K                  |
| 15 | V170K                  |
| 16 | L159A/Y161A/K162A/V170A |
| 17 | L159E                  |
| 18 | L210F                  |
| 19 | L157A/S155A/L159A      |
| 20 | L157A/S155A/L159A/Q153A |
| 21 | R109A/K136A            |

(continued)

|  | CD73 mutants |
|---|---|
| 22 | R109A/K136A/E134A |
| 23 | F65A/H103A |
| 24 | H103A/W327A |
| 25 | F265A/H304A |

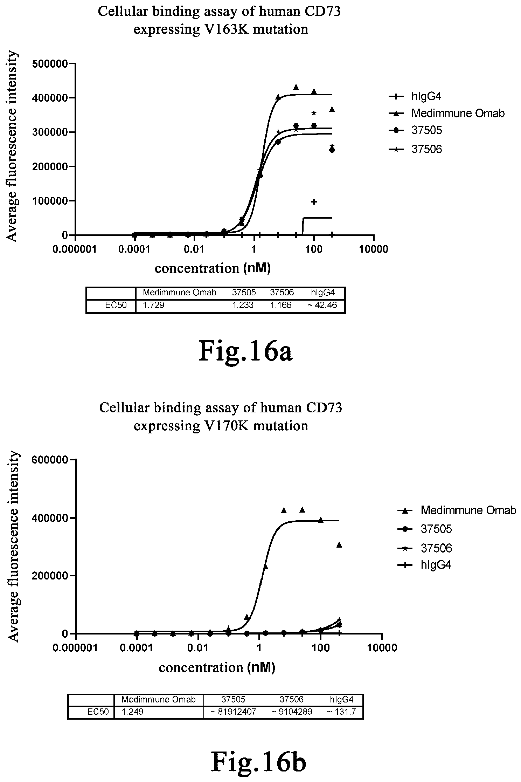

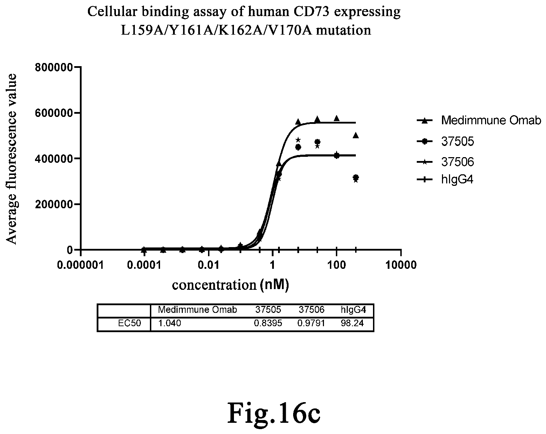

The detection results are shown in Figure 16: CD73 molecules with V163K, V170K and L159A/Y161A/K162A/V170A mutations have reduced binding ability to the antibodies ADI-37505 and ADI-37506 of the present invention, while the other 22 mutants normally bind to the antibodies of the present invention. It is therefore presumed that the CD73 binding epitope to which ADI-37505 and ADI-37506 bind is located at amino acids 159, 161, 162, 163 and 170 of CD73 molecule.

As shown in Table 7, Innate 6E1, BMS 4-2, Surface, Omab and CPI-006 have attenuated binding ability to the corresponding mutants of CD73 molecules, whereas these mutants normally bind to the CD73 antibody molecules of the present invention. It is therefore speculated that the CD73 binding epitope to which the CD73 antibodies of the present invention bind is not exactly the same as that of Innate 6E1, BMS 4-2, Surface, Omab and CPI-006.

**Table 7. Analysis of the difference in human CD73 molecule binding between the antibodies of the present invention and the control antibody**

|  | Bin number | CD73 | CD73 variants affecting binding |
|---|---|---|---|
| 4-2 (BMS) | 1 | N-terminus | R109A/K136A<br>R109A/K136A/E134A |
|  |  |  | K136A/Y161A/K162A/V170A<br>L159A/Y161A/K162A/V170A<br>L159E<br>L210F<br>F265A/H304A |
| 6E1 (Innate) | 1 | N-terminus | Y161A/K162A/V170A<br>L157A/S155A/L159A<br>L157A/S155A/L159A/Q153A<br>H103A/W327A<br>K136A/Y161A/K162A/V170A<br>V163K<br>L159A/Y161A/K162A/V170A<br>L159E |
| CPI-006 (Corvus) | 2 | N-terminus | K232A/R234A<br>E296A/R297A<br>K136A/Y161A/K162A/V170A |
| Omab (Medimmune) | 1 | N-terminus | K206A/N211A |
| Surface (Norvatis) | 1 | N-terminus | Y161A/K162A/V170A<br>K136A/Y161A/K162A/V170A<br>V163K<br>V170K<br>L159A/Y161A/K162A/V170A |
| Hu101-28 (I mab) | 3 | C-terminus |  |
| 37505 | 1 | N-terminus | V163K<br>V170K<br>L159A/Y161A/K162A/V170A |

(continued)

|  | Bin number | CD73 | CD73 variants affecting binding |
|---|---|---|---|
| 37506 | 1 | N-terminus | V163K<br>V170K<br>L159A/Y161A/K162A/V170A |

**Claims**

1. An anti-CD73 antibody or antigen-binding fragment thereof, comprising

   (i) three complementarity determining region HCDRs of the heavy chain variable region as shown in SEQ ID NO:55, and three complementarity determining region LCDRs of the light chain variable region as shown in SEQ ID NO: 91, or
   (ii) three complementarity determining region HCDRs of the heavy chain variable region as shown in SEQ ID NO:56, 57 or 58, and three complementarity determining region LCDRs of the light chain variable region as shown in SEQ ID NO: 92, or
   (iii) three complementarity determining region HCDRs of the heavy chain variable region as shown in any one of SEQ ID NOs:59-64, and three complementarity determining region LCDRs of the light chain variable region as shown in SEQ ID NO: 93, or
   (iv) three complementarity determining region HCDRs of the heavy chain variable region as shown in SEQ ID NO:65, 66 or 67, and three complementarity determining region LCDRs of the light chain variable region as shown in SEQ ID NO: 94, or
   (v) three complementarity determining region HCDRs of the heavy chain variable region as shown in SEQ ID NO:68, and three complementarity determining region LCDRs of the light chain variable region as shown in SEQ ID NO: 95, or
   (vi) three complementarity determining region HCDRs of the heavy chain variable region as shown in SEQ ID NO:69, and three complementarity determining region LCDRs of the light chain variable region as shown in SEQ ID NO: 96, or
   (vii) three complementarity determining region HCDRs of the heavy chain variable region as shown in SEQ ID NO:70, 71 or 72, and three complementarity determining region LCDRs of the light chain variable region as shown in SEQ ID NO: 97.

2. An anti-CD73 antibody or antigen-binding fragment thereof, comprising three complementarity determining region HCDRs of the heavy chain variable region, and three complementary determining region LCDRs of the light chain variable region, wherein the HCDR1 comprises or consists of the amino acid sequence as shown in any one of SEQ ID Nos:1-15, 132-133 or 136-149, the HCDR2 comprises or consists of the amino acid sequence as shown in any one of SEQ ID Nos: 16-30, 134 or 135, the HCDR3 comprises or consists of the amino acid sequence as shown in any one of SEQ ID Nos: 31-37 or 150-156, the LCDR1 comprises or consists of the amino acid sequence as shown in any one of SEQ ID Nos: 38-43, the LCDR2 comprises or consists of the amino acid sequence as shown in any one of SEQ ID Nos: 44-48, and the LCDR3 comprises or consists of the amino acid sequence as shown in any one of SEQ ID Nos: 49-54.

3. An anti-CD73 antibody or antigen-binding fragment thereof, comprising a heavy chain variable region and/or a light chain variable region, wherein

   the heavy chain variable region comprises:

   (i) three complementarity determining regions (HCDRs) comprised in the VH of any antibody listed in Table B; or
   (ii) a combination of HCDR1, HCDR2 and HCDR3 as shown in Table A or Table D;
   and/or

   the light chain variable region comprises:

   (i) three complementarity determining regions (LCDRs) comprised in the VL of any antibody listed in Table B; or
(ii) a combinations of LCDR1, LCDR2 and LCDR3 as shown in Table A or Table D.

4. The anti-CD73 antibody or antigen-binding fragment thereof of any one of claims 1-3, comprising a heavy chain variable region VH and/or a light chain variable region VL, wherein,

(a) the heavy chain variable region VH

(i) comprises an amino acid sequence having at least 90%, 91%, 92%, 93%, 94%, 95%, 96%, 97%, 98% or 99% identity to the amino acid sequence as shown in any one of SEQ ID Nos: 55-72; or
(ii) comprises or consists of the amino acid sequence as shown in any one of SEQ ID Nos: 55-72; or
(iii) comprises or consists of an amino acid sequence having one or more (preferably no more than 10, more preferably no more than 5, 4, 3, 2, 1) amino acid changes (preferably amino acid substitutions, insertion or deletions, more preferably amino acid conservative substitutions) compared to the amino acid sequence as shown in any one of SEQ ID NOs: 55-72, preferably, the amino acid change does not occur in the CDR regions;
and/or

(b) the light chain variable region VL

(i) comprises or consists of an amino acid sequence having at least 90%, 91%, 92%, 93%, 94%, 95%, 96%, 97%, 98% or 99% identity to the amino acid sequence as shown in any one of SEQ ID Nos: 91-97; or
(ii) comprises or consists of the amino acid sequence as shown in any one of SEQ ID Nos: 91-97; or
(iii) comprises or consists of an amino acid sequence having one or more (preferably no more than 10, more preferably no more than 5, 4, 3, 2, 1) amino acid changes (preferably amino acid substitutions, insertion or deletions, more preferably amino acid conservative substitutions) compared to the amino acid sequence as shown in any one of SEQ ID NOs: 91-97.

5. The anti-CD73 antibody or antigen-binding fragment thereof of any one of claims 1-4, comprising

(i) a heavy chain variable region comprising or consisting of an amino acid sequence having at least 90% sequence identity to the amino acid sequence as shown in SEQ ID NO: 55, and/or a light chain variable region comprising or consisting of an amino acid sequence having at least 90% sequence identity to the amino acid sequence as shown in SEQ ID NO: 91, or
(ii) a heavy chain variable region comprising or consisting of an amino acid sequence having at least 90% sequence identity to the amino acid sequence as shown in SEQ ID NO: 56, 57 or 58, and/or a light chain variable region comprising or consisting of an amino acid sequence having at least 90% sequence identity to the amino acid sequence as shown in SEQ ID NO: 92, or
(iii) a heavy chain variable region comprising or consisting of an amino acid sequence having at least 90% sequence identity to the amino acid sequence as shown in any one of SEQ ID NOs: 59-64, and/or a light chain variable region comprising or consisting of an amino acid sequence having at least 90% sequence identity to the amino acid sequence as shown in SEQ ID NO: 93, or
(iv) a heavy chain variable region comprising or consisting of an amino acid sequence having at least 90% sequence identity to the amino acid sequence as shown in SEQ ID NO: 65, 66 or 67, and/or a light chain variable region comprising or consisting of an amino acid sequence having at least 90% sequence identity to the amino acid sequence as shown in SEQ ID NO: 94, or
(iv) a heavy chain variable region comprising or consisting of an amino acid sequence having at least 90% sequence identity to the amino acid sequence as shown in SEQ ID NO: 68, and/or a light chain variable region comprising or consisting of an amino acid sequence having at least 90% sequence identity to the amino acid sequence as shown in SEQ ID NO: 95, or
(iv) a heavy chain variable region comprising or consisting of an amino acid sequence having at least 90% sequence identity to the amino acid sequence as shown in SEQ ID NO: 69, and/or a light chain variable region comprising or consisting of an amino acid sequence having at least 90% sequence identity to the amino acid sequence as shown in SEQ ID NO: 96, or
(iv) a heavy chain variable region comprising or consisting of an amino acid sequence having at least 90% sequence identity to the amino acid sequence as shown in SEQ ID NO: 70, 71 or 72, and/or a light chain variable region comprising or consisting of an amino acid sequence having at least 90% sequence identity to the amino acid sequence as shown in SEQ ID NO: 97.

6. The isolated anti-CD73 antibody or antigen-binding fragment thereof of any one of claims 1-5, comprising a heavy chain and/or a light chain, wherein,

(a) the heavy chain

(i) comprises an amino acid sequence having at least 85%, 90%, 91%, 92%, 93%, 94%, 95%, 96%, 97%, 98% or 99% identity to the amino acid sequence as shown in any one of SEQ ID Nos: 106-123 and comprises the corresponding CDR sequences of the sequence as shown in any one of SEQ ID Nos: 106-123;
(ii) comprises or consists of the amino acid sequence as shown in any one of SEQ ID NOs: 106-123; or
(iii) comprises an amino acid sequence having one or more (preferably no more than 20 or 10, more preferably no more than 5, 4, 3, 2, 1) amino acid changes (preferably amino acid substitutions, more preferably conservative amino acid substitutions) compared to the amino acid sequence as shown in any one of SEQ ID NOs: 106-123, preferably, the amino acid changes do not occur in the heavy chain CDR regions, more preferably, the amino acid changes do not occur in the heavy chain variable region; and/or

(b) the light chain

(i) comprises an amino acid sequence having at least 85%, 90%, 91%, 92%, 93%, 94%, 95%, 96%, 97%, 98% or 99% identity to the amino acid sequence as shown in any one of SEQ ID Nos: 124-130 and comprises the corresponding CDR sequences of the sequence as shown in any one of SEQ ID Nos: 124-130;
(ii) comprises or consists of the amino acid sequence as shown in any one of SEQ ID NOs: 124-130; or
(iii) comprises an amino acid sequence having one or more (preferably no more than 20 or 10, more preferably no more than 5, 4, 3, 2, 1) amino acid changes (preferably amino acid substitutions, more preferably conservative amino acid substitutions) compared to the amino acid sequence as shown in any one of SEQ ID NOs: 124-130, preferably, the amino acid changes do not occur in the light chain CDR regions, more preferably, the amino acid changes do not occur in the light chain variable region.

7. The anti-CD73 antibody or antigen-binding fragment thereof of any one of claims 1-6, comprising:

(i) a heavy chain comprising or consisting of an amino acid sequence having at least 90% sequence identity to the amino acid sequence as shown in SEQ ID NO: 106, and/or a light chain comprising or consisting of an amino acid sequence having at least 90% sequence identity to the amino acid sequence as shown in SEQ ID NO: 124, or
(ii) a heavy chain comprising or consisting of an amino acid sequence having at least 90% sequence identity to the amino acid sequence as shown in any one of SEQ ID NOs: 107-109, and/or a light chain comprising or consisting of an amino acid sequence having at least 90% sequence identity to the amino acid sequence as shown in SEQ ID NO: 125, or
(iii) a heavy chain comprising or consisting of an amino acid sequence having at least 90% sequence identity to the amino acid sequence as shown in any one of SEQ ID NOs: 110-115, and/or a light chain comprising or consisting of an amino acid sequence having at least 90% sequence identity to the amino acid sequence as shown in SEQ ID NO: 126, or
(iv) a heavy chain comprising or consisting of an amino acid sequence having at least 90% sequence identity to the amino acid sequence as shown in SEQ ID NOs: 116-118, and/or a light chain comprising or consisting of an amino acid sequence having at least 90% sequence identity to the amino acid sequence as shown in SEQ ID NO: 127,
(v) a heavy chain comprising or consisting of an amino acid sequence having at least 90% sequence identity to the amino acid sequence as shown in SEQ ID NO: 119, and/or a light chain comprising or consisting of an amino acid sequence having at least 90% sequence identity to the amino acid sequence as shown in SEQ ID NO: 128, or
(vi) a heavy chain comprising or consisting of an amino acid sequence having at least 90% sequence identity to the amino acid sequence as shown in SEQ ID NO: 120, and/or a light chain comprising or consisting of an amino acid sequence having at least 90% sequence identity to the amino acid sequence as shown in SEQ ID NO: 129, or
(vii) a heavy chain comprising or consisting of an amino acid sequence having at least 90% sequence identity to the amino acid sequence as shown in any one of SEQ ID NOs: 121-123, and/or a light chain comprising or consisting of an amino acid sequence having at least 90% sequence identity to the amino acid sequence as shown in SEQ ID NO: 130.

8. The anti-CD73 antibody or antigen-binding fragment thereof of any one of claims 1 to 7, wherein the antibody is a humanized or human antibody.

9. The anti-CD73 antibody or antigen-binding fragment thereof of any one of claims 1 to 8, wherein the antigen-binding fragment is selected from the group consisting of Fab, Fab', Fab'-SH, Fv, single chain antibody (e.g. scFv) or ( Fab')2, single domain antibody, diabody (dAbs), camelid antibody (heavy chain antibody) or linear antibody.

10. The anti-CD73 antibody or antigen-binding fragment thereof of any one of claims 1 to 9, comprising a framework sequence, wherein at least a part of the framework sequence is human consensus framework sequence.

11. An anti-CD73 antibody or antigen-binding fragment thereof, which competes with the antibody of any one of claims 1 to 10 for binding to CD73, or antagonizes or blocks the binding of the antibody of any one of claims 1 to 10 to CD73.

12. The anti-CD73 antibody or antigen-binding fragment thereof of any one of claims 1 to 10, having one or more properties of:

   (1) binding to CD73 (*e.g.,* human CD73) with high affinity, *e.g.*, with an equilibrium dissociation constant (KD) of less than about 100 nM;
   (2) blocking the enzymatic activity of the soluble CD73;
   (3) reversing the inhibition of CD4+ T cell proliferation by AMP-adenosine;
   (4) reversing the inhibition of CD8+ T cell proliferation by AMP-adenosine;
   (5) reversing the inhibition of T cell activity by AMP-adenosine.

13. A nucleic acid encoding the anti-CD73 antibody or antigen-binding fragment thereof of any one of claims 1 to 12.

14. A vector comprising the nucleic acid of claim 13, preferably the vector is an expression vector.

15. A host cell comprising the vector of claim 14, preferably, the host cell is a prokaryotic cell or a eukaryotic cell; more preferably, the host cell is selected from a yeast cell, mammalian cell or other cells suitable for preparing antibodies or antigen binding fragments thereof, preferably CHO cell and 293 cell.

16. A method of preparing an anti-CD73 antibody or antigen-binding fragment thereof, comprising culturing the host cell of claim 15 under a condition suitable for expressing the nucleic acid encoding the anti-CD73 or antigen-binding fragment thereof according to any one of claims 1 to 12, optionally isolating the antibody or antigen-binding fragment thereof, and optionally the method further comprising recovering the anti-CD73 antibody or antigen-binding fragment thereof from the host cell.

17. An anti-CD73 antibody or antigen-binding fragment thereof prepared by the method of claim 16.

18. A pharmaceutical composition comprising the anti-CD73 antibody or antigen-binding fragment thereof of any one of claims 1 to 12 and 17, and optionally a pharmaceutically acceptable carrier.

19. An immunoconjugate comprising the anti-CD73 antibody or antigen-binding fragment thereof according to any one of claims 1 to 12 and 17.

20. A pharmaceutical combination comprising the anti-CD73 antibody or antigen-binding fragment thereof of any one of claims 1-12 and 17 and an anti-PD1 antibody, preferably the anti-PD1 antibody is sintilimab.

21. Use of the anti-CD73 antibody or antigen-binding fragment thereof of any one of claims 1-12 and 17 in the manufacture of a medicament for the treatment of cancer or tumor.

22. Use of the pharmaceutical combination of claim 20 in the manufacture of a medicament for the treatment of cancer or tumor.

23. The use of claims 21-22, wherein the cancer or tumor is breast cancer, lung cancer or melanoma.

24. Use of the anti-CD73 antibody or antigen-binding fragment thereof of any one of claims 1-12 and 17 or the pharmaceutical combination of claim 20 in the manufacture of a medicament for reversing the inhibition of T cell proliferation by CD73.

25. The use of claim 24, wherein the T cell is CD4+ T cell or CD8+ T cell.

26. Use of the anti-CD73 antibody or antigen-binding fragment thereof of any one of claims 1-12 and 17 or the pharmaceutical combination of claim 20 in the manufacture of a medicament for activating T cell activity.

27. An antibody-binding epitope of CD73, comprising a fragment of amino acids 159-170 shown in SEQ ID NO: 131, preferably, the epitope comprises amino acids 159, 161, 162, 163 and 170 shown in SEQ ID NO: 131.

28. A method of preventing and/or treating a CD73-related disease or disorder, such as cancer, comprising administering to a subject an effective amount of the antibody or antigen-binding fragment thereof according to any one of claims 1-12 and 17, the pharmaceutical composition of claim 18 or the immunoconjugate of claim 19 or the pharmaceutical combination of claim 20.

29. A method for detecting CD73 in a sample, the method comprising

(a) contacting the sample with the antibody or antigen-binding fragment thereof of any one of claims 1-12 and 17; and
(b) detecting the formation of a complex between the antibody or antigen-binding fragment thereof and CD73; optionally, the antibody is detectably labeled.

**Fig.1**

**Fig.2a**

**Fig.2b**

| | Medimmune Omab | ADI-37505 | ADI-37506 | hIgG4 | Blank well |
|---|---|---|---|---|---|
| IC50 | 3.869 | 1.470 | 1.270 | ~ 6.914e-015 | 0.004109 |

Fig.3

| | Medimmune Omab | ADI-37505 | ADI-37506 | hIgG4 |
|---|---|---|---|---|
| IC50 | 0.08223 | 0.07184 | 0.08974 | ~ 35933366393334 |

Fig.4

| | ADI-37505 | ADI-37506 | Medimmune Omab | hIgG4 |
|---|---|---|---|---|
| EC50 | 2.020 | 1.931 | 3.986 | ~ 2470223114 |

## Fig.5

CD4 cell proliferation

| | hIgG4 | Medimmune Omab | ADI-37505 | ADI-37506 | ADI-37513 | ADI-37497 | ADI-37503 | ADI-37542 |
|---|---|---|---|---|---|---|---|---|
| EC50 | ~ 119.1 | 0.004667 | 0.001338 | 0.001918 | 0.4521 | 0.5154 | 0.001741 | 0.1172 |

## Fig.6

Fig.7

CD8 T cell proliferation

| | Medimmune Omab | ADI-37505 | ADI-37506 |
|---|---|---|---|
| EC50 | 0.007885 | ~ 0.006898 | 0.007757 |

Fig.8

Fig.9a

Fig.9b

Fig.9c

Fig.10

Fig.11

Body weight change

Fig.12

Fig.13

Fig.14

Binding activity of CD73 N-terminus

| | BMS 4-2 | Innate 6E1 | Medimmune Omab | Corvus CPI-006 | Novartis Surface | Imab hu101-28 | 37505 | 37506 | hIgG4 |
|---|---|---|---|---|---|---|---|---|---|
| EC50 | 283.9 | 14.22 | 4.084 | 5.453 | 2.117 | ~ 58475 | 4.224 | 8.036 | ~ 1928 |

Fig.15a

Binding activity of CD73 C-terminus

| | BMS 4-2 | Innate 6E1 | Medimmune Omab | Corvus CPI-006 | Novartis Surface | Imab hu101-28 | 37505 | 37506 | hIgG4 |
|---|---|---|---|---|---|---|---|---|---|
| EC50 | ~ 96.52 | ~ 298.2 | 51.20 | ~ 360.9 | ~ 342.4 | 137.5 | 75812 | ~ 189263 | ~ 142.3 |

Fig.15b

Cellular binding assay of human CD73
expressing V163K mutation

| | | Medimmune Omab | 37505 | 37506 | hIgG4 |
|---|---|---|---|---|---|
| EC50 | | 1.729 | 1.233 | 1.166 | ~ 42.46 |

# Fig.16a

Cellular binding assay of human CD73
expressing V170K mutation

| | | Medimmune Omab | 37505 | 37506 | hIgG4 |
|---|---|---|---|---|---|
| EC50 | | 1.249 | ~ 81912407 | ~ 9104289 | ~ 131.7 |

# Fig.16b

Cellular binding assay of human CD73 expressing
L159A/Y161A/K162A/V170A mutation

| | Medimmune Omab | 37505 | 37506 | hIgG4 |
|---|---|---|---|---|
| EC50 | 1.040 | 0.8395 | 0.9791 | 98.24 |

Fig.16c

<table>
<tr><td colspan="2" align="center">**INTERNATIONAL SEARCH REPORT**</td><td>International application No.<br>**PCT/CN2021/101233**</td></tr>
</table>

**A. CLASSIFICATION OF SUBJECT MATTER**

C07K 16/28(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K A61P

Documentation searched other than minimum documentation to the extent that such documents are included in the fields searched

Electronic data base consulted during the international search (name of data base and, where practicable, search terms used)

CNABS, CNTXT, SIPOABS, DWPI, VEN, WOTXT, USTXT, EPTXT, CNKI, 万方数据库, baidu学术, PUBMED, ELSEVIER, SPRINGER, ISI web of knowledge, sciencedirect, NCBI, Genbank, EMBL, STN, China Patents Biological Sequence Search System: CD73, 分化簇73, 胞外-5'-核苷酸酶, 胞外-5'-NT, 5'-nucleotidase, anti-cd73, cluster of differentiation-73, ecto-5'- nucleotidase, 抗体, 抗原结合片段, antibody, 表位, epitope, pd-1, 信达生物制药（苏州）有限公司, innovent, 荆华, 沈浩然, 唐蓉, 刘军建, Hua jin, Haoran shen, Rong tang, Junjian liu, SEQ ID NOs: 1-72, 91-97, 132-156

**C. DOCUMENTS CONSIDERED TO BE RELEVANT**

| Category* | Citation of document, with indication, where appropriate, of the relevant passages | Relevant to claim No. |
|---|---|---|
| X | WO 2019/224025 A2 (BOEHRINGER INGELHEIM INTERNATIONAL GMBH) 28 November 2019 (2019-11-28)<br>claims 1-45, description page 2 lines 19-22, page 17 lines 1-13, page 39 line 26- page 40 line 24, page 41 line 21- page 48 page 9, page 66 lines 6-11, page 83 lines 1-24, embodiments 1-9 | 11, 13-29 |
| A | WO 2019/224025 A2 (BOEHRINGER INGELHEIM INTERNATIONAL GMBH) 28 November 2019 (2019-11-28)<br>claims 1-45, description page 2 lines 19-22, page 17 lines 1-13, page 39 line 26- page 40 line 24, page 41 line 21- page 48 page 9, page 66 lines 6-11, page 83 lines 1-24, embodiments 1-9 | 1-10, 12 |
| X | CN 109476740 A (SQUIBB BRISTOL MYERS CO.) 15 March 2019 (2019-03-15)<br>claims 1-64, description paragraphs [0858]-[0865] | 11, 13-29 |
| A | CN 109476740 A (SQUIBB BRISTOL MYERS CO.) 15 March 2019 (2019-03-15)<br>claims 1-64, description paragraphs [0858]-[0865] | 1-10, 12 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 September 2021** | **18 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2021/101233** |

### C. DOCUMENTS CONSIDERED TO BE RELEVANT

| Category* | Citation of document, with indication, where appropriate, of the relevant passages | Relevant to claim No. |
|---|---|---|
| X | CN 107001474 A (SQUIBB BRISTOL MYERS CO.) 01 August 2017 (2017-08-01) claims 1-62 | 11, 13-29 |
| A | CN 107001474 A (SQUIBB BRISTOL MYERS CO.) 01 August 2017 (2017-08-01) claims 1-62 | 1-10, 12 |
| X | WO 2019/200256 A1 (BRISTOL-MYERS SQUIBB COMPANY) 17 October 2019 (2019-10-17) claims 1-97, description pages 75-77 | 11, 13-29 |
| A | WO 2019/200256 A1 (BRISTOL-MYERS SQUIBB COMPANY) 17 October 2019 (2019-10-17) claims 1-97, description pages 75-77 | 1-10, 12 |
| X | WO 2019/173692 A2 (AGENUS INC.) 12 September 2019 (2019-09-12) claims 1-48, 113-115, 130-132, 137, 148-205, description paragraphs [00206]-[00208] | 11, 13-29 |
| A | WO 2019/173692 A2 (AGENUS INC.) 12 September 2019 (2019-09-12) claims 1-48, 113-115, 130-132, 137, 148-205, description paragraphs [00206]-[00208] | 1-10, 12 |
| X | CN 110785187 A (NOVARTIS AG et al.) 11 February 2020 (2020-02-11) claims 1-154, embodiment 2 | 11, 13-29 |
| A | CN 110785187 A (NOVARTIS AG et al.) 11 February 2020 (2020-02-11) claims 1-154, embodiment 2 | 1-10, 12 |
| PX | WO 2021/044005 A1 (SYMPHOGEN A/S) 11 March 2021 (2021-03-11) claims 1-35, description embodiment 8, table 9 | 11, 13-29 |

Form PCT/ISA/210 (second sheet) (January 2015)

## INTERNATIONAL SEARCH REPORT

| International application No. |
| --- |
| **PCT/CN2021/101233** |

**Box No. I      Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2021/101233**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **28**
   because they relate to subject matter not required to be searched by this Authority, namely:

   [1]  claim 28 relates to a method for preventing, or treating a CD73-related disease or symptom as cancer, and to a method of treating disease pursuant to PCT Rule 39.1(iv). The present report is provided on the basis of a corresponding use of the antibody and other products in the preparation of a drug.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2021/101233**

| Patent document cited in search report | | | Publication date (day/month/year) | Patent family member(s) | | | Publication date (day/month/year) |
|---|---|---|---|---|---|---|---|
| WO | 2019/224025 | A2 | 28 November 2019 | KR | 20210013135 | A | 03 February 2021 |
| | | | | AR | 115389 | A1 | 13 January 2021 |
| | | | | EA | 202092735 | A1 | 22 April 2021 |
| | | | | CN | 112166124 | A | 01 January 2021 |
| | | | | EP | 3569618 | A1 | 20 November 2019 |
| | | | | CA | 3093882 | A1 | 28 November 2019 |
| | | | | TW | 202005982 | A | 01 February 2020 |
| | | | | BR | 112020017676 | A2 | 29 December 2020 |
| | | | | EP | 3797119 | A2 | 31 March 2021 |
| | | | | US | 2019352420 | A1 | 21 November 2019 |
| | | | | AU | 2019274626 | A1 | 17 September 2020 |
| | | | | WO | 2019224025 | A3 | 02 January 2020 |
| CN | 109476740 | A | 15 March 2019 | AU | 2017228470 | A1 | 30 August 2018 |
| | | | | EA | 201891983 | A1 | 28 February 2019 |
| | | | | WO | 2017152085 | A1 | 08 September 2017 |
| | | | | BR | 112018067368 | A2 | 15 January 2019 |
| | | | | KR | 20180118725 | A | 31 October 2018 |
| | | | | AU | 2017228470 | A8 | 23 April 2020 |
| | | | | CA | 3016187 | A1 | 08 September 2017 |
| | | | | MX | 2018010473 | A | 28 September 2018 |
| | | | | JP | 2019514844 | A | 06 June 2019 |
| | | | | IL | 261395 | D0 | 31 October 2018 |
| | | | | EA | 201891983 | A8 | 28 May 2020 |
| | | | | EP | 3423494 | A1 | 09 January 2019 |
| | | | | US | 2019284293 | A1 | 19 September 2019 |
| | | | | SG | 11201806861 S | A | 27 September 2018 |
| | | | | WO | 2017152085 | A8 | 16 April 2020 |
| | | | | SG | CN 10201913033 U | A | 30 March 2020 |
| CN | 107001474 | A | 01 August 2017 | US | 2018127513 | A1 | 10 May 2018 |
| | | | | CL | 2018001414 | A1 | 24 August 2018 |
| | | | | TW | I711630 | B | 01 December 2020 |
| | | | | AR | 102698 | A1 | 15 March 2017 |
| | | | | SI | 3221363 | T1 | 30 September 2020 |
| | | | | TN | 2017000203 | A1 | 19 October 2018 |
| | | | | JP | 6805142 | B2 | 23 December 2020 |
| | | | | EP | 3725808 | A1 | 21 October 2020 |
| | | | | LT | 3221363 | T | 10 August 2020 |
| | | | | DK | 3221363 | T3 | 10 August 2020 |
| | | | | EP | 3221363 | B1 | 06 May 2020 |
| | | | | AU | 2015349878 | A1 | 25 May 2017 |
| | | | | US | 10167343 | B2 | 01 January 2019 |
| | | | | SG | 11201703192 S | A | 30 May 2017 |
| | | | | HU | E050596 | T2 | 28 December 2020 |
| | | | | RS | 60631 | B1 | 30 September 2020 |
| | | | | MX | 2017006624 | A | 21 August 2017 |
| | | | | MA | 40309 | A1 | 31 January 2018 |
| | | | | US | 9605080 | B2 | 28 March 2017 |
| | | | | US | 10100129 | B2 | 16 October 2018 |
| | | | | US | 2019062456 | A1 | 28 February 2019 |

Form PCT/ISA/210 (patent family annex) (January 2015)

**INTERNATIONAL SEARCH REPORT**
Information on patent family members

| International application No. |
| --- |
| **PCT/CN2021/101233** |

| Patent document cited in search report | | | Publication date (day/month/year) | Patent family member(s) | | | Publication date (day/month/year) |
| --- | --- | --- | --- | --- | --- | --- | --- |
| | | | | JP | 2021061837 | A | 22 April 2021 |
| | | | | CL | 2017001296 | A1 | 16 February 2018 |
| | | | | US | 2017253665 | A1 | 07 September 2017 |
| | | | | BR | 112017010110 | A2 | 30 January 2018 |
| | | | | PT | 3221363 | T | 23 July 2020 |
| | | | | WO | 2016081748 | A2 | 26 May 2016 |
| | | | | KR | 20170080699 | A | 10 July 2017 |
| | | | | ES | 2807182 | T3 | 22 February 2021 |
| | | | | WO | 2016081748 | A3 | 15 September 2016 |
| | | | | EA | 202090956 | A3 | 30 December 2020 |
| | | | | SG | CN 10201913004 U | A | 30 March 2020 |
| | | | | EP | 3221363 | A2 | 27 September 2017 |
| | | | | UY | 36404 | A | 01 June 2016 |
| | | | | JP | 2017537620 | A | 21 December 2017 |
| | | | | EA | 201790986 | A1 | 31 October 2017 |
| | | | | EA | 202090956 | A2 | 30 September 2020 |
| | | | | PH | 12017500918 | A1 | 20 November 2017 |
| | | | | EA | 035766 | B1 | 07 August 2020 |
| | | | | ME | 03806 | B | 20 April 2021 |
| | | | | IL | 252353 | D0 | 31 July 2017 |
| | | | | US | 2019055320 | A1 | 21 February 2019 |
| | | | | HR | P20201176 | T1 | 13 November 2020 |
| | | | | HK | 1244817 | A1 | 17 August 2018 |
| | | | | PE | 20170782 | A1 | 04 July 2017 |
| | | | | TW | 201625693 | A | 16 July 2016 |
| | | | | CA | 2968357 | A1 | 26 May 2016 |
| | | | | US | 2016145350 | A1 | 26 May 2016 |
| | | | | PL | 3221363 | T3 | 11 January 2021 |
| WO | 2019/200256 | A1 | 17 October 2019 | IL | 277886 | D0 | 30 November 2020 |
| | | | | AU | 2019252795 | A1 | 29 October 2020 |
| | | | | SG | 11202009839 P | A | 27 November 2020 |
| | | | | CN | 112218657 | A | 12 January 2021 |
| | | | | CA | 3096674 | A1 | 17 October 2019 |
| | | | | EP | 3773714 | A1 | 17 February 2021 |
| | | | | BR | 112020020826 | A2 | 19 January 2021 |
| | | | | KR | 20200142542 | A | 22 December 2020 |
| WO | 2019/173692 | A2 | 12 September 2019 | TW | 201945391 | A | 01 December 2019 |
| | | | | CA | 3093468 | A1 | 12 September 2019 |
| | | | | IL | 276731 | D0 | 30 September 2020 |
| | | | | CR | 20200392 | A | 17 November 2020 |
| | | | | EA | 202091710 | A1 | 16 February 2021 |
| | | | | WO | 2019173692 | A3 | 14 November 2019 |
| | | | | EP | 3762421 | A2 | 13 January 2021 |
| | | | | CL | 2020002314 | A1 | 05 March 2021 |
| | | | | CN | 112074535 | A | 11 December 2020 |
| | | | | AR | 114275 | A1 | 12 August 2020 |
| | | | | KR | 20200130403 | A | 18 November 2020 |
| | | | | BR | 112020017382 | A2 | 26 January 2021 |
| | | | | US | 2019352418 | A1 | 21 November 2019 |

Form PCT/ISA/210 (patent family annex) (January 2015)

**INTERNATIONAL SEARCH REPORT**
Information on patent family members

International application No.

**PCT/CN2021/101233**

| Patent document cited in search report | | | Publication date (day/month/year) | Patent family member(s) | | | Publication date (day/month/year) |
|---|---|---|---|---|---|---|---|
| | | | | AU | 2019231791 | A1 | 03 September 2020 |
| | | | | SG | 11202007927 W | A | 29 September 2020 |
| CN | 110785187 | A | 11 February 2020 | CL | 2019003796 | A1 | 31 July 2020 |
| | | | | UY | 37779 | A | 31 January 2019 |
| | | | | CU | 20190109 | A7 | 20 October 2020 |
| | | | | CR | 20190593 | A | 10 May 2020 |
| | | | | CO | 2019014414 | A2 | 24 April 2020 |
| | | | | PE | 20200717 | A1 | 21 July 2020 |
| | | | | BR | 112019027104 | A2 | 18 August 2020 |
| | | | | EC | SP19090538 | A | 28 February 2020 |
| | | | | SG | 11201912473 P | A | 30 January 2020 |
| | | | | WO | 2018237157 | A1 | 27 December 2018 |
| | | | | EP | 3641812 | A1 | 29 April 2020 |
| | | | | EA | 202090104 | A1 | 09 April 2020 |
| | | | | JP | 2020530263 | A | 22 October 2020 |
| | | | | IL | 271539 | D0 | 27 February 2020 |
| | | | | US | 2019031766 | A1 | 31 January 2019 |
| | | | | KR | 20200021087 | A | 27 February 2020 |
| | | | | AU | 2018290237 | A1 | 16 January 2020 |
| | | | | PH | 12019502875 | A1 | 26 October 2020 |
| | | | | TW | 201905001 | A | 01 February 2019 |
| | | | | JO | P20190291 | A1 | 22 December 2018 |
| | | | | CA | 3066774 | A1 | 27 December 2018 |
| WO | 2021/044005 | A1 | 11 March 2021 | US | 2021070876 | A1 | 11 March 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180194858 A1
- WO 2018013611 A1
- WO 2016055609 A1
- US 5500362 A
- US 5821337 A
- US 6737056 B
- US 7332581 B
- WO 2004056312 A
- US 6194551 B
- WO 9951642 A
- US 7371826 B
- US 5648260 A

- US 5624821 A
- WO 9429351 A
- US 7521541 B
- WO 2009036379 A
- WO 2010105256 A
- WO 2012009568 A
- US 9938356 B2
- WO 2016081748 A2
- WO 2018237157 A1
- WO 2017100670 A1
- WO 2018137598 A1
- WO 2016131950 A1

**Non-patent literature cited in the description**

- **ZHANG et al.** *Cancer Res,* 2010, vol. 70, 6407-11
- Fundamental Immunology. Raven Press, 1989
- **KABAT et al.** Sequences of Proteins of Immunological Interest. Public Health Service, National Institutes of Health, 1991
- **KINDT et al.** Kuby Immunology. WH Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. Department of Health and Human Services, National Institutes of Health, 1987
- **NORTH et al.** A New Clustering of Antibody CDR Loop Conformations. *Journal of Molecular Biology,* 2011, vol. 406, 228-256

- **KABAT et al.** Sequences of Proteins of Immunological Interest. Ed. Public Health Service, National Institutes of Health, 1991
- **RAVETCH AND KINET.** *Annu. Rev. Immunol.,* 1991, vol. 9, 457-92
- **CLYNES et al.** *PNAS (USA),* 1998, vol. 95, 652-656
- **FLATMAN et al.** *J. Chromatogr.,* 2007, vol. B848, 79-87
- **NEEDLEMA AND WUNSCH.** *J. Mol. Biol.,* 1970, vol. 48, 444-453
- *CABIOS,* 1989, vol. 4, 11-17
- **SHIELD et al.** *JBC,* 2002, vol. 277, 26733
- **SHIELDS et al.** *J. Biol. Chem.,* 2001, vol. 9 (2), 6591-6604
- **IDUSOGIE et al.** *J. Immunol.,* 2000, vol. 164, 4178-4184
- **DUNCAN & WINTER.** *Nature,* 1988, vol. 322, 738-40
- **R.C. ROWE ; P. J. SESKEY ; S.C. OWEN.** Handbook of Pharmaceutical Excipients. Pharmaceutical Press
- **ESTEP, P et al.** High throughput solution Based measurement of antibody-antigen affinity and epitope binning. *MAbs,* 2013, vol. 5 (2), 270-8